# EUROPEAN PATENT APPLICATION

(11) **EP 4 674 959 A1**
(43) Date of publication of application: **07.01.2026**
(21) Application number: 24763925.5
(22) Date of filing: 27.02.2024
(51) Int. Cl.: C12N 15/09, C07K 19/00, C12N 9/16, C12N 15/31, C12N 15/55, C12N 15/62

(54) **NICKING ENZYME, DNA EDITING SYSTEM, TARGET DNA EDITING METHOD, AND METHOD FOR PRODUCING CELL IN WHICH TARGET DNA IS EDITED**

(30) Priority: 28.02.2023 JP 2023030560
(71) Applicant: Sumitomo Chemical Company, Limited, Tokyo 103-6020 (JP); Hiroshima University, Higashi-Hiroshima-shi Hiroshima 739-8511 (JP)
(72) Inventor: YOSHIMA Tadahiko, Osaka-shi, Osaka 554-8558 (JP); SAKUMA Tetsushi, Hiroshima-shi, Hiroshima 739-8511 (JP); YAMAMOTO Takashi, Hiroshima-shi, Hiroshima 739-8511 (JP); NISHIBORI Nahoko, Hiroshima-shi, Hiroshima 739-8511 (JP)
(74) Representative: J A Kemp LLP
(86) International application number: PCT/JP2024/007113
(87) International publication number: WO 2024/181446

(57) **Abstract**

An artificial nicking enzyme comprising two domains ND1 and ND1n linked via an ND1 linker, wherein
ND1 is at least one polypeptide selected from the group consisting of (a) a polypeptide comprising an amino acid sequence set forth in SEQ ID NO: 98, as well as variants thereof which are polypeptides comprising an amino acid sequence wherein the amino acids corresponding to the 66th and 83rd amino acids of the amino acid sequence set forth in SEQ ID NO: 98 are aspartic acid, and,
ND1n is at least one polypeptide selected from the group consisting of (an) a polypeptide comprising an amino acid sequence which is a substituted amino acid wherein the 66th aspartic acid and/or the 83rd aspartic acid of the amino acid sequence set forth in SEQ ID NO: 98 is substituted with another arbitrary amino acid, as well as variants thereof which are polypeptides comprising an amino acid sequence wherein the amino acid corresponding to the 66th aspartic acid and/or the 83rd aspartic acid of the amino acid sequence set forth in SEQ ID NO: 98 is a substituted amino acid substituted with an arbitrary amino acid other than aspartic acid, the nicking enzyme.

## Description

### [Technical Field]

The present invention relates to a nicking enzyme, as well as a DNA editing system using the same, a method for editing target DNA, and a method for producing a cell in which target DNA has been edited.

### [Background Art]

In recent years, attention has been focused on DNA editing technology using the CRISPR-Cas system, particularly the CRISPR-Cas9 system containing Cas9 protein that has lost part or all of its nuclease activity (dCas9, nCas9) and its guide RNA; for example, the Base Editor (BE) system (Komor et al., Nature 533, 2016, p. 420-424 (NPL 1)) and the Target-AID system (Nishida et al., Science 353, aaf8729, 2016 (NPL 2)) have been developed thus far. Also, by the present inventors, a DNA editing technology using the aforementioned CRISPR-Cas9 system and a fusion protein containing TALE and a nucleic acid base converting enzyme (for example, deaminase) has been developed (International Publication No. WO 2020/050377 (PTL 1)). However, these conventional technologies are dependent on the CRISPR-Cas system, and in order to perform genome editing using these, it is necessary to also introduce guide RNA into cells, therefore a technology with higher safety capable of genome editing using only protein is desired.

As a technology capable of genome editing using only protein, TALEN (Transcription activator-like effector nuclease), an artificial restriction enzyme developed in 2010 as a second-generation genome editing technology, is known. TALEN is a fusion protein having the nuclease domain of FokI, which is a Type IIS restriction enzyme (FokI nuclease domain), as the nuclease domain and TALE as the DNA binding domain, wherein the TALEs of a pair of TALENs each bind to opposite strands of the target DNA, and the FokI nuclease domains form a dimer with each other, thereby exhibiting site-specific double-strand cleavage activity (nuclease activity) of DNA. Regarding the FokI nuclease domain, up to now, there exist a report that linked two FokI nuclease domains and bound them to a zinc finger array (ZF) or TALE to induce double-strand breaks (Minczuk et al., Nucleic Acids Research, 36 (12), 2008, p. 3926-3938 (NPL 3)) and a report that induced single-strand breaks (nicks) using a structure with a mutation introduced into one of the FokI nuclease domains (Yan Luo et al., Scientific Reports, 6:20657, 2016, doi: 10.1038/srep20657 (NPL 4)).

Furthermore, the present inventors developed two novel nuclease domains (nuclease domain 1: ND1, and nuclease domain 2: ND2) different from the conventional FokI nuclease domain, and have succeeded in editing a target site on target DNA by means of an artificial nucleic acid cleaving enzyme containing these nuclease domains and a DNA binding domain such as ZF or TALE (International Publication No. WO 2020/045281 (PTL 2)).

### [Citation List]

### [Patent Literature]

[PTL 1] International Publication No. WO 2020/050377
[PTL 2] International Publication No. WO 2020/045281

### [Non Patent Literature]

[NPL 1] Komor et al., Nature 533, 2016, p. 420-424
[NPL 2] Nishida et al., Science 353, aaf8729, 2016
[NPL 3] Minczuk et al., Nucleic Acids Research, 36 (12), 2008, p. 3926-3938
[NPL 4] Yan Luo et al., Scientific Reports, 6:20657, 2016, doi: 10.1038/srep20657

### [Summary of Invention]

### [Technical Problem]

From the above conventional technologies, it has been shown that for efficient DNA editing, introducing a nick (single-strand break) into double-stranded DNA is important. However, in the above BE system and the like, formation of an R-loop by the CRISPR-Cas system and the accompanying exposure of single-stranded DNA are essential, and the introduction of a nick remains an auxiliary action. Therefore, the present inventors considered whether it might be possible to develop an R-loop-independent DNA editing technology through a mechanism different from the action of nicks in such conventional technologies, namely, through the relaxation of the higher-order structure of the genome or the creation of a partial single-stranded DNA region by the introduction of a nick.

Furthermore, for example, in Zongyi Yi et al., Nature Biotechnology, published on May 22, 2023, https://doi.org/10.1038/s41587-023-01791-y (Reference I), a technology for editing a target site on mitochondrial DNA by combination of a fusion protein containing a nickase such as MutH or Nt.BspD6I(C) and TALE, with a deaminase is described. However, since MutH requires a specific recognition motif (GATC), it has the problem of lacking versatility, and upon investigation by the present inventors, it was found that with the technology using Nt.BspD6I(C) described in said Reference I, editing of nuclear DNA or exogenous DNA other than mitochondrial DNA within cells is difficult.

The present invention was made in view of the problems possessed by the above conventional technologies, and aims to provide a method capable of specifically and efficiently performing editing of target DNA within cells other than mitochondrial DNA by a nucleic acid base converting enzyme, using only protein, without depending on a CRISPR-Cas system such as Cas9, as well as a nicking enzyme useful therefor.

### [Solution to Problem]

The present inventors, in order to achieve the above object, considered whether a highly original structure capable of introducing a nick as a single molecule could be made using nuclease domain 1 (ND1), which is an alternative factor for the FokI nuclease domain. Therefore, they investigated conversion into a nickase of the site-specific DNA cleaving enzyme (DNA double-strand cleaving enzyme) comprising a fusion nuclease domain (scND1) containing two ND1s linked via a linker and a DNA binding domain, already developed in International Application PCT/JP2022/044398, and by modifying the N-terminal side or C-terminal side of the two NDls into a nuclease activity-deficient mutant sequence, succeeded in making it a site-specific nicking enzyme (nickase active form). Incidentally, based on NPL 4 which modified the FokI nuclease domain, for example, when the N-terminal side ND1 was made into a nuclease activity-deficient mutant sequence (for example, from the N-terminal side, in the case of TALE -> nuclease activity-deficient mutant sequence of ND1 -> ND1), it was assumed that a nick would be introduced to the opposite strand (TALE non-recognition strand) of the strand having the DNA binding domain recognition sequence. However, contrary to this, surprisingly, it became clear that with the above nicking enzyme modified from ND1, a nick is introduced to the strand having the DNA binding domain recognition sequence (TALE recognition strand). Furthermore, by using such a nicking enzyme in combination with a fusion protein containing TALE and a nucleic acid base converting enzyme (for example, deaminase), they found that bases at the target site of target DNA within cells other than mitochondrial DNA, such as nuclear DNA and exogenous DNA, can also be efficiently substituted and edited, leading to the completion of the present invention.

Aspects of the present invention provided by such findings are as follows.
[1] An artificial nicking enzyme comprising two domains ND1 and ND1n linked via an ND1 linker, wherein
   ND1 is at least one polypeptide selected from the group consisting of the following (a) to (c):
      (a) a polypeptide comprising an amino acid sequence set forth in SEQ ID NO: 98
      (b) a polypeptide comprising an amino acid sequence wherein one or a plurality of amino acids are substituted, deleted, inserted and/or added in the amino acid sequence set forth in SEQ ID NO: 98, and wherein the amino acids corresponding to 66th and 83rd amino acids of the amino acid sequence set forth in SEQ ID NO: 98 are aspartic acid
      (c) a polypeptide comprising an amino acid sequence having 80% or more homology with the amino acid sequence set forth in SEQ ID NO: 98, and wherein the amino acids corresponding to the 66th and 83rd amino acids of the amino acid sequence set forth in SEQ ID NO: 98 are aspartic acid
      and,
   ND1n is at least one polypeptide selected from the group consisting of the following (an) to (cn):
      (an) a polypeptide comprising an amino acid sequence which is a substituted amino acid wherein the 66th aspartic acid and/or the 83rd aspartic acid of the amino acid sequence set forth in SEQ ID NO: 98 is substituted with another arbitrary amino acid,
      (bn) a polypeptide comprising an amino acid sequence wherein one or a plurality of amino acids are substituted, deleted, inserted and/or added in the amino acid sequence set forth in SEQ ID NO: 98, and wherein the amino acid corresponding to the 66th aspartic acid and/or the 83rd aspartic acid of the amino acid sequence set forth in SEQ ID NO: 98 is a substituted amino acid substituted with an arbitrary amino acid other than aspartic acid, and
      (cn) a polypeptide comprising an amino acid sequence having 80% or more homology with the amino acid sequence set forth in SEQ ID NO: 98, and wherein the amino acid corresponding to the 66th aspartic acid and/or the 83rd aspartic acid of the amino acid sequence set forth in SEQ ID NO: 98 is a substituted amino acid substituted with an arbitrary amino acid other than aspartic acid.
[2] The nicking enzyme according to [1], wherein the length of the ND1 linker is 30 to 300 amino acid residues.
[3] A fusion protein comprising a first DNA binding domain and the nicking enzyme according to [1] or [2].
[4] A DNA editing system comprising:
   a first fusion protein which is the fusion protein according to [3], and
   a second fusion protein comprising a second DNA binding domain and a nucleic acid base converting enzyme.
[5] The DNA editing system according to [4], further comprising a third fusion protein comprising a third DNA binding domain and a transcription regulatory factor.
[6] A method for editing target DNA, comprising:
   a step of bringing the DNA editing system according to [4] or [5] into contact with target DNA and editing a base at a target site of the target DNA by the nucleic acid base converting enzyme activity, wherein
   the distance between a first DNA binding domain recognition sequence recognized by the first DNA binding domain or its complementary sequence and a second DNA binding domain recognition sequence recognized by the second DNA binding domain or its complementary sequence is 8 to 48 bases.
[7] A method for producing a cell in which target DNA has been edited, comprising:
   a step of introducing the DNA editing system according to [4] or [5] into a cell or expressing it within the cell to bring it into contact with target DNA within the cell other than mitochondrial DNA, and editing a base at a target site of the target DNA by the nucleic acid base converting enzyme activity, wherein
   the distance between a first DNA binding domain recognition sequence recognized by the first DNA binding domain or its complementary sequence and a second DNA binding domain recognition sequence recognized by the second DNA binding domain or its complementary sequence is 8 to 48 bases.
[8] The method according to [6] or [7], wherein
   the first DNA binding domain recognition sequence or its complementary sequence is present via a first spacer of 4 to 16 bases on the 5' side or the 3' side of the target site, and
   the second DNA binding domain recognition sequence or its complementary sequence is present via a second spacer of 3 to 31 bases on the side opposite to the first DNA binding domain recognition sequence or its complementary sequence of the target site.
[9] A kit for use in the method according to [6] or [7] or [8], comprising:
   at least one selected from the group consisting of the first fusion protein, a first fusion protein expression vector, and a polynucleotide encoding the first fusion protein, and at least one selected from the group consisting of the second fusion protein, a second fusion protein expression vector, and a polynucleotide encoding the second fusion protein, wherein
   the first fusion protein expression vector is at least one selected from the group consisting of (i) a vector comprising a polynucleotide encoding ND1, ND1n, the ND1 linker, and the first DNA binding domain, and (ii) a vector comprising a polynucleotide encoding ND1, ND1n, and the ND1 linker, and an insertion site for a polynucleotide encoding the first DNA binding domain, and
   the second fusion protein expression vector is at least one selected from the group consisting of (iii) a vector comprising a polynucleotide encoding the nucleic acid base converting enzyme and the second DNA binding domain, and (iv) a vector comprising a polynucleotide encoding the nucleic acid base converting enzyme and an insertion site for a polynucleotide encoding the second DNA binding domain.
[10] The kit according to [9], further comprising at least one selected from the group consisting of a third fusion protein comprising a third DNA binding domain and a transcription regulatory factor, a third fusion protein expression vector, and a polynucleotide encoding the third fusion protein, wherein
   the third fusion protein expression vector is at least one selected from the group consisting of (vii) a vector comprising a polynucleotide encoding a transcription regulatory factor and a third DNA binding domain, and (viii) a vector comprising a polynucleotide encoding the transcription regulatory factor and an insertion site for a polynucleotide encoding the third DNA binding domain.
[11] The fusion protein according to [3], further comprising a nucleic acid base converting enzyme.
[12] A method for editing target DNA, comprising a step of bringing the fusion protein according to [11] into contact with target DNA and editing a base at a target site of the target DNA by the nucleic acid base converting enzyme activity.
[13] A method for producing a cell in which target DNA has been edited, comprising:
   a step of introducing the fusion protein according to [11] into a cell or expressing it within the cell to bring it into contact with target DNA within the cell other than mitochondrial DNA, and editing a base at a target site of the target DNA by the nucleic acid base converting enzyme activity.
[14] A kit for use in the method according to [12] or [13], comprising:
   at least one selected from the group consisting of the fusion protein, a fusion protein expression vector, and a polynucleotide encoding the fusion protein, wherein
   the fusion protein expression vector is at least one selected from the group consisting of (v) a vector comprising a polynucleotide encoding ND1, ND1n, the ND1 linker, a nucleic acid base converting enzyme, and the first DNA binding domain, and (vi) a vector comprising a polynucleotide encoding ND1, ND1n, the ND1 linker, and the nucleic acid base converting enzyme, and an insertion site for a polynucleotide encoding the first DNA binding domain.

### [Advantageous Effects of Invention]

According to the present invention, it becomes possible to provide a method capable of specifically and efficiently performing editing of target DNA within cells other than mitochondrial DNA by a nucleic acid base converting enzyme, using only protein, without depending on a CRISPR-Cas system such as Cas9, as well as a nicking enzyme useful therefor.

### [Brief Description of Drawings]

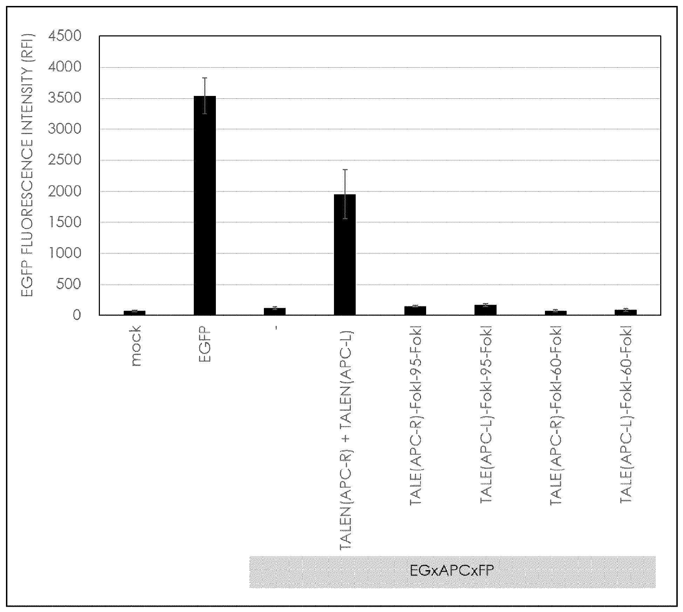

[Fig. 1] Fig. 1 is a graph showing results of detecting double-strand cleavage activity of TALE-scFokI containing two FokI nuclease domains linked via a linker by the SSA assay method. Note that "95" in the figure indicates the 95 amino acid residue HTS95 linker, and "60" indicates the 60 amino acid residue GGGGSx12 linker (the same applies hereinafter).

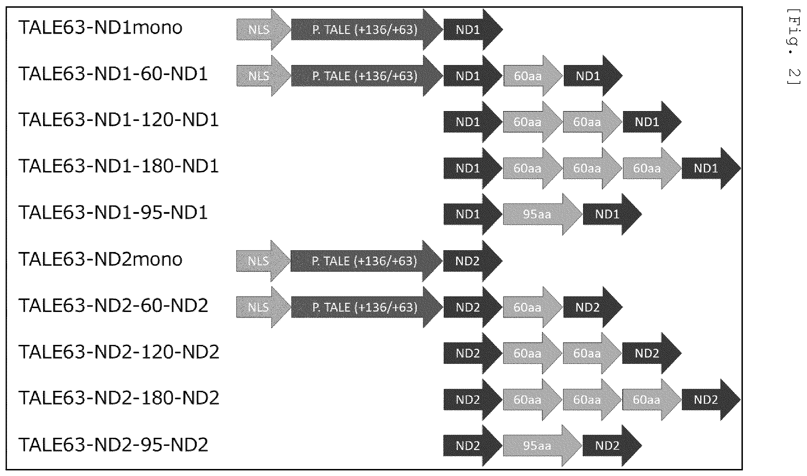

[Fig. 2] Fig. 2 is a conceptual diagram showing the configuration of TALE-scND1 and TALE-scND2 containing two nuclease domains (ND1 or ND2) linked via a linker, and TALE-ND1mono and TALE-ND2mono containing one nuclease domain. The case of using TALE63 is shown as an example of TALE (DNA binding domain). Note that "120" in the figure indicates the 120 amino acid residue GGGGSx24 linker, and "180" indicates the 180 amino acid residue GGGGSx36 linker (the same applies hereinafter).

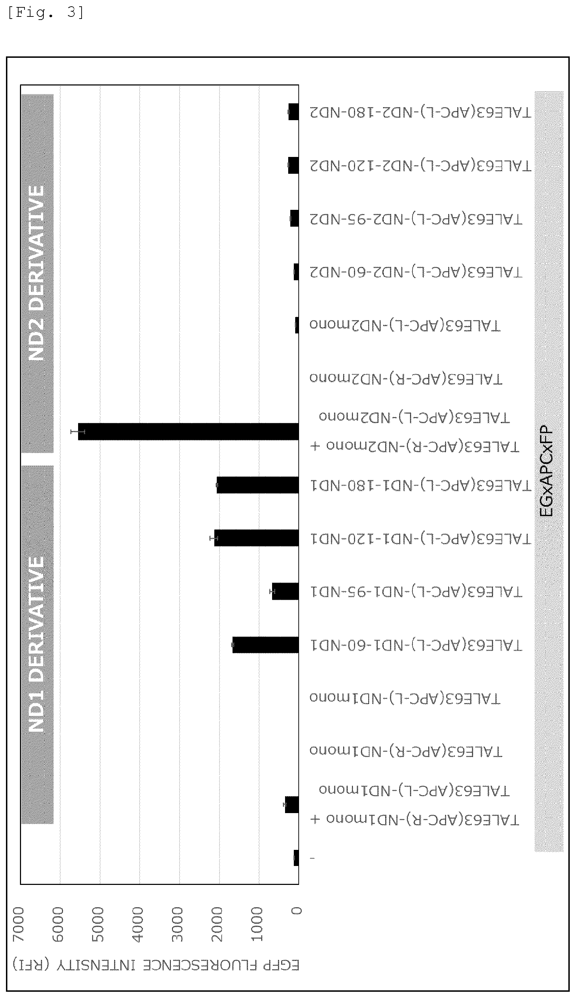

[Fig. 3] Fig. 3 is a graph showing results of detecting double-strand cleavage activity by the SSA assay method using TALE-scND1 and TALE-ND1mono (ND1 derivative), as well as TALE-scND2 and TALE-ND2mono (ND2 derivative) shown in Fig. 2.

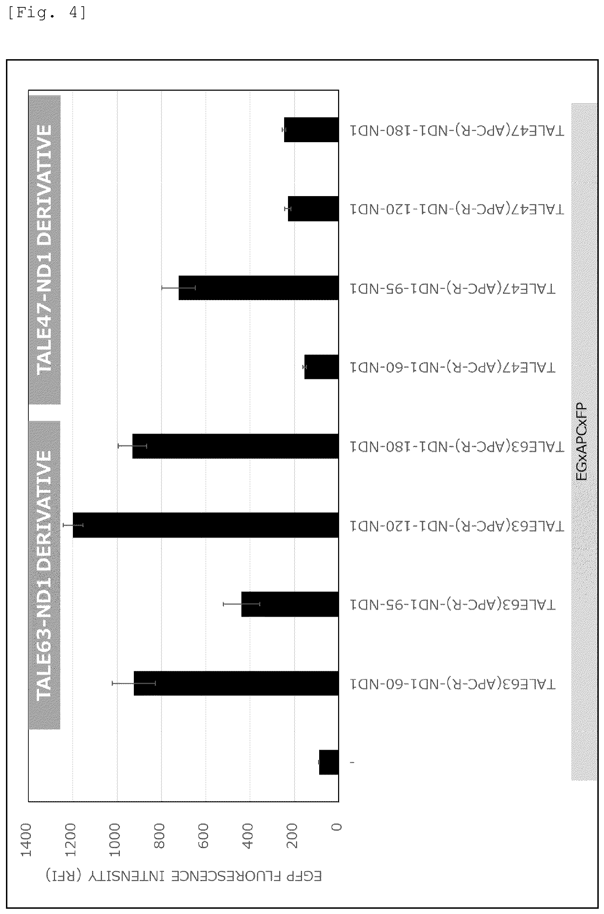

[Fig. 4] Fig. 4 is a graph showing results of detecting the effect of linker length on the double-strand cleavage activity of TALE-scND1 by the SSA assay method in the case where TALE47 was used and the case where TALE63 was used as TALE (DNA binding domain).

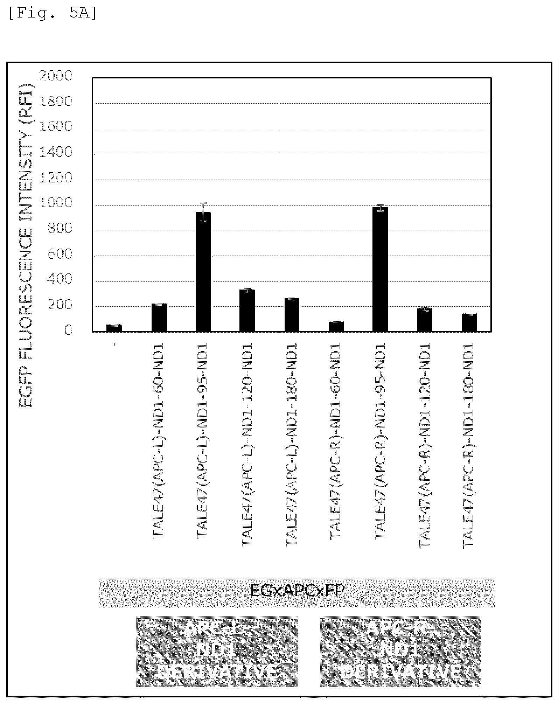

[Fig. 5A] Fig. 5A is a graph showing results of evaluating the effect of target gene type and linker length on the double-strand cleavage activity of TALE-scND1 by the SSA assay method. APC was used as the target gene, and TALE47 was used as TALE.

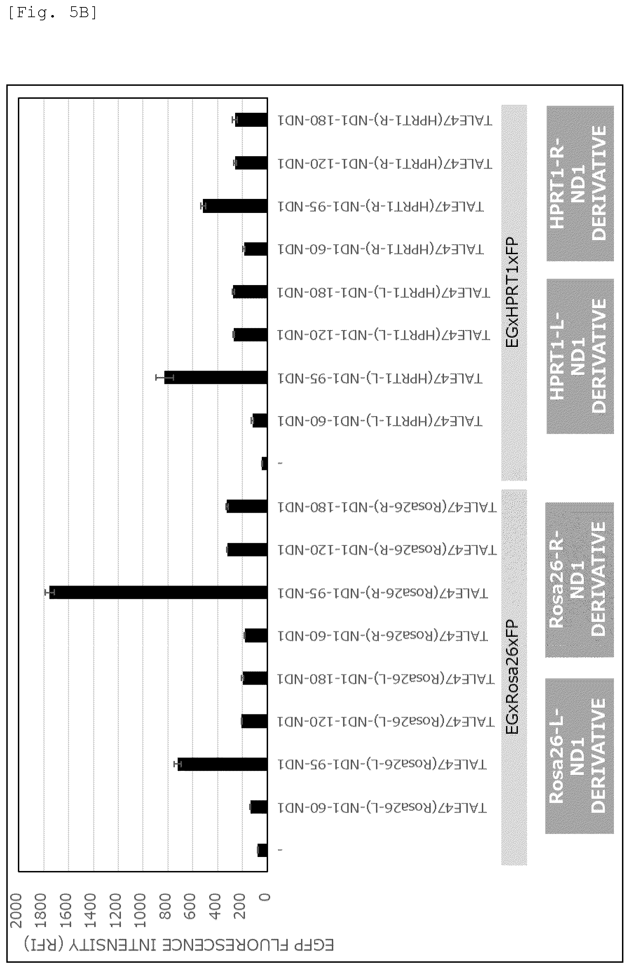

[Fig. 5B] Fig. 5B is a graph showing results of evaluating the effect of target gene type and linker length on the double-strand cleavage activity of TALE-scND1 by the SSA assay method. Rosa26 or HPRT1 was used as the target gene, and TALE47 was used as TALE.

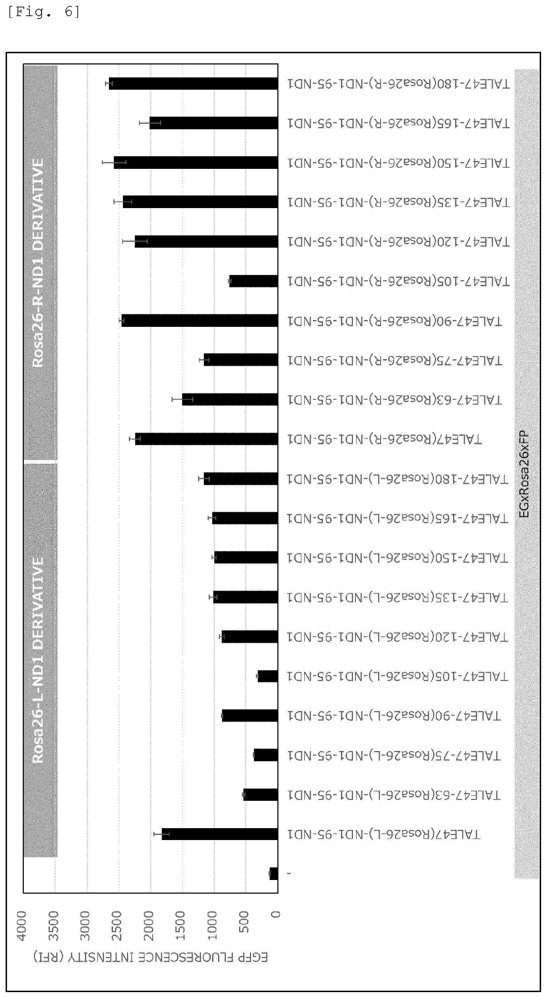

[Fig. 6] Fig. 6 is a graph showing results of evaluating the effect of lengthening the C-terminal domain of TALE on the double-strand cleavage activity of TALE-scND1 by the SSA assay method. Rosa26 was used as the target gene, TALE47 was used as TALE, and the HTS95 linker was used as the linker connecting the two NDls.

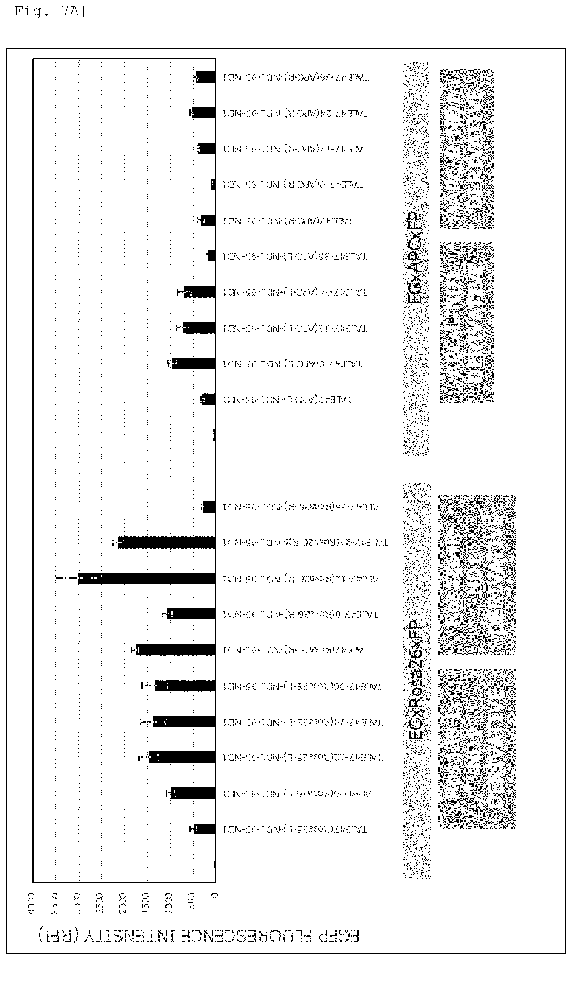

[Fig. 7A] Fig. 7A is a graph showing results of evaluating the effect of shortening the C-terminal domain of TALE on the double-strand cleavage activity of TALE-scND1 by the SSA assay method. Rosa26 or APC was used as the target gene, TALE47 was used as TALE, and the HTS95 linker was used as the linker connecting the two NDls.

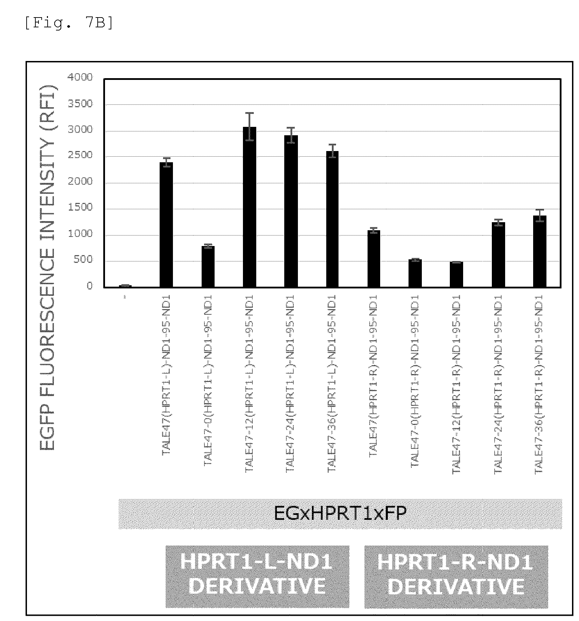

[Fig. 7B] Fig. 7B is a graph showing results of evaluating the effect of shortening the C-terminal domain of TALE on the double-strand cleavage activity of TALE-scND1 by the SSA assay method. HPRT1 was used as the target gene, TALE47 was used as TALE, and the HTS95 linker was used as the linker connecting the two NDls.

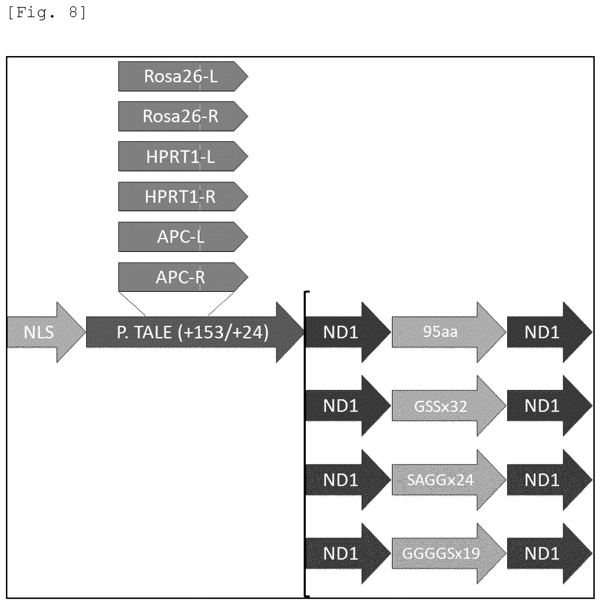

[Fig. 8] Fig. 8 is a conceptual diagram showing the configuration of TALE-scND1 containing two NDls linked via different types of linkers.

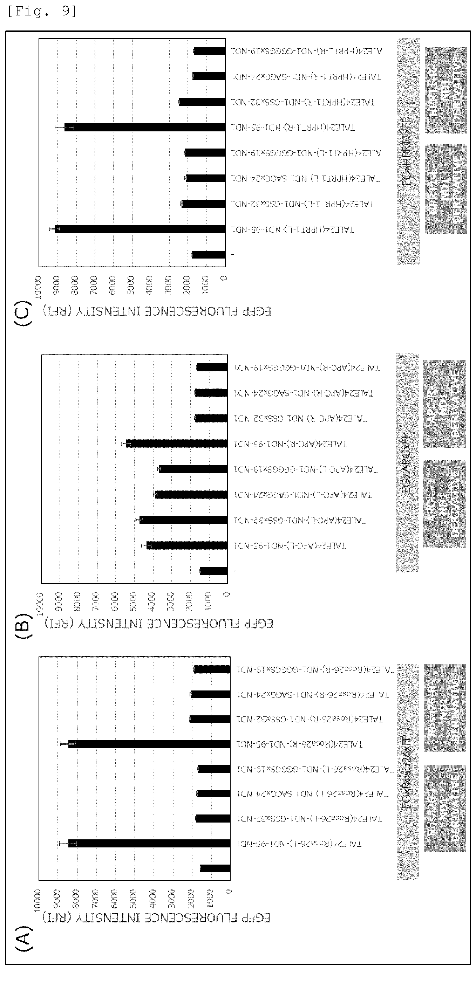

[Fig. 9] Fig. 9 is a graph showing results of detecting double-strand cleavage activity by the SSA assay method using TALE-scND1 shown in Fig. 8. As linkers, HTS95 linker (95 amino acid residues), GSSx32 linker (96 amino acid residues), SAGGx24 linker (96 amino acid residues), and GGGGSx19 linker (95 amino acid residues) were used. As target genes, (A) Rosa26, (B) APC, and (C) HPRT1 were used, and as TALE, TALE24 was used.

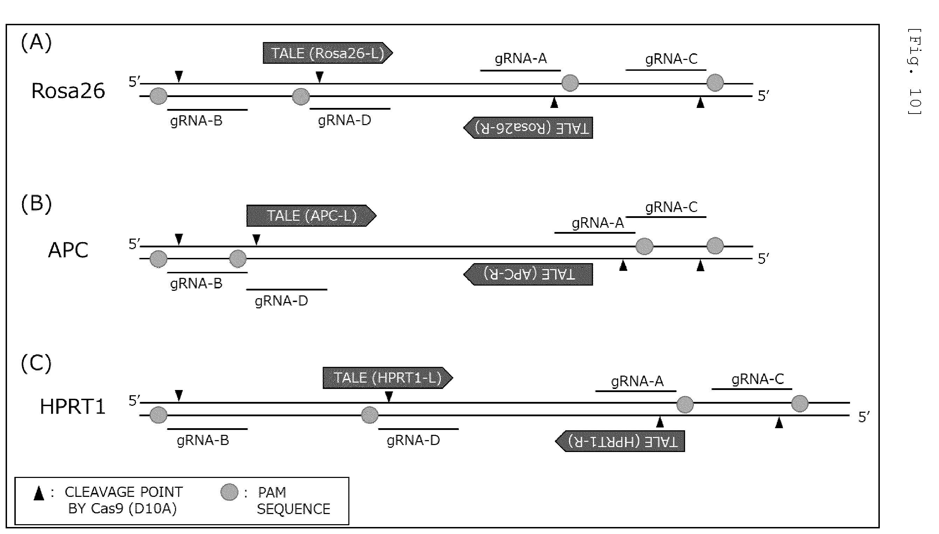

[Fig. 10] Fig. 10 is a conceptual diagram showing the positional relationship between the PAM sequence and the cleavage point by Cas9 (D10A) on each target gene, and TALE and guide RNA, in [Test Example 1] <2. Results> (3) (i). As target genes, (A) Rosa26, (B) APC, and (C) HPRT1 were used.

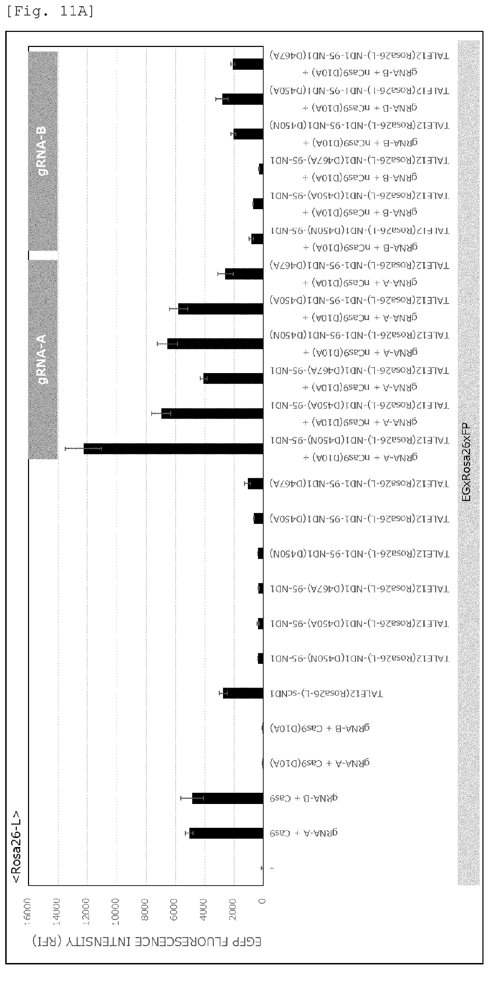

[Fig. 11A] Fig. 11A is a graph showing results of detecting the DNA cleavage activity of various ND1 mutants of TALE-scND1 by the SSA assay method in [Test Example 1] <2. Results> (3) (i). gRNA-A or gRNA-B designed on the target gene Rosa26 and nCas9 (D10A) were co-expressed, and it was evaluated which strand of the double-stranded DNA was being cleaved. As TALE, TALE12 (Rosa26-L) was used.

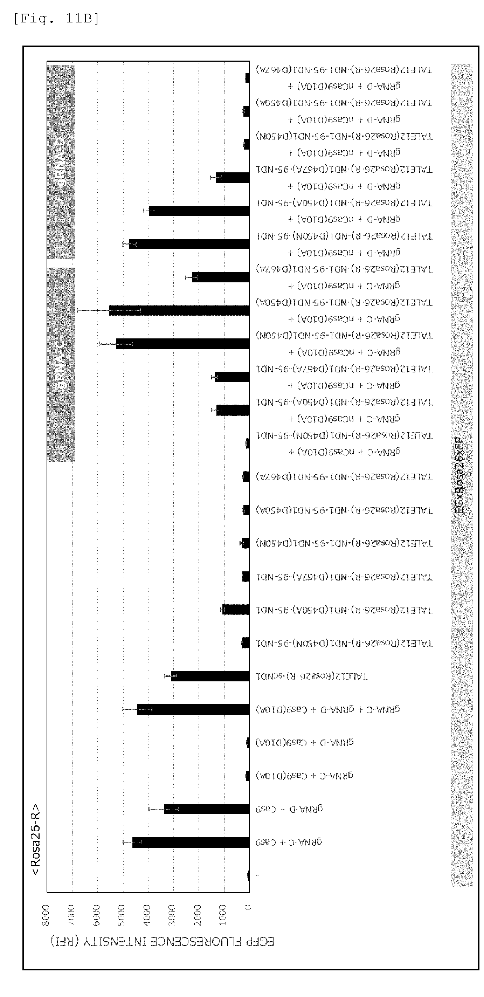

[Fig. 11B] Fig. 11B is a graph showing results of detecting the DNA cleavage activity of various ND1 mutants of TALE-scND1 by the SSA assay method in [Test Example 1] <2. Results> (3) (i). gRNA-C or gRNA-D designed on the target gene Rosa26 and nCas9 (D10A) were co-expressed, and it was evaluated which strand of the double-stranded DNA was being cleaved. As TALE, TALE12 (Rosa26-R) was used.

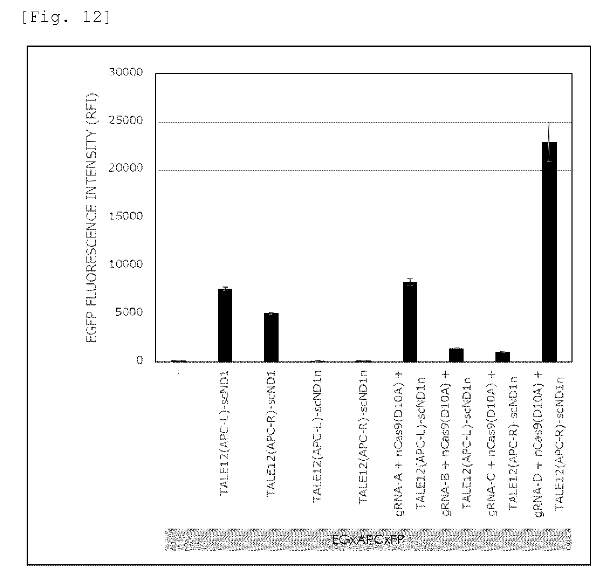

[Fig. 12] Fig. 12 is a graph showing results of detecting the DNA cleavage activity of various ND1 mutants of TALE-scND1 by the SSA assay method in [Test Example 1] <2. Results> (3) (i). APC was used as the target gene, gRNA-A or gRNA-B designed on APC and nCas9 (D10A) were co-expressed, and it was evaluated which strand of the double-stranded DNA was being cleaved. As TALE, TALE12 (APC-L) was used. Also, gRNA-C or gRNA-D designed on APC and nCas9 (D10A) were co-expressed, and it was evaluated which strand of the double-stranded DNA was being cleaved. As TALE, TALE12 (APC-R) was used.

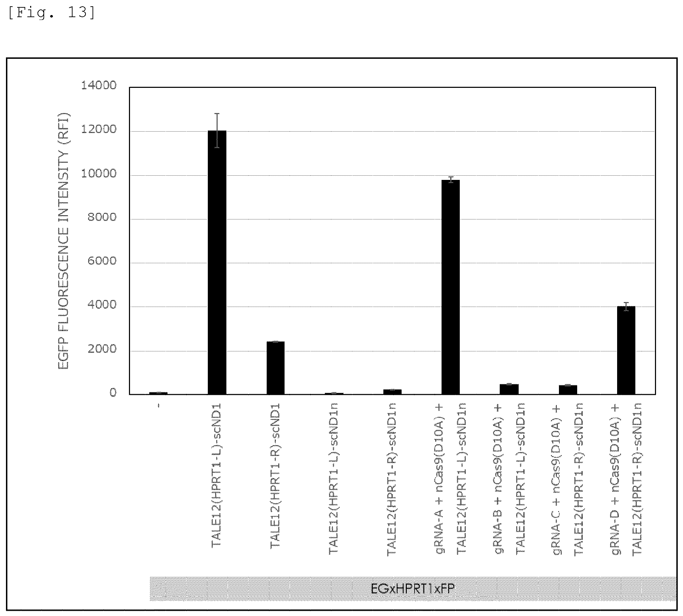

[Fig. 13] Fig. 13 is a graph showing results of detecting the DNA cleavage activity of various ND1 mutants of TALE-scND1 by the SSA assay method in [Test Example 1] <2. Results> (3) (i). HPRT1 was used as the target gene, gRNA-A or gRNA-B designed on HPRT1 and nCas9 (D10A) were co-expressed, and it was evaluated which strand of the double-stranded DNA was being cleaved. As TALE, TALE12 (HPRT1-L) was used. Also, gRNA-C or gRNA-D designed on HPRT1 and nCas9 (D10A) were co-expressed, and it was evaluated which strand of the double-stranded DNA was being cleaved. As TALE, TALE12 (HPRT1-R) was used.

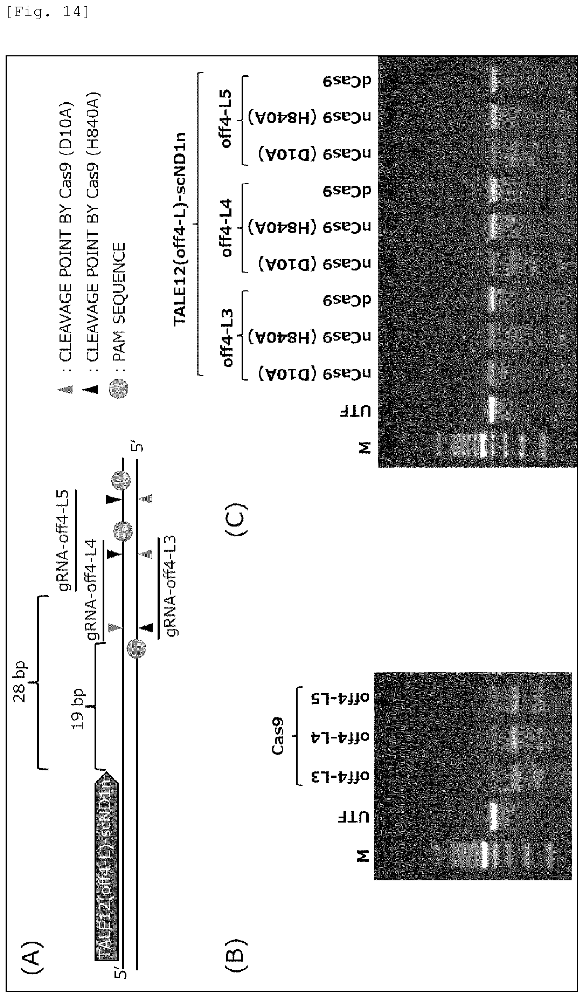

[Fig. 14] In [Test Example 1] <2. Results> (3) (ii), (A) is a conceptual diagram showing the positional relationship of the PAM sequence, the cleavage point by nCas9, the guide RNA, and the TALE of TALE12 (off4-L)-scND1n, (B) is a photograph of agarose gel electrophoresis showing the results of performing a T7E1 assay after introducing into cells together with Cas9 for the purpose of confirming that the guide RNAs (gRNA-off4-L3, gRNA-off4-L4, gRNA-off4-L5) designed on APC used for nickase activity confirmation function normally, (C) is a photograph of agarose gel electrophoresis showing the results of performing a T7E1 assay after introducing into cells in these combinations for the purpose of confirming whether mutations occur when TALE12(off4-L)-scND1n is co-expressed with any one of gRNA-off4-L3, gRNA-off4-L4 and gRNA-off4-L5, as well as any one of nCas9 (D10A), nCas9 (H840A) and dCas9, and coexists with either nCas9 (D10A) or nCas9 (H840A).

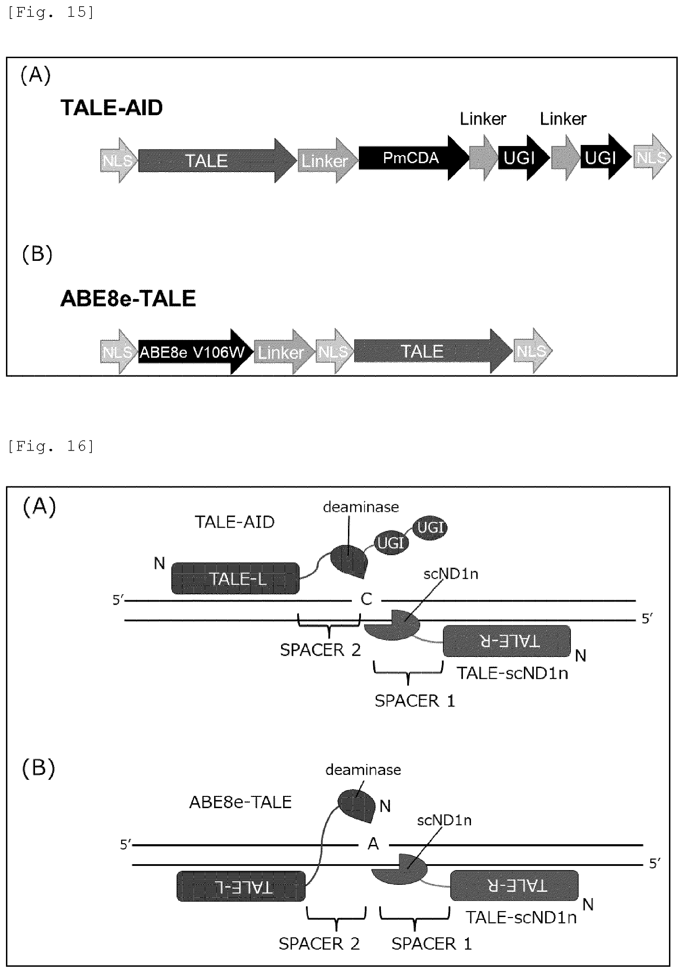

[Fig. 15] Fig. 15 is a conceptual diagram showing the configuration of (A) TALE-AID and (B) ABE8e-TALE in [Test Example 1] <2. Results> (4).
[Fig. 16] Fig. 16 is (A) a conceptual diagram showing one embodiment of base editing by cooperation between TALE-scND1n and TALE-AID, and (B) a conceptual diagram showing one embodiment of base editing by cooperation between TALE-scND1n and ABE8e-TALE.

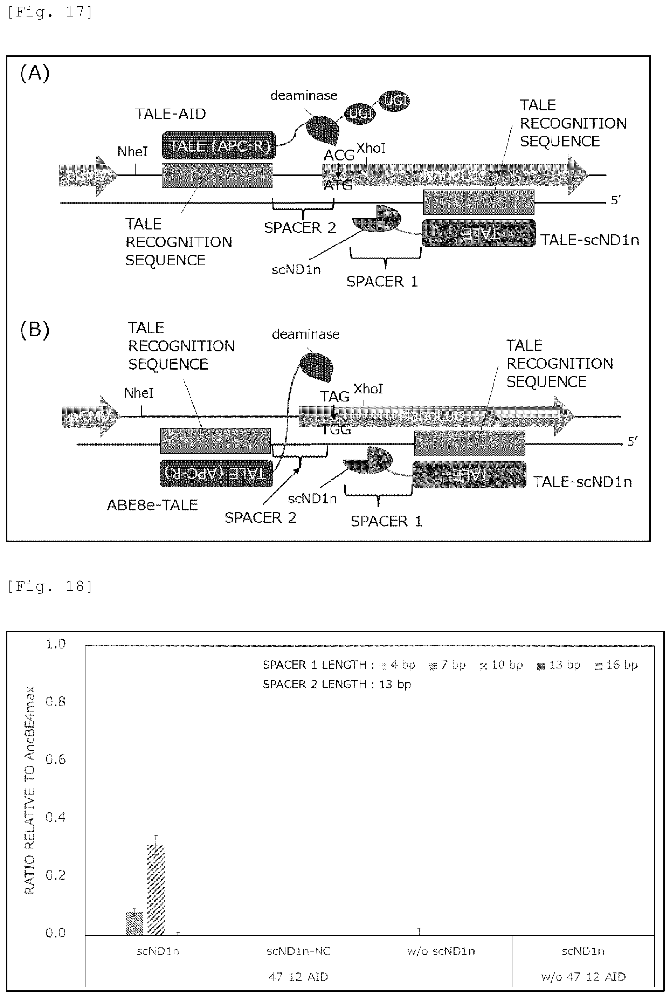

[Fig. 17] Fig. 17 is a conceptual diagram showing the structure of the NanoLuc expression reporter for evaluating base substitution activity prepared in [Test Example 1] <1. Method> (12), in [Test Example 1] <2. Results> (4). (A) shows one embodiment in the case where TALE-AID was used as the TALE-deaminase, and (B) shows one embodiment in the case where ABE8e-TALE was used as the TALE-deaminase.
[Fig. 18] Fig. 18 is a graph showing evaluation results by reporter assay for confirming the base substitution activity by cooperation between TALE-scND1n and TALE-deaminase. As the TALE-deaminase, TALE47(APC-R)-12-AID was used, and as TALE-scND1n, TALE12(Rosa26-L)-scND1n was used, respectively.

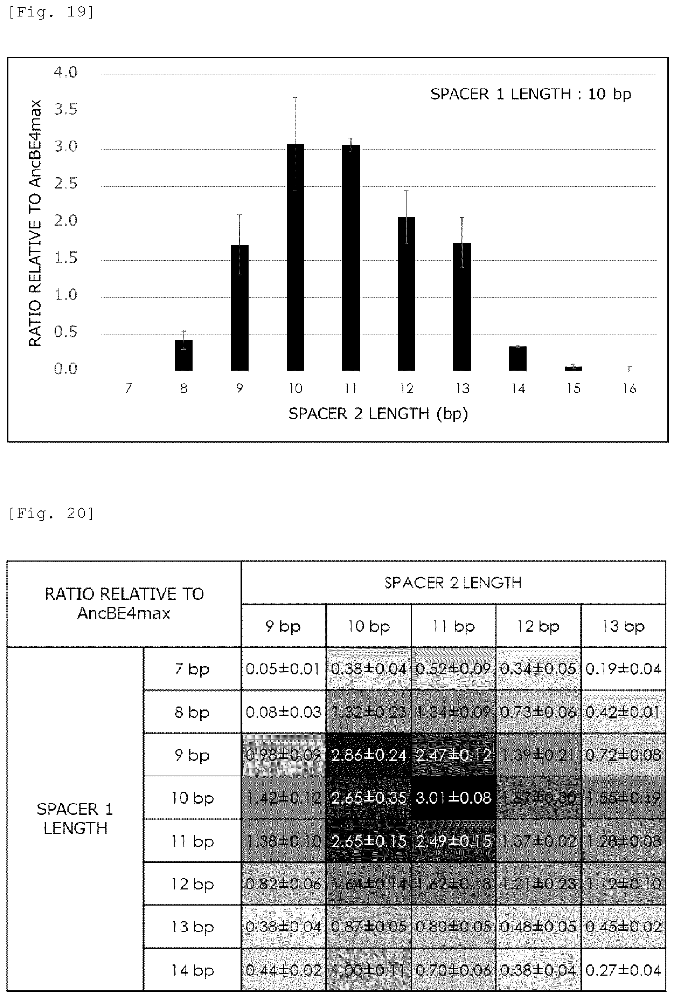

[Fig. 19] Fig. 19 is a graph showing evaluation results by reporter assay for examining the optimal length of spacer 2 in the base substitution activity by cooperation between TALE-scND1n and TALE-AID. A reporter in which the spacer 2 length was from 7 to 16 bases, varying by one base each time, was used. As TALE-AID, TALE47 (APC-R) -12-AID was used, and as TALE-scND1n, TALE12(Rosa26-LM.2)-scND1n was used.
[Fig. 20] Fig. 20 is a diagram (heatmap) showing evaluation results by reporter assay of the base substitution activity when both spacer 1 length and spacer 2 length were varied in the base substitution activity by cooperation between TALE-scND1n and TALE-AID. A reporter in which the spacer 2 length was from 9 to 13 bases, varying by one base each time, was used. As TALE12-scND1n, one into which a TALE repeat domain corresponding to the nucleotide sequence of eight types of TALE recognition sequences on the reporter: Rosa26-LM3.2, Rosa26-LM2.2, Rosa26-LM1.2, Rosa26-LM.2, Rosa26-LM.2+1, Rosa26-LM.2+2, Rosa26-LM.2+3, Rosa26-LM.2+4 was inserted, was used. As TALE-AID, TALE47(APC-R)-12-AID was used.

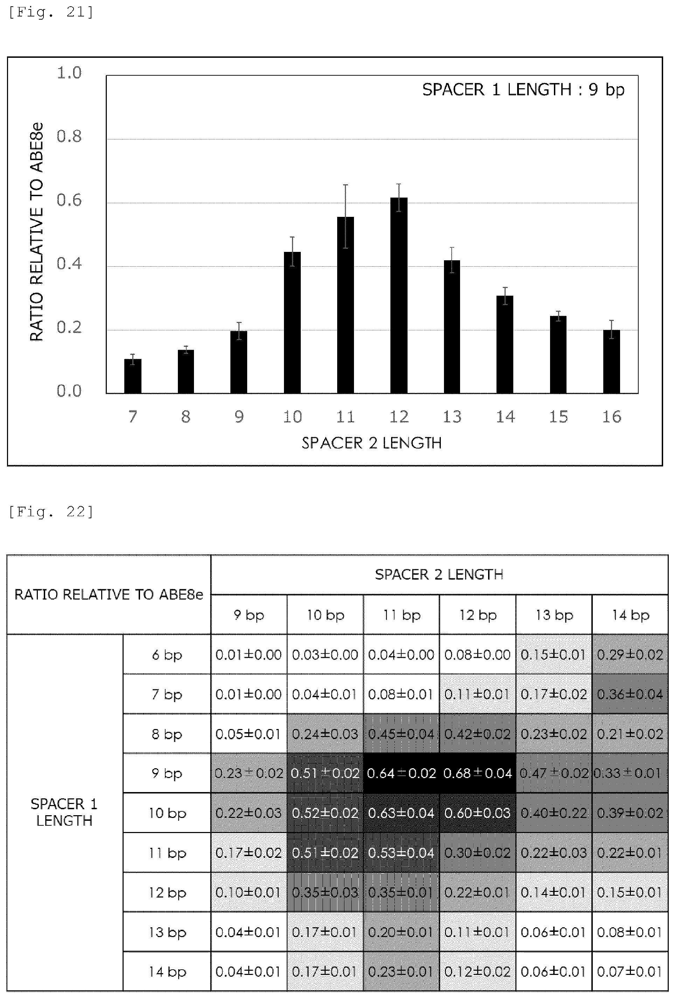

[Fig. 21] Fig. 21 is a graph showing evaluation results by reporter assay for examining the optimal length of spacer 2 in the base substitution activity by cooperation between TALE-scND1n and ABE8e-TALE. A reporter in which the spacer 2 length was from 7 to 16 bases, varying by one base each time, was used. As ABE8e-TALE, ABE8e-32-TALE47(APC-R) was used, and as TALE-scND1n, TALE12(Rosa26-LM1.2)-scND1n was used.
[Fig. 22] Fig. 22 is a diagram (heatmap) showing evaluation results by reporter assay of the base substitution activity when both spacer 1 length and spacer 2 length were varied in the base substitution activity by cooperation between TALE-scND1n and ABE8e-TALE. A reporter in which the spacer 2 length was from 9 to 14 bases, varying by one base each time, was used. As TALE12-scND1n, one into which a TALE repeat domain corresponding to the nucleotide sequence of nine types of TALE recognition sequences on the reporter: Rosa26-LM4.2, Rosa26-LM3.2, Rosa26-LM2.2, Rosa26-LM1.2, Rosa26-LM.2, Rosa26-LM.2+1, Rosa26-LM.2+2, Rosa26-LM.2+3, Rosa26-LM.2+4 was inserted, was used. As ABE8e-TALE, ABE8e-32-TALE47(APC-R) was used.

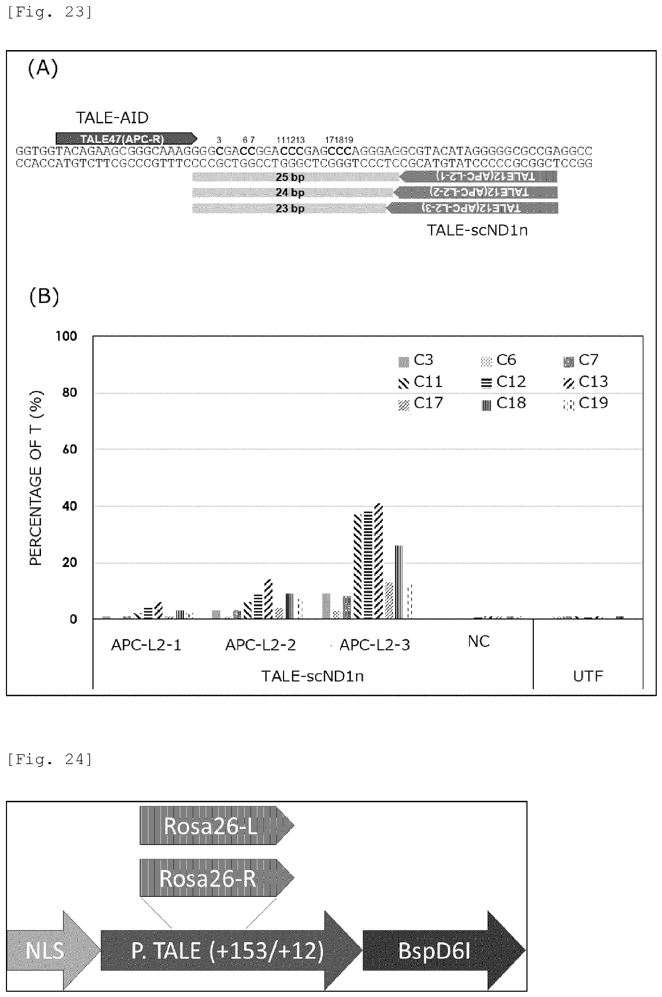

[Fig. 23] Fig. 23 is a graph showing the percentage of T at each target base analyzed by EditR in the base substitution activity towards endogenous DNA by cooperation between TALE-scND1n and TALE-AID. As TALE-AID, TALE47(APC-R)-12-AID was used, and as TALE-scND1n, TALE12(APC-L2-1)-scND1n, TALE12(APC-L2-2)-scND1n, and TALE12 (APC-L2-3) -scND1n were used, respectively. (A) is a conceptual diagram showing the recognition position of TALE of TALE47(APC-R)-12-AID and the three types of TALE-scND1n on APC, as well as the position of the target base, and (B) is a graph showing the percentage of T at each C, which is the target base.
[Fig. 24] Fig. 24 is a conceptual diagram showing the structure of TALE-BspD6I used for evaluation of nicking activity of nickase BspD6I by SSA assay. BspD6I was linked in place of scND1n to a TALE structure similar to TALE12-scND1n, and a TALE repeat domain corresponding to the nucleotide sequence of Rosa26-L, Rosa26-R was inserted.

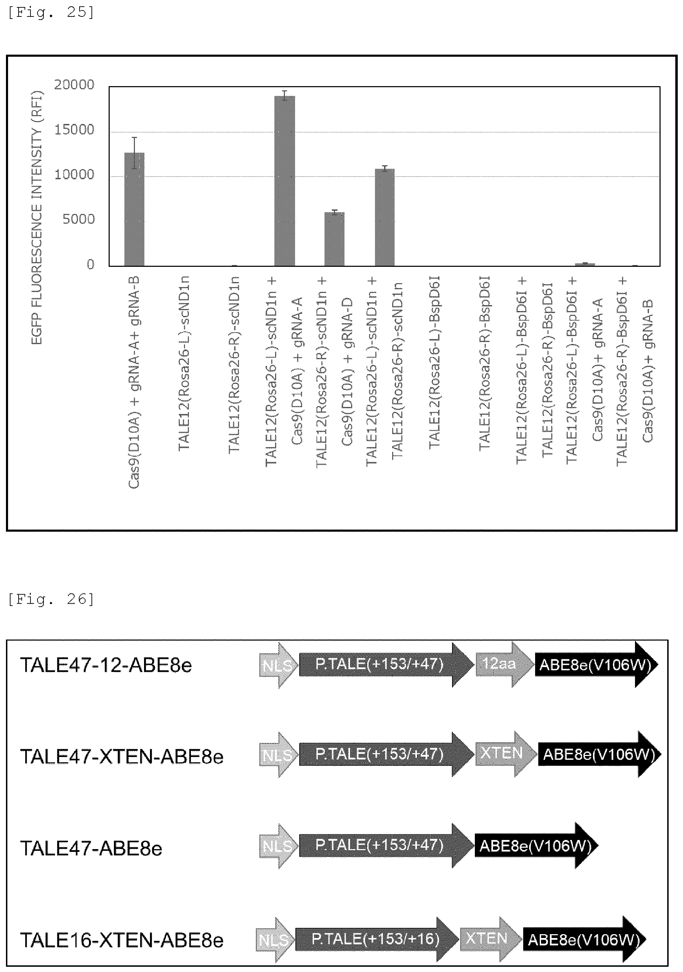

[Fig. 25] Fig. 25 is a graph showing results of detecting the nickase activity of TALE12-BspD6I within the nucleus by the SSA assay method in [Test Example 2] <2. Results> (1). Double nicking activity by each combination of TALE12(Rosa26-L)-scND1n, TALE12(Rosa26-R)-scND1n, TALE12(Rosa26-L)-BspD6I, TALE12(Rosa26-R)-BspD6I, nCas9(D10A), and guide RNA (gRNA-A, B, D) was evaluated.
[Fig. 26] Fig. 26 is a conceptual diagram showing the configuration of various structures of TALE-ABE8e linked via different types of linkers.

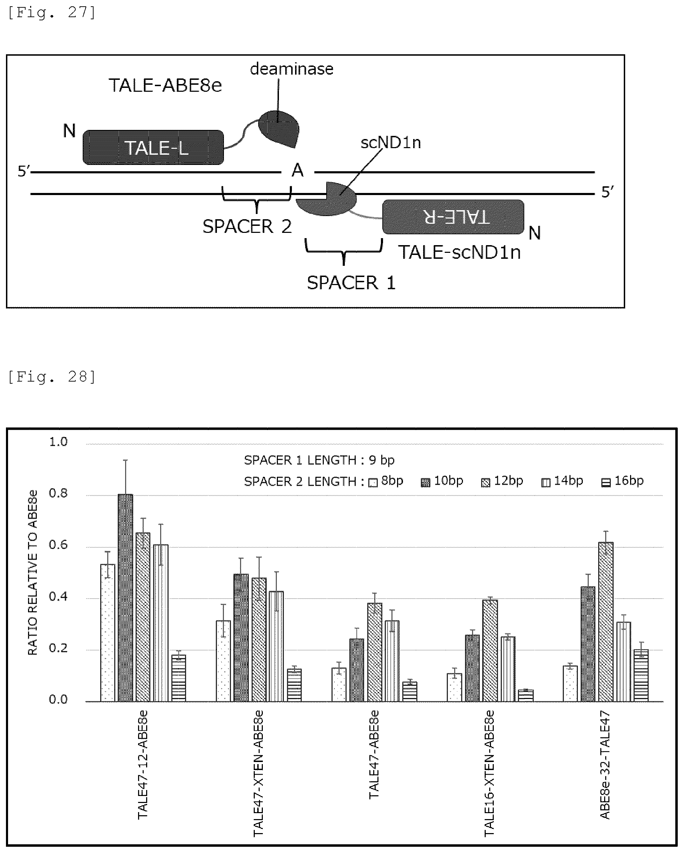

[Fig. 27] Fig. 27 is a conceptual diagram showing one embodiment of base editing by cooperation between TALE-scND1n and TALE-ABE8e.
[Fig. 28] Fig. 28 is a graph showing evaluation results by reporter assay for examining the optimal length of spacer 2 in the base substitution activity by cooperation between TALE-scND1n and various structures of TALE-ABE8e in [Test Example 2] <2. Results> (2).

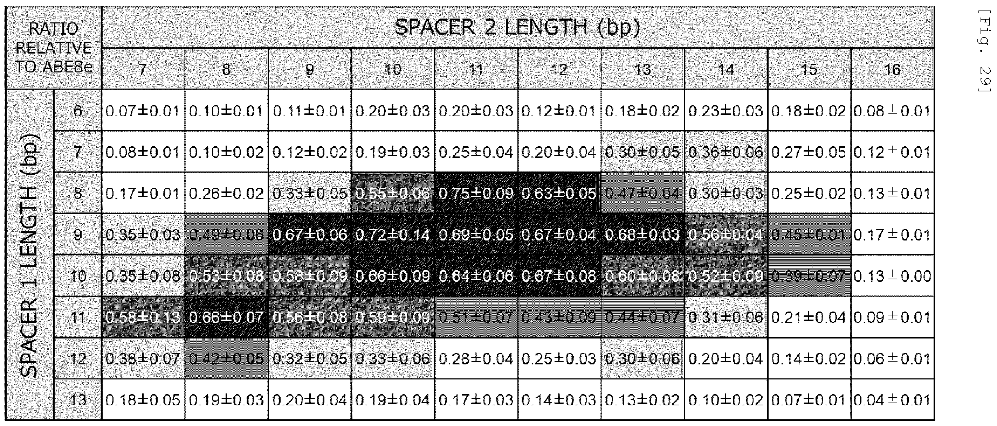

[Fig. 29] Fig. 29 is a diagram (heatmap) showing evaluation results by reporter assay of the base substitution activity when both spacer 1 length and spacer 2 length were varied in the base substitution activity by cooperation between TALE-scND1n and TALE-ABE8e. A reporter in which the spacer 2 length was from 7 to 16 bases, varying by one base each time, was used. As TALE12-scND1n, one into which a TALE repeat domain corresponding to the nucleotide sequence of eight types of TALE recognition sequences on the reporter: Rosa26-LM4.2, Rosa26-LM3.2, Rosa26-LM2.2, Rosa26-LM1.2, Rosa26-LM.2, Rosa26-LM.2+1, Rosa26-LM.2+2, Rosa26-LM.2+3 was inserted, was used. As TALE-ABE8e, TALE47(APC-R)-12-ABE8e was used.

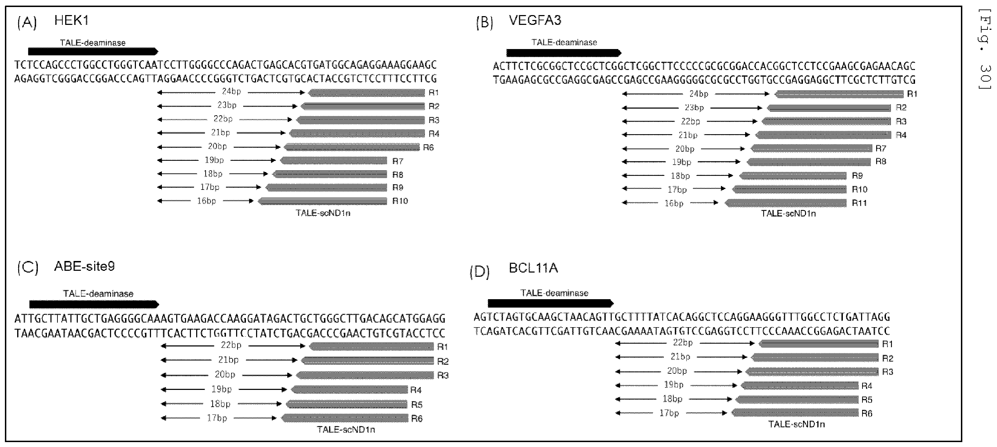

[Fig. 30] Fig. 30 is a conceptual diagram showing the positional relationship between the TALE recognition sequence of TALE-scND1n and the TALE recognition sequence of TALE47-12-AID or TALE-ABE8e on each target site ((A) HEK1, (B) VEGFA3, (C) ABE-site9, (D) BCL11A).

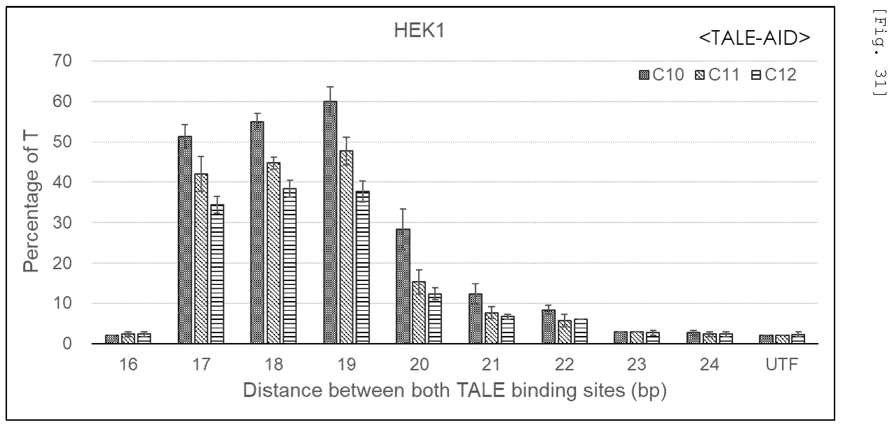

[Fig. 31] Fig. 31 is a graph showing the percentage of T at each C, which is each target base on the HEK1 site, analyzed by EditR in the base substitution activity towards endogenous DNA by cooperation between TALE-scND1n with different TALE recognition sequence distances and TALE-AID. As TALE-AID, TALE47(HEK1-L)-12-AID was used, and as TALE-scND1n, TALE12 (HEK1-R1 to R10)-scND1n was used, respectively.

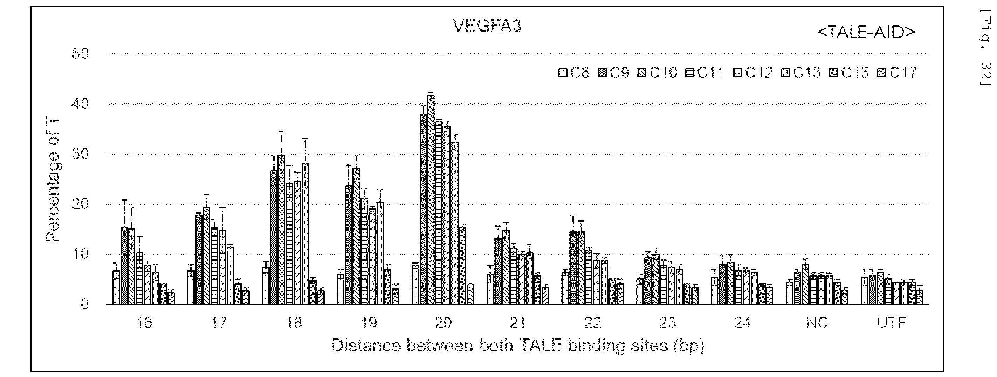

[Fig. 32] Fig. 32 is a graph showing the percentage of T at each C, which is each target base on the VEGFA3 site, analyzed by EditR in the base substitution activity towards endogenous DNA by cooperation between TALE-scND1n with different TALE recognition sequence distances and TALE-AID. As TALE-AID, TALE47(VEGFA3-L)-12-AID was used, and as TALE-scND1n, TALE12 (VEGFA3-R1 to R11)-scND1n was used, respectively.

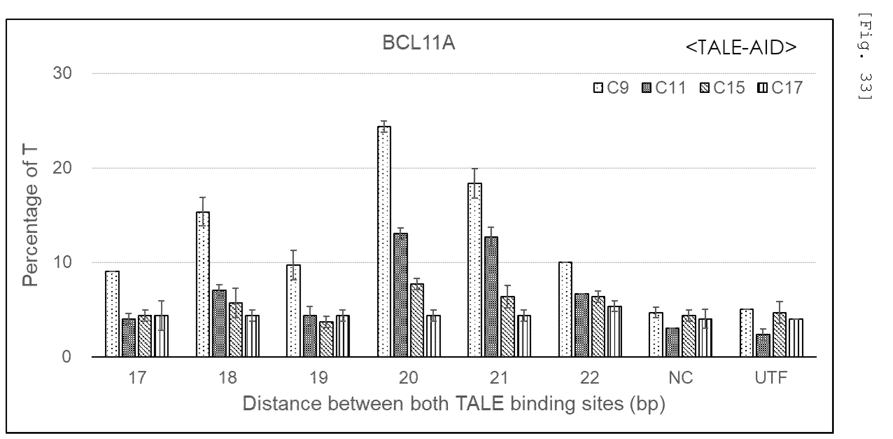

[Fig. 33] Fig. 33 is a graph showing the percentage of T at each C, which is each target base on the BCL11A site, analyzed by EditR in the base substitution activity towards endogenous DNA by cooperation between TALE-scND1n with different TALE recognition sequence distances and TALE-AID. As TALE-AID, TALE47(BCL11A-L)-12-AID was used, and as TALE-scND1n, TALE12 (BCL11A-R1 to R6)-scND1n was used, respectively.

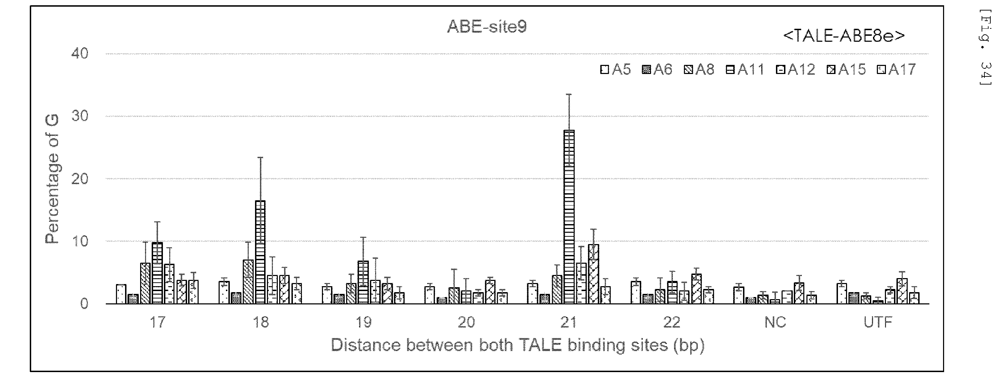

[Fig. 34] Fig. 34 is a graph showing the percentage of G at each A, which is each target base on the ABE-site9 site, analyzed by EditR in the base substitution towards endogenous DNA by cooperation between TALE-scND1n with different inter-TALE distances and TALE-ABE8e. As TALE-ABE8e, TALE47 (ABE-site9-L)-12-ABE8e was used, and as TALE-scND1n, TALE12 (ABE-site9-R1 to R6)-scND1n was used, respectively.

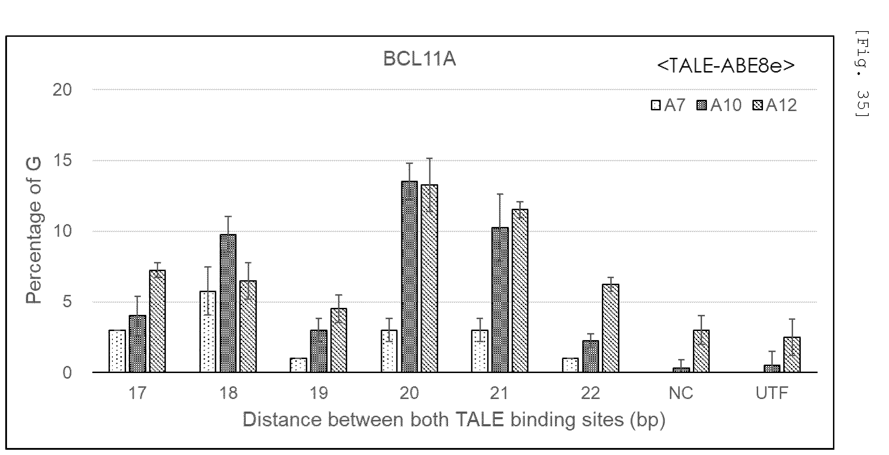

[Fig. 35] Fig. 35 is a graph showing the percentage of G at each A, which is each target base on the BCL11A site, analyzed by EditR in the base substitution towards endogenous DNA by cooperation between TALE-scND1n with different inter-TALE distances and TALE-ABE8e. As TALE-ABE8e, TALE47(BCL11A-L)-12-ABE8e was used, and as TALE-scND1n, TALE12(BCL11A-R1 to R6)-scND1n was used, respectively.

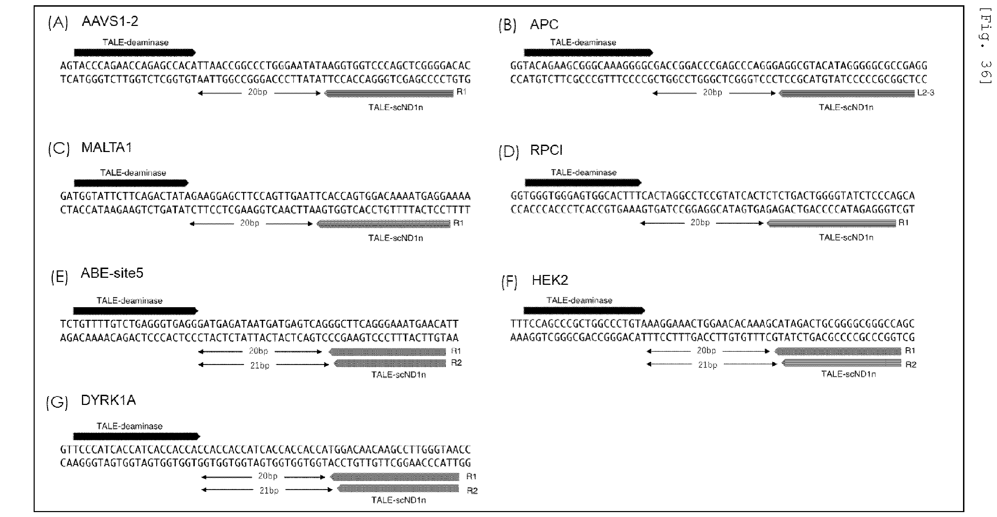

[Fig. 36] Fig. 36 is a conceptual diagram showing the positional relationship between the TALE recognition sequence of TALE-scND1n and the TALE recognition sequence of TALE47-12-AID or TALE47-12-ABE8e on each target site ((A) AAVS1-2, (B) APC, (C) MALTA1, (D) RPCI, (E) ABE-site5, (F) HEK2, (G) DYRK1A).

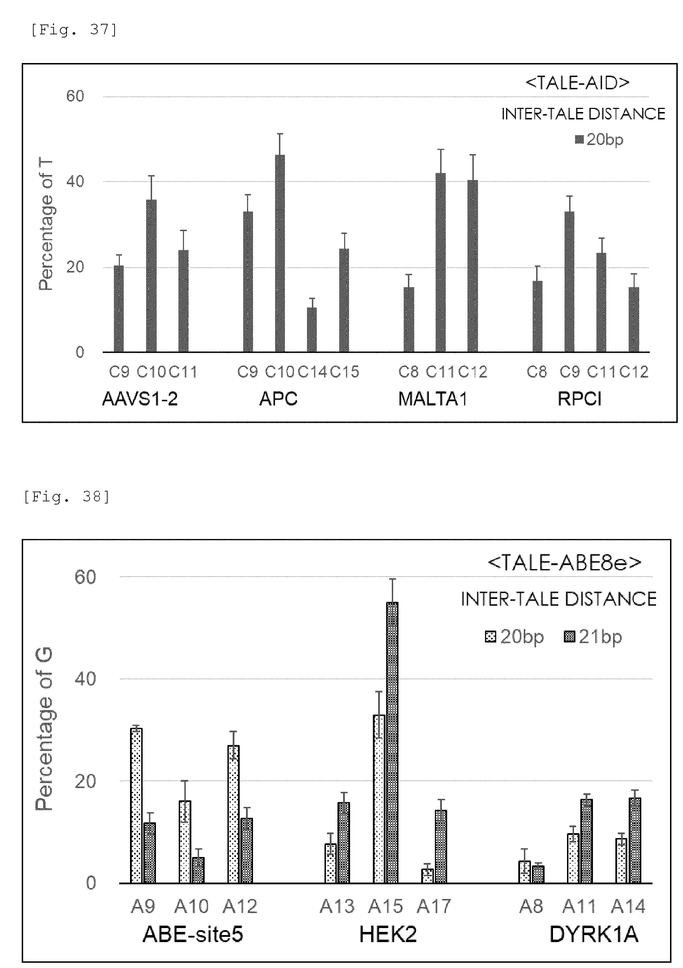

[Fig. 37] Fig. 37 is a graph showing the percentage of T at each C, which is each target base on the AAVS1-2, APC, MALTA1, and RPCI sites, analyzed by EditR in the base substitution activity towards endogenous DNA by cooperation between TALE-scND1n and TALE-AID. At the AAVS1-2 site, TALE47 (AAVS1-2-L)-12-AID was used as TALE-AID, and TALE12(AAVS1-2-R1)-scND1n was used as TALE-scND1n; at the APC site, TALE47 (APC-R3)-12-AID was used as TALE-AID, and TALE12(APC-L2-3)-scND1n was used as TALE-scND1n; at the MALTA1 site, TALE47(MALTA1-L)-12-AID was used as TALE-AID, and TALE12(MALTA1-R1)-scND1n was used as TALE-scND1n; at the RPCI site, TALE47(RPCI-L)-12-AID was used as TALE-AID, and TALE12(RPCI-R1)-scND1n was used as TALE-scND1n, respectively.
[Fig. 38] Fig. 38 is a graph showing the percentage of G at each A, which is each target base on the ABE-site5, HEK2, and DYRK1A sites, analyzed by EditR in the base substitution activity towards endogenous DNA by cooperation between TALE-scND1n and TALE-ABE8e. At the ABE-site5 site, TALE47(ABE-site5-L)-12-ABE8e was used as TALE-ABE8e, and TALE12(ABE-site5-R1)-scND1n or TALE12 (ABE-site5-R2) -scND1n was used as TALE-scND1n; at the HEK2 site, TALE47(HEK2-L)-12-ABE8e was used as TALE-ABE8e, and TALE12(HEK2-R1)-scND1n or TALE12(HEK2-R2)-scND1n was used as TALE-scND1n; at the DYRK1A site, TALE47(DYRK1A-L)-12-ABE8e was used as TALE-ABE8e, and TALE12(DYRK1A-R1)-scND1n or TALE12(DYRK1A-R2)-scND1n was used as TALE-scND1n, respectively.

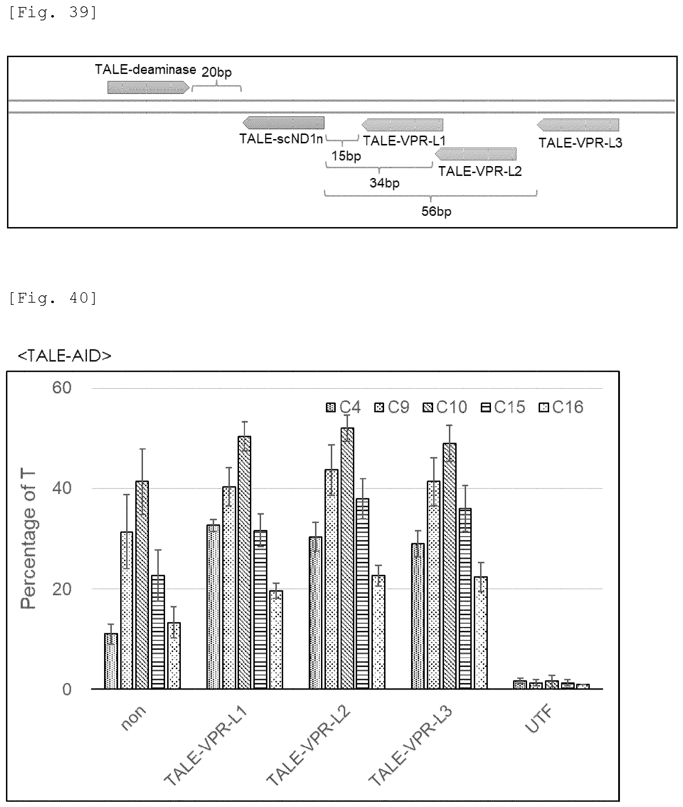

[Fig. 39] Fig. 39 is a conceptual diagram showing the positional relationship on the APC site between the TALE recognition sequence of TALE-deaminase and TALE-scND1n prepared in [Test Example 2] <1. Method> (7) (8), and the TALE recognition sequence of TALE-VPR prepared in [Test Example 2] <1. Method> (9).
[Fig. 40] Fig. 40 is a graph showing the percentage of T at each C, which is each target base on the APC site, analyzed by EditR in the base substitution activity towards endogenous DNA by cooperation among TALE-scND1n, TALE-AID, and TALE-VPR. As TALE-AID, TALE47 (APC-R3)-12-AID was used, as TALE-scND1n, TALE12(APC-L2-3)-scND1n was used, and as TALE-VPR, TALE47(APC-TA-L1 to L3)-12-VPR was used, respectively.

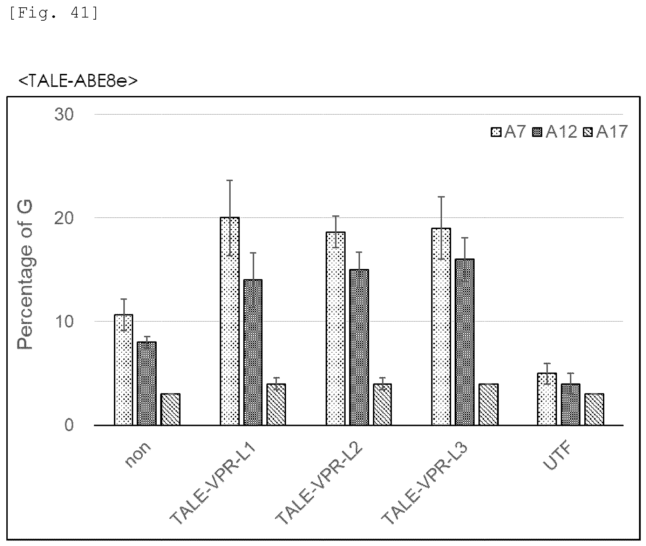

[Fig. 41] Fig. 41 is a graph showing the percentage of G at each A, which is each target base on the APC site, analyzed by EditR in the base substitution activity towards endogenous DNA by cooperation among TALE-scND1n, TALE-ABE8e, and TALE-VPR. As TALE-ABE8e, TALE47 (APC-R3)-12-ABE8e was used, as TALE-scND1n, TALE12(APC-L2-3)-scND1n was used, and as TALE-VPR, TALE47 (APC-TA-L1 to L3)-12-VPR was used, respectively.

### [Description of Embodiments]

Hereinafter, preferred embodiments of the present invention will be described more specifically by way of examples, but the present invention is not limited thereto.

### <Nicking Enzyme>

The nicking enzyme of the present invention is an artificial nicking enzyme comprising two domains ND1 and ND1n linked via an ND1 linker (in this specification, sometimes referred to as "single-chain ND1n" or "scND1n"). The nicking enzyme of the present invention has the activity of cleaving only one strand of double-stranded DNA (nickase activity).

### (ND1)

"ND1" according to the present invention is nuclease domain 1, which is one of the nuclease domains found by the present inventors through screening of homologous sequences whose identity with the FokI nuclease domain exists in the range of 35% to 70% (PTL 2). The amino acid sequence of the full-length protein containing ND1 (as a representative example, derived from Bacillus sp. SGD-V-76) is shown in SEQ ID NO: 97. ND1 is typically (a) a polypeptide comprising an amino acid sequence set forth in SEQ ID NO: 98 (amino acid residues 391 to 585 of the amino acid sequence of SEQ ID NO: 97), more preferably a polypeptide consisting of the amino acid sequence set forth in SEQ ID NO: 98. Note that the amino acid sequence set forth in SEQ ID NO: 98 has 70% identity with the amino acid sequence of the FokI nuclease domain.

"ND1" according to the present invention includes a nuclease domain consisting of an amino acid sequence having high homology with the amino acid sequence set forth in SEQ ID NO: 98, as long as it has double-strand cleavage activity (nuclease activity) of DNA when two molecules are linked via an ND1 linker. Such nuclease domains include, for example, ND1 derived from other bacteria and variants of ND1 (natural variants and artificial variants).

Therefore, embodiments of "ND1" according to the present invention also include (b) an amino acid sequence wherein one or a plurality of amino acids are substituted, deleted, inserted and/or added in the amino acid sequence set forth in SEQ ID NO: 98. However, since it is necessary that it is not the nuclease activity-deficient mutant sequence described below, in (b), it is necessary that at least the amino acids corresponding to the 66th and 83rd positions of the amino acid sequence set forth in SEQ ID NO: 98 are aspartic acid.

Here, in an amino acid sequence, "amino acid sequence wherein substituted, deleted, inserted, and/or added" indicates an amino acid sequence in which amino acids (amino acid residues) within the said amino acid sequence have undergone substitution, deletion, insertion, or addition, or an amino acid sequence in which a combination of two or more of these has been performed. Furthermore, "plurality" indicates an integer of 30, 20, 19, 18, 17, 16, 15, 14, 13, 12, 11, 10, 9, 8, 7, 6, 5, 4, 3, or 2. In the amino acid sequence of the polypeptide of (b), as for the phrase one or a plurality, the number is preferably 1 to 30 amino acid residues, 1 to 20 amino acid residues (for example, 1 to 10, 1 to 5, 1 to 3, 2 or less).

Furthermore, embodiments of "ND1" according to the present invention also include (c) an amino acid sequence having 80% or more homology with the amino acid sequence set forth in SEQ ID NO: 98. However, also in (c), since it is necessary that it is not the nuclease activity-deficient mutant sequence described below, it is necessary that at least the amino acids corresponding to the 66th and 83rd amino acids of the amino acid sequence set forth in SEQ ID NO: 98 are aspartic acid.

Here, in the case of homology of amino acid sequences, when a reference amino acid sequence and a subject amino acid sequence are aligned using amino acid sequence analysis software or the like, an amino acid on the subject amino acid sequence that is at the same position as an amino acid on the reference amino acid sequence (reference amino acid) (subject amino acid) may be the same amino acid as the said reference amino acid, or may be an amino acid having the same properties as the said reference amino acid. In the case of identity of amino acid sequences, the said subject amino acid is the same amino acid as the said reference amino acid. Groups of amino acids having the same properties are well known in the technical field to which the present invention belongs, and for example, they can be classified by acidic amino acids (aspartic acid and glutamic acid); basic amino acids (lysine, arginine, histidine); in neutral amino acids, amino acids having a hydrocarbon chain (glycine, alanine, valine, leucine, isoleucine, proline), amino acids having a hydroxy group (serine, threonine), amino acids containing sulfur (cysteine, methionine), amino acids having an amide group (asparagine, glutamine), amino acids having an imino group (proline), amino acids having an aromatic group (phenylalanine, tyrosine, tryptophan).

Such homology and identity of amino acid sequences are determined by comparing two sequences aligned in a state where sequence matching is maximized. Methods for determining the numerical value (%) of sequence homology or identity are known to those skilled in the art. As algorithms for obtaining optimal alignment and sequence identity, any algorithm known to those skilled in the art (for example, BLAST algorithm, FASTA algorithm, etc.) can be utilized, and the sequence homology or identity of amino acid sequences can be determined using sequence analysis software such as BLASTP, FASTA, etc., for example. Furthermore, in the amino acid sequence of the polypeptide of (c), the homology with the amino acid sequence of (a) may be 80% or more, but is preferably 85% or more, 90% or more, 95% or more (for example, 96% or more, 97% or more, 98% or more, 99% or more). More preferably, by identity, it is 80% or more, 85% or more, 90% or more, 95% or more (for example, 96% or more, 97% or more, 98% or more, 99% or more).

Furthermore, in an amino acid sequence, an amino acid "corresponding to" a specific amino acid indicates an amino acid that is at the same position as the said specific amino acid (the said reference amino acid (amino acid residue), for example, the 66th or 83rd aspartic acid of the amino acid sequence set forth in SEQ ID NO: 98) when amino acid sequences are aligned using amino acid sequence analysis software (for example, GENETYX-MAC, Sequencher, ClustalW, etc.) (for example, parameters: default values (that is, initial setting values)).

### (ND1n)

"ND1n" according to the present invention is a modified domain obtained by modifying the amino acid sequence of ND1 into a mutant sequence in which nuclease activity is deficient (nuclease activity-deficient mutant sequence). Such a nuclease activity-deficient mutant sequence of ND1n is not particularly limited as long as the double-strand cleavage activity of DNA is deficient when linked to ND1 via an ND1 linker, but typically, it is (an) a polypeptide comprising an amino acid sequence which is a substituted amino acid wherein the 66th aspartic acid and/or the 83rd aspartic acid of the amino acid sequence set forth in SEQ ID NO: 98 is substituted with another arbitrary amino acid, more preferably a polypeptide consisting of the said amino acid sequence comprising the said substituted amino acid. As the aspartic acid to be substituted with the said substituted amino acid, either the 66th aspartic acid or the 83rd aspartic acid, or both, may be selected, but it is preferable that at least the 66th aspartic acid is substituted.

Furthermore, "ND1n" according to the present invention also includes variants consisting of an amino acid sequence having high homology with such (an). Therefore, embodiments of "ND1n" according to the present invention include (bn) a polypeptide comprising an amino acid sequence wherein one or a plurality of amino acids are substituted, deleted, inserted and/or added in the amino acid sequence set forth in SEQ ID NO: 98, and wherein the amino acid corresponding to the 66th aspartic acid and/or the 83rd aspartic acid of the amino acid sequence set forth in SEQ ID NO: 98 is a substituted amino acid substituted with an arbitrary amino acid other than aspartic acid; as well as (cn) a polypeptide comprising an amino acid sequence having 80% or more homology with the amino acid sequence set forth in SEQ ID NO: 98, and wherein the amino acid corresponding to the 66th aspartic acid and/or the 83rd aspartic acid of the amino acid sequence set forth in SEQ ID NO: 98 is a substituted amino acid substituted with an arbitrary amino acid other than aspartic acid.

In the amino acid sequence of the polypeptide of (bn), as for the phrase one or a plurality, the number is preferably 1 to 30 amino acid residues, 1 to 20 amino acid residues (for example, 1 to 10, 1 to 5, 1 to 3, 2 or less). Furthermore, in the amino acid sequence of the polypeptide of (cn), the homology with the amino acid sequence of (an) may be 80% or more, but is preferably 85% or more, 90% or more, 95% or more (for example, 96% or more, 97% or more, 98% or more, 99% or more). More preferably, by identity, it is 80% or more, 85% or more, 90% or more, 95% or more (for example, 96% or more, 97% or more, 98% or more, 99% or more).

In (bn) and (cn), as the substituted amino acid substituted with an arbitrary amino acid other than aspartic acid, an amino acid other than the said acidic amino acids is preferable, and alanine or asparagine is more preferable. Furthermore, as the amino acid to be substituted with the said substituted amino acid, the amino acid corresponding to the 66th aspartic acid or the amino acid corresponding to the 83rd aspartic acid, or both, may be selected, but it is preferable that at least the amino acid corresponding to the 66th aspartic acid is substituted.

As a method for substituting the amino acid corresponding to the 66th aspartic acid and/or the 83rd aspartic acid with the said substituted amino acid, conventionally known methods or methods according thereto can be appropriately adopted, for example, known methods such as site-directed mutagenesis (Kunkel et al. (Proc. Natl. Acad. Sci. USA (1985) 82, p. 488-492)) and overlap extension PCR can be appropriately adopted. Furthermore, primers and the like used for modification of the said amino acid can be appropriately designed by conventionally known methods or methods according thereto, based on the target amino acid sequence (amino acid sequence set forth in SEQ ID NO: 98, etc.) and the target modification.

Note that in the present invention, whether a given domain, ND1 or ND1n, possesses or lacks DNA double-strand cleavage activity (nuclease activity) when linked to ND1 via an ND1 linker can be appropriately confirmed by methods known to those skilled in the art. For example, as described in the Test Examples below, it can be confirmed by expressing in cells a fusion construct in which "the following TALE -> the domain of interest -> the following ND1 linker -> the amino acid sequence set forth in SEQ ID NO: 98 (ND1)" are linked in this order from the N-terminal side, and determining whether or not the target DNA corresponding to the TALE undergoes double-strand cleavage. In this method, an SSA assay system targeting a reporter gene on a plasmid (for example, an EGFP gene into which a TALE recognition sequence has been inserted) may be used, and evaluation may be performed using reporter activity as an indicator. For example, if the nuclease activity when using the said fusion construct is 50% or more of the nuclease activity when expressing a construct linked in the order "TALE -> amino acid sequence set forth in SEQ ID NO: 98 (ND1) - > ND1 linker -> amino acid sequence set forth in SEQ ID NO: 98 (ND1)" from the N-terminal side, it can be judged that it has nuclease activity, and if it is less than 50%, it can be judged that it is deficient in nuclease activity.

### (ND1 Linker)

The "ND1 linker" according to the present invention is a polypeptide having the function of linking the above two domains ND1 and ND1n. Regarding the chain length of such an ND1 linker, as long as the nicking enzyme of the present invention can exert nickase activity, there is no particular limitation in its chain length or type, and it is usually 30 to 300 amino acid residues, but preferably 50 to 200 amino acid residues, more preferably 70 to 100 amino acid residues.

Regarding the type of ND1 linker according to the present invention, there is no particular limitation as long as the nicking enzyme of the present invention can exert nickase activity, but for example, HTS95 linker consisting of the HTS95 amino acid sequence described in Sun, N., & Zhao, H. (2014) Molecular BioSystems, 10(3), p. 446-453 (amino acid residues 197 to 292 of the amino acid sequence of SEQ ID NO: 61), GSS linker consisting of GSS or repeats thereof, SAGG linker consisting of SAGG (SEQ ID NO: 183) or repeats thereof, GGGGS linker consisting of GGGGS (SEQ ID NO: 99) or repeats thereof, XTEN linker (SEQ ID NO: 184), etc., can be mentioned, and the HTS95 linker is preferable.

In the nicking enzyme of the present invention, ND1 and ND1n may be located on either the N-terminal side or the C-terminal side via the ND1 linker, but when formed into a fusion protein with TALE, which is the DNA binding protein described below, from the viewpoint of exhibiting particularly specific nickase activity for the DNA strand, that is, excellent specific activity for the TALE recognition strand, it is preferable that they are linked in the order of ND1n - > ND1 linker -> ND1 from the N-terminal side.

In the nicking enzyme of the present invention, the linkage between ND1 and ND1n and the ND1 linker can be performed at the nucleic acid level and the amino acid level. That is, by linking the respective polynucleotides by ligation reaction such that the order becomes "polynucleotide encoding ND1n -> polynucleotide encoding ND1 linker -> polynucleotide encoding ND1" or "polynucleotide encoding ND1 -> polynucleotide encoding ND1 linker -> polynucleotide encoding ND1n," a polynucleotide encoding the nicking enzyme of the present invention can be prepared. By inserting the polynucleotide thus obtained into an expression vector and expressing it in a suitable host cell, the nicking enzyme of the present invention linked at the amino acid level in the order of "ND1n -> ND1 linker -> ND1" or "ND1 -> ND1 linker -> ND1n" can be obtained.

As the said expression vector, it can be appropriately selected from vectors used in the relevant field, and examples include plasmid vectors, viral vectors, phage vectors, phagemid vectors, BAC vectors, YAC vectors, MAC vectors, HAC vectors. As the host cell into which the said expression vector is introduced, it can be appropriately selected considering compatibility with the expression vector, and examples include prokaryotes such as Escherichia coli, Actinomycetes, Archaea, and cells of eukaryotes such as yeast, sea urchin, silkworm, zebrafish, mouse, rat, frog, tobacco, Arabidopsis thaliana, rice. Note that "polynucleotide" encompasses both DNA and RNA (mRNA), and in each polynucleotide, codon optimization may be performed for purposes such as enhancing intracellular expression efficiency.

Furthermore, the nicking enzyme of the present invention can also be prepared by artificial synthesis based on amino acid sequence information. Furthermore, the nicking enzyme of the present invention may have epitope tags for purification or detection (for example, FLAG tag, HA tag, etc.) or various trafficking signals (for example, nuclear localization signal, plastid transit signal, etc.) added.

### <First Fusion Protein>

The fusion protein of the present invention is a fusion protein comprising a first DNA binding domain and the above-described nicking enzyme of the present invention (in this specification, sometimes referred to as "first fusion protein"). The first fusion protein of the present invention binds to the vicinity of a target site on target DNA (first DNA binding domain recognition sequence) via the first DNA binding domain, and cleaves one strand of the double-stranded DNA (introduces a nick) by the nicking enzyme, functioning as a site-specific nicking enzyme. The said nicking enzyme, including its preferred embodiments, is as described above.

### (First DNA Binding Domain)

The "first DNA binding domain" according to the present invention is not particularly limited as long as it is a protein domain capable of specifically binding to an arbitrary DNA sequence (first DNA binding domain recognition sequence). Examples of the first DNA binding domain include TALE, zinc finger array (ZF), PPR protein (Pentatricopeptide Repeat Protein), and at least one selected from the group consisting of TALE and zinc finger array (ZF) is preferable, and TALE is more preferable.

### [TALE]

TALE (Transcription activator-like effector nuclease) is typically a protein secreted by proteobacteria of the genus Xanthomonas that activates gene transcription in host plants, and is a general term including TALE-like proteins possessed by bacteria of the genus Ralstonia. "TALE" according to the present invention includes at least an N-terminal domain and a TALE repeat domain, and may further include a C-terminal domain.

The TALE repeat domain is composed of multiple, for example 10 to 30, preferably 13 to 25, more preferably 15 to 20, tandem repeats (TALE repeats) of the TALE sequence that form a right-handed superhelix. One typical TALE repeat unit (one TALE sequence) consists of 33 to 35 amino acids, and recognizes a specific base of DNA by a variable residue (repeat variable diresidue: RVD) consisting of the 12th and 13th two amino acid residues. Examples of RVDs that specifically recognize bases include HD which recognizes C, NG which recognizes T, NI which recognizes A, NN which recognizes G or A, and NS which recognizes A, C, G or T. Based on the DNA recognition mechanism of such TALE repeat domains, by artificially linking TALE sequences that recognize specific bases, it is possible to create a TALE that can recognize and bind to a desired nucleotide sequence (TALE recognition sequence) on DNA.

The said TALE sequences of TALE according to the present invention may each be appropriately modified with respect to the wild-type amino acid sequence, as long as the TALE repeat domain can recognize and bind to the TALE recognition sequence. For example, in the said TALE sequence, independently in each, the position of the RVD may be changed, one or a plurality (for example, 10 or less, 9 or less, 8 or less, 7 or less, 6 or less, preferably 5 or less or 4 or less, more preferably 3 or less or 2 or less) of amino acid residues other than RVD may be substituted, deleted, inserted and/or added, and the 12th and 13th two amino acid residues of RVD may be substituted with other amino acid residues to enhance specificity for A, T, C and G.

The TALE repeat domain of TALE according to the present invention, when used in the DNA editing system described below, is designed such that it recognizes a nucleotide sequence or its complementary sequence present via a first spacer of 4 to 16 bases in length on the 5' side or 3' side of the target site of the target DNA, that is, the TALE recognition sequence recognized by TALE or its complementary sequence becomes a nucleotide sequence present via a first spacer of 4 to 16 bases in length on the 5' side or 3' side of the said target site. Technology for designing and producing such a desired TALE is known; for example, a TALE having high binding activity to the said nucleotide sequence can be produced by techniques described in Miller et al., Nat Biotechnol 29 (2011) 143-148; Sakuma et al., Sci Rep 3, 3379 (2013); or by the Platinum Gate system described in the aforementioned Sakuma et al. (2013) .

As the N-terminal domain of TALE according to the present invention, a wild-type amino acid sequence (for example, the amino acid sequence of the N-terminal domain contained in Addgene's pTALETF_v2 (ID: 32185-32188)) can be used, but as long as it does not negatively affect the function of the first fusion protein of the present invention (binding ability to the TALE recognition sequence, etc.), it may be appropriately modified with respect to the said wild-type amino acid sequence. For example, it may be one in which one or a plurality (for example, 50 or less, 30 or less, 20 or less, 10 or less, 9 or less, 8 or less, 7 or less, 6 or less, preferably 5 or less or 4 or less, more preferably 3 or less or 2 or less) of amino acid residues are substituted, deleted, inserted and/or added. Furthermore, it may contain a FLAG tag for purification or detection, a nuclear localization signal (NLS) for translocating the TALE-nicking enzyme to the cell nucleus, etc.

The chain length of such an N-terminal domain is preferably 49 to 287 amino acid residues, more preferably 80 to 200 amino acid residues, still more preferably 120 to 180 amino acid residues.

Specific examples of the N-terminal domain of TALE according to the present invention include, in addition to the above, for example, the amino acid sequence of the N-terminal domain contained in Addgene's ptCMV-136/63-VR-HD (ID: 50699) and the amino acid sequence of the N-terminal domain contained in Addgene's ptCMV-153/47-VR-HD (ID: 50703), but are not limited thereto.

When TALE according to the present invention further includes a C-terminal domain, as such a C-terminal domain of TALE, a wild-type amino acid sequence (for example, the amino acid sequence of the C-terminal domain contained in Addgene's pTALETF_v2 (ID: 32185-32188) (WT: number of amino acid residues = 180)) can be used, but as long as it does not negatively affect the function of the first fusion protein of the present invention (binding ability to the TALE recognition sequence, etc.), it may be appropriately modified with respect to the said wild-type amino acid sequence. For example, it may be one in which one or a plurality (for example, 50 or less, 30 or less, 20 or less, 10 or less, 9 or less, 8 or less, 7 or less, 6 or less, preferably 5 or less or 4 or less, more preferably 3 or less or 2 or less) of amino acid residues are substituted, deleted, inserted and/or added.

When TALE according to the present invention further includes a C-terminal domain, the C-terminal domain of the said TALE may be one in which a part of the amino acid sequence on the C-terminal side in the wild-type amino acid sequence has been removed. As such a C-terminal domain, the chain length of the C-terminal domain is preferably 1 to 200 amino acid residues, more preferably 10 to 200 amino acid residues, still more preferably 10 to 190 amino acid residues, even more preferably 20 to 180 amino acid residues.

Specific examples of the said C-terminal domain include, in addition to the above, for example, the amino acid sequence of the C-terminal domain contained in Addgene's pTALEN_v2 (ID: 32189-32192) (number of amino acid residues = 63), the amino acid sequence of the C-terminal domain contained in Addgene's ptCMV-153/47-VR-NG (ID: 50704) (number of amino acid residues = 47), but are not limited thereto.

As a preferred embodiment of TALE according to the present invention, the embodiment of TALE of Platinum TALEN (Japanese Unexamined Patent Application Publication No. 2015-33365), in which amino acids at two specific positions of one TALE repeat unit change every four TALE repeat units, is mentioned as preferable.

### [Zinc Finger Array]

The zinc finger array according to the present invention refers to the DNA binding region among zinc finger nucleases (ZFN) containing a DNA binding region and a nuclease domain. A zinc finger array usually consists of 2 to 15, preferably 3 to 8, more preferably 4 to 6 zinc finger proteins, and each zinc finger protein is linked directly or via a linker. Each zinc finger protein contains one or more zinc atoms and a helix structure that recognizes specific bases, and one zinc finger protein recognizes specific three bases of DNA.

The zinc finger array according to the present invention is not particularly limited, and may be appropriately modified, as long as each array can recognize and bind to the DNA binding domain recognition sequence. For example, it may be one in which one or a plurality (for example, 50 or less, 30 or less, 20 or less, 10 or less, 9 or less, 8 or less, 7 or less, 6 or less, preferably 5 or less or 4 or less, more preferably 3 or less or 2 or less) of amino acid residues are substituted, deleted, inserted and/or added.

The zinc finger array according to the present invention, when used in the DNA editing system described below, is designed such that it recognizes a nucleotide sequence or its complementary sequence present via a first spacer of 4 to 16 bases in length on the 5' side or 3' side of the target site of the target DNA, that is, the zinc finger array recognition sequence recognized by the zinc finger array or its complementary sequence becomes a nucleotide sequence present via a first spacer of 4 to 16 bases in length on the 5' side or 3' side of the said target site. Technology for designing and producing such a zinc finger array is known, and for example, it can be appropriately designed and produced by referring to the methods described in Beerli et al., Nat Biotechnol 20, 135-141 (2002), Sander et al., Nat Methods 8, 67-69 (2011), etc.

### (First Linker)

In the first fusion protein of the present invention, the nicking enzyme and the first DNA binding domain may be directly linked, or may be linked via a linker. When a linker is present, this is sometimes referred to as the "first linker" in this specification. As the first linker, there is no particular limitation in its chain length or type, as long as it does not inhibit the effects of the present invention (binding ability to the first DNA binding domain recognition sequence, nickase activity of the nicking enzyme, etc.). The chain length of the first linker is usually 2 to 180 amino acid residues, preferably 2 to 120 amino acid residues. For example, when the said first DNA binding domain is TALE, the distance between the TALE repeat domain and the said nicking enzyme is such that the chain length of the first linker can be adjusted so that the distance, including the chain length of the N-terminal domain of TALE (in the case of the order from the N-terminal side: nicking enzyme -> first DNA binding domain) or the C-terminal domain (in the case of the order from the N-terminal side: first DNA binding domain -> nicking enzyme), more preferably the C-terminal domain, is 1 to 200 amino acid residues, preferably 10 to 200 amino acid residues, more preferably 10 to 190 amino acid residues, still more preferably 20 to 180 amino acid residues. In this case, the distance between the TALE repeat domain and the said nicking enzyme is, in the case of the order from the N-terminal side: first DNA binding domain -> nicking enzyme, the number of amino acid residues from the amino acid residue adjacent to the C-terminus on the C-terminal side of the TALE repeat domain as the 1st residue, up to the residue adjacent to the N-terminus on the N-terminal side of the nicking enzyme, and in the case of the order from the N-terminal side: nicking enzyme -> first DNA binding domain, it is the number of amino acid residues from the amino acid residue adjacent to the N-terminus on the N-terminal side of the TALE repeat domain as the 1st residue, up to the residue adjacent to the C-terminus on the C-terminal side of the nicking enzyme. The type of the first linker according to the present invention is not particularly limited, and for example, those mentioned as ND1 linkers may be appropriately used.

In the first fusion protein, the nicking enzyme and the first DNA binding domain may be located on either the N-terminal side or the C-terminal side, optionally via the first linker. Furthermore, the first DNA binding domain may be arranged at both ends of the nicking enzyme to form a sandwich type (for the sandwich type, refer to Mori et al (2009) Biochemical and Biophysical Research Communications, 390(3), 694-697). Among these, as the first fusion protein of the present invention, from the viewpoint of exhibiting particularly specific nickase activity for the DNA strand, that is, excellent specific activity for the TALE recognition strand, when combined with the second fusion protein described below, it is preferable that they are linked in the order of first DNA binding domain -> (optionally first linker) -> nicking enzyme from the N-terminal side.

In the first fusion protein, the linkage between the nicking enzyme and the first DNA binding domain can be performed at the nucleic acid level and the amino acid level. That is, by linking the respective polynucleotides by ligation reaction such that the order becomes "polynucleotide encoding nicking enzyme -> polynucleotide encoding first DNA binding domain" or "polynucleotide encoding first DNA binding domain -> polynucleotide encoding nicking enzyme," a polynucleotide encoding the first fusion protein of the present invention can be prepared. By inserting the polynucleotide thus obtained into an expression vector and expressing it in a suitable host cell, the first fusion protein of the present invention linked at the amino acid level in the order of "nicking enzyme -> first DNA binding domain" or "first DNA binding domain -> nicking enzyme" can be obtained. The said expression vector and host cell are the same as those mentioned for the nicking enzyme of the present invention.

Furthermore, the first fusion protein of the present invention can also be prepared by artificial synthesis based on amino acid sequence information. Furthermore, the first fusion protein of the present invention may have epitope tags for purification or detection (for example, FLAG tag, HA tag, etc.) or various trafficking signals (for example, nuclear localization signal, plastid transit signal, etc.) added.

### <DNA Editing System>

The DNA editing system of the present invention comprises the above-described first fusion protein of the present invention and a second fusion protein comprising a second DNA binding domain and a nucleic acid base converting enzyme. The first fusion protein, including its preferred embodiments, is as described above. Furthermore, as the DNA editing system of the present invention, from the viewpoint of promoting base conversion by cooperation between the first fusion protein and the second fusion protein, it is also preferable to further comprise a third fusion protein comprising a third DNA binding domain and a transcription regulatory factor.

### (Second Fusion Protein)

The second fusion protein according to the present invention is a fusion protein comprising a second DNA binding domain and a nucleic acid base converting enzyme. Examples of the second DNA binding domain according to the present invention, including its preferred embodiments, include those described for the first DNA binding domain. When the first DNA binding domain is TALE, the second DNA binding domain is preferably at least one selected from the group consisting of TALE and zinc finger array, and more preferably TALE.

### [Nucleic Acid Base Converting Enzyme]

In the present invention, a nucleic acid base converting enzyme means an enzyme capable of converting a target base to another base without cleaving the DNA strand, by catalyzing a reaction that converts a substituent on the purine or pyrimidine ring of a DNA base to another group or atom, or causes its removal.

Such a nucleic acid base converting enzyme is not particularly limited as long as it can catalyze the above reaction, and examples include deaminase and glycosylase, and it may be only one type of these or a combination of two or more types. Among these, as the nucleic acid base converting enzyme according to the present invention, deaminase is preferable.

The said deaminase is an enzyme that catalyzes a deamination reaction converting an amino group of a base to a carbonyl group, and belongs to the nucleic acid/nucleotide deaminase superfamily. Examples of such deaminases include cytidine deaminase capable of substituting cytosine or 5-methylcytosine with uracil or thymine, respectively, adenosine deaminase capable of substituting adenine with hypoxanthine, and guanosine deaminase capable of substituting guanine with xanthine, and they can be used appropriately according to the target base substitution.

The origin of the said deaminase is not particularly limited, and examples include lamprey; mammals such as human, monkey, pig, cow, horse, rat, mouse. As such deaminases, examples of cytidine deaminase include APOBEC (rAPOBEC1 derived from rat, hAPOBEC1, hAPOBEC2, hAPOBEC3 (hAPOBEC3A, 3B, 3C, 3D (3E), 3F, 3G, 3H), hAPOBEC4 derived from human, etc.); Anc689 which is the ancestral amino acid sequence of APOBEC; AID (Activation-induced cytidine deaminase (AICDA)) derived from mammals (e.g., human, pig, cow, horse, monkey, etc.); PmCDA1 (Petromyzon marinus cytosine deaminase 1) derived from lamprey, which is a family of AID. Furthermore, examples of adenosine deaminase include TadA derived from Escherichia coli. Each of the above deaminases includes their respective variants (for example, TadA-8e, TadA7.10, and further variants thereof, etc.). Among these, the said deaminase is preferably at least one selected from the group consisting of APOBEC, PmCDA1, Anc689, and TadA (each including its variants).

Furthermore, when the second fusion protein according to the present invention comprises two or more deaminases, the combination thereof can be appropriately selected according to the target base substitution, for example, a combination of the same or different cytidine deaminases, a combination of the same or different adenosine deaminases, a combination of the same or different guanosine deaminases, a combination of cytidine deaminase and adenosine deaminase, a combination of cytidine deaminase and guanosine deaminase, a combination of adenosine deaminase and guanosine deaminase can be mentioned. Among these, as the combination of deaminases, a combination of TadA and PmCDA1 is preferable.

Note that the nucleotide sequences and amino acid sequences of these deaminases are known and can be obtained from public databases (Genbank, etc.). Furthermore, the said deaminase may be one that has been appropriately modified (for example, introduction of substitution, deletion, insertion and/or addition of amino acid residues) based on these known nucleotide sequences and amino acid sequences of deaminases, as long as it has deamination activity (deaminase activity).

### [Second Linker]

In the second fusion protein according to the present invention, the nucleic acid base converting enzyme and the second DNA binding domain may be directly linked, or may be linked via a linker. When a linker is present, this is sometimes referred to as the "second linker" in this specification. As the second linker, there is no particular limitation in its chain length or type, as long as it does not inhibit the effects of the present invention (binding ability to the second DNA binding domain recognition sequence, nucleic acid base converting activity, nickase activity of the first fusion protein, etc.). Examples of such a second linker, including its preferred embodiments, include linker 1 described in International Publication No. WO 2022/050377. For example, when the second DNA binding domain is TALE, in the second fusion protein, the chain length of the second linker can be adjusted so that the distance between the TALE repeat domain and the said nucleic acid base converting enzyme, including the chain length of the N-terminal domain or C-terminal domain (more preferably the C-terminal domain) of TALE, is 1 to 630 amino acid residues. In this case, the distance between the TALE repeat domain and the said nucleic acid base converting enzyme (the number of amino acid residues from the amino acid residue adjacent to the C-terminus on the C-terminal side (or the amino acid residue adjacent to the N-terminus on the N-terminal side) of the TALE repeat domain as the 1st residue, up to the residue adjacent to the N-terminus on the N-terminal side (or the residue adjacent to the C-terminus on the C-terminal side) of the nucleic acid base converting enzyme) is more preferably 10 to 500 amino acid residues, still more preferably 25 to 300 amino acid residues, even more preferably 52 to 174 amino acid residues.

Furthermore, the second fusion protein may comprise two or more (for example, two) nucleic acid base converting enzymes, and in this case, the nucleic acid base converting enzyme may be only one type or a combination of two or more types. For example, when the second fusion protein according to the present invention comprises two nucleic acid base converting enzymes (first nucleic acid base converting enzyme, second nucleic acid base converting enzyme), these may be linked in the order of first nucleic acid base converting enzyme, second DNA binding domain, second nucleic acid base converting enzyme. Among these, the second fusion protein according to the present invention preferably comprises one nucleic acid base converting enzyme, and is preferably linked in the order of second DNA binding domain -> (optionally second linker) -> nucleic acid base converting enzyme from the N-terminal side, or linked in the order of nucleic acid base converting enzyme -> (optionally second linker) - > second DNA binding domain from the N-terminal side, and more preferably linked in the order of second DNA binding domain -> (optionally second linker) -> nucleic acid base converting enzyme from the N-terminal side.

Furthermore, the second fusion protein according to the present invention preferably comprises a base excision repair inhibitor linked via a linker on the C-terminal side, that is, the C-terminus of the said nucleic acid base converting enzyme (when the said nucleic acid base converting enzyme is located on the C-terminal side of the second DNA binding domain) or the C-terminus of the second DNA binding domain (when the said nucleic acid base converting enzyme is located only on the N-terminal side of the second DNA binding domain). Examples of such a linker and base excision repair inhibitor, including their preferred embodiments, include linker 2 and the base excision repair inhibitor described in International Publication No. WO 2022/050377.

In the second fusion protein, the linkage between the nucleic acid base converting enzyme and the second DNA binding domain can be performed at the nucleic acid level and the amino acid level, and the method thereof is the same as the linkage between the nicking enzyme and the first DNA binding domain in the first fusion protein described above.

Furthermore, the second fusion protein can also be prepared by artificial synthesis based on amino acid sequence information. Furthermore, the second fusion protein of the present invention may have epitope tags for purification or detection (for example, FLAG tag, HA tag, etc.) or various trafficking signals (for example, nuclear localization signal, plastid transit signal, etc.) added.

### (Third Fusion Protein)

The third fusion protein according to the present invention is a fusion protein comprising a third DNA binding domain and a transcription regulatory factor. Examples of the third DNA binding domain according to the present invention, including its preferred embodiments, include those described for the first DNA binding domain. When the first DNA binding domain is TALE, the third DNA binding domain is preferably at least one selected from the group consisting of TALE and zinc finger array, and more preferably TALE.

### [Transcription Regulatory Factor]

In the present invention, a transcription regulatory factor refers to a factor that binds to an enhancer of DNA and regulates its transcription, and is more preferably a transcription activator that promotes transcription. Examples of such transcription regulatory factors include VPR, Rta, p65, Hsf1, TCF4, MEF2A, MEF2C, MEF2D, p53, E2F1, VP16, VP64, VP128, VP160, and it may be one type of these or a combination of two or more types. The nucleotide sequences and amino acid sequences of these transcription regulatory factors are known and can be obtained from public databases (Genbank, etc.).

### [Third Linker]

In the third fusion protein according to the present invention, the transcription regulatory factor and the third DNA binding domain may be directly linked, or may be linked via a linker. When a linker is present, this is sometimes referred to as the "third linker" in this specification. As the third linker, there is no particular limitation in its chain length or type, as long as it does not inhibit the effects of the present invention (binding ability to the third DNA binding domain recognition sequence, nucleic acid base converting activity, nickase activity of the first fusion protein, etc.). For example, when the third DNA binding domain is TALE, in the third fusion protein, the chain length of the third linker can be adjusted so that the distance between the TALE repeat domain and the said transcription regulatory factor, including the chain length of the N-terminal domain or C-terminal domain (more preferably the C-terminal domain) of TALE, is 1 to 630 amino acid residues. In this case, the distance between the TALE repeat domain and the said transcription regulatory factor (the number of amino acid residues from the amino acid residue adjacent to the C-terminus on the C-terminal side (or the amino acid residue adjacent to the N-terminus on the N-terminal side) of the TALE repeat domain as the 1st residue, up to the residue adjacent to the N-terminus on the N-terminal side (or the residue adjacent to the C-terminus on the C-terminal side) of the transcription regulatory factor) is usually 10 to 500 amino acid residues, preferably 25 to 300 amino acid residues, and more preferably 52 to 174 amino acid residues. The type of the third linker according to the present invention is not particularly limited, and for example, those mentioned as the second linker may be appropriately used.

In the third fusion protein according to the present invention, the transcription regulatory factor and the third DNA binding domain may be located on either the N-terminal side or the C-terminal side, optionally via the third linker. Furthermore, in the third fusion protein, the linkage between the transcription regulatory factor and the third DNA binding domain can be performed at the nucleic acid level and the amino acid level, and the methods thereof are the same as the linkage between the nicking enzyme and the first DNA binding domain in the first fusion protein described above. Furthermore, the third fusion protein according to the present invention may have epitope tags for purification or detection (for example, FLAG tag, HA tag, etc.) or various trafficking signals (for example, nuclear localization signal, plastid transit signal, etc.) added.

In the DNA editing system of the present invention, the first fusion protein, the second fusion protein, and the third fusion protein may each independently be in the form of a protein, in the form of a polynucleotide (DNA, RNA) encoding the said protein, or in the form of a vector (expression vector) that expresses the said protein. Furthermore, the DNA editing system of the present invention may be a composition comprising two or more of the first fusion protein, the second fusion protein, and optionally the third fusion protein, a combination thereof, or a kit comprising the said combination.

### <Method for Editing Target DNA>

The method for editing target DNA of the present invention comprises:
a step of bringing the DNA editing system of the present invention described above into contact with target DNA and editing a base at a target site of the target DNA by the said nucleic acid base converting enzyme activity, wherein
the distance between a first DNA binding domain recognition sequence recognized by the first DNA binding domain or its complementary sequence and a second DNA binding domain recognition sequence recognized by the second DNA binding domain or its complementary sequence is 8 to 48 bases.

Furthermore, as the method for editing target DNA of the present invention, it is also more preferable that
the first DNA binding domain recognition sequence recognized by the first DNA binding domain or its complementary sequence is present via a first spacer of 4 to 16 bases on the 5' side or the 3' side of the target site, and
the second DNA binding domain recognition sequence recognized by the second DNA binding domain or its complementary sequence is present via a second spacer of 3 to 31 bases on the side opposite to the first DNA binding domain recognition sequence or its complementary sequence of the target site.

Furthermore, when the DNA editing system of the present invention further comprises a third fusion protein, as the method for editing target DNA of the present invention, it is preferable that a third DNA binding domain recognition sequence recognized by the third DNA binding domain is present via a third spacer of 1 to 500 bases on the 5' side or 3' side of the first DNA binding domain recognition sequence opposite to the target site.

### (Target DNA)

In the present invention, DNA containing a target site that is the subject of the intended DNA editing is referred to as "target DNA". The target DNA according to the present invention is double-stranded DNA, and to indicate the correspondence with the DNA editing system described above, for convenience, it is assumed to be either a structure (structure 1) wherein at least one strand comprises, in order from the 5' side, a first DNA binding domain recognition sequence or its complementary sequence, a first spacer (sometimes referred to as "spacer 1"), the said target site, a second spacer (sometimes referred to as "spacer 2"), and a second DNA binding domain recognition sequence or its complementary sequence, or a structure (structure 2) wherein at least one strand comprises, in order from the 5' side, a second DNA binding domain recognition sequence or its complementary sequence, spacer 2, the said target site, spacer 1, and a first DNA binding domain recognition sequence or its complementary sequence. Furthermore, when the DNA editing system of the present invention further comprises a third fusion protein, it is assumed to be a structure (structure 3) comprising a third DNA binding domain recognition sequence via a third spacer (sometimes referred to as "spacer 3") on the 5' side or 3' side of the first DNA binding domain recognition sequence opposite to the target site.

"Comprising the complementary sequence of the DNA binding domain recognition sequence" on the 5' side or 3' side, or on the side opposite to the target site indicates that the complementary strand of the said strand comprises the DNA binding domain recognition sequence. That is, the first DNA binding domain recognition sequence and the second DNA binding domain recognition sequence, and optionally the third DNA binding domain recognition sequence, may each independently be set on the same strand as the said target site, or may be set on its complementary strand, and the first DNA binding domain recognition sequence and the second DNA binding domain recognition sequence may be set on the same strand, or may be set on mutually opposite strands. However, it is preferable that the first DNA binding domain recognition sequence and the third DNA binding domain recognition sequence are on the same strand.

The target site according to the present invention refers to one base targeted for editing by the said nucleic acid base converting enzyme (preferably deamination by deaminase). However, this does not deny that bases in the vicinity on both sides of the said one base (especially when a base targeted for deamination by deaminase exists; preferably, bases located between the total spacer lengths described below, more preferably 1 to 10 bases each on the 5' side and 3' side of the target site, still more preferably 1 to 5 bases, even more preferably 1 to 2 bases) are further edited (in the case of deaminase, deaminated).

The first DNA binding domain recognition sequence, the second DNA binding domain recognition sequence, and the third DNA binding domain recognition sequence are sequences recognized by the first DNA binding domain, the second DNA binding domain, and the third DNA binding domain, respectively. The number of bases of each DNA binding domain recognition sequence, when each said DNA binding domain is TALE, is preferably 10 to 30 bases, preferably 13 to 25 bases, more preferably 15 to 22 bases, independently for each. Furthermore, when each said DNA binding domain is a zinc finger array, it is preferably 6 to 45 bases, preferably 9 to 24 bases, more preferably 12 to 18 bases, independently for each.

The first DNA binding domain recognition sequence is preferably selected such that it is present via spacer 1 of 4 to 16 bases in length on the 5' side or 3' side of the said target site. When the chain length of spacer 1 is within such a range, there is a tendency for the one base at the said target site to be substituted with higher specificity and efficiency. The chain length of the said spacer 1 is the length from the base adjacent to the 5' side or 3' side of the base of the target site as the 1st base, up to the base adjacent to the 3' side or 5' side of the first DNA binding domain recognition sequence or its complementary sequence. The chain length of such spacer 1 is more preferably 5 to 14 bases, still more preferably 6 to 13 bases, even more preferably 8 to 11 bases.

The second DNA binding domain recognition sequence is preferably selected such that it is present via spacer 2 of 3 to 31 bases in length on the side opposite to the first DNA binding domain recognition sequence of the said target site. When the chain length of spacer 2 is within such a range, there is a tendency for the one base at the said target site to be substituted with higher specificity and efficiency. The chain length of the said spacer 2 is the length from the base adjacent to the 5' side or 3' side of the base of the target site as the 1st base, up to the base adjacent to the 3' side or 5' side of the second DNA binding domain recognition sequence or its complementary sequence. The chain length of such spacer 2 is more preferably 5 to 25 bases or 7 to 31 bases, still more preferably 7 to 19 bases, even more preferably 8 to 15 bases.

The chain length of spacer 1 and the chain length of spacer 2 can be arbitrarily combined, respectively, as long as they satisfy the condition of the total spacer length described below, but for example, combinations of chain length of spacer 1 / chain length of spacer 2 of 4 to 16 bases / 3 to 31 bases, 5 to 14 bases / 5 to 25 bases, 6 to 13 bases / 7 to 19 bases, 8 to 11 bases / 8 to 15 bases are preferable.

Furthermore, the distance between the first DNA binding domain recognition sequence and the second DNA binding domain recognition sequence (the length from the base adjacent to the 5' side or 3' side of the first DNA binding domain recognition sequence or its complementary sequence as the 1st base, up to the base adjacent to the 3' side or 5' side of the second DNA binding domain recognition sequence or its complementary sequence, including the target site. In this specification, sometimes referred to as "total spacer length") is 8 to 48 bases from the viewpoint of being able to substitute the one base at the said target site with high specificity and efficiency. The said total spacer length is preferably 11 to 40 bases or 12 to 48 bases, more preferably 14 to 27 bases, still more preferably 17 to 24 bases. Furthermore, depending on the type of nucleic acid base converting enzyme contained in the second fusion protein, for example, in the case of TadA (including variants), it is more preferable that the said total spacer length is 17 to 19 bases and 21 to 22 bases, and still more preferable that it is 17 to 18 bases and 21 bases.

The third DNA binding domain recognition sequence is preferably selected such that it is present via spacer 3 of 1 to 500 bases on the 5' side or 3' side of the first DNA binding domain recognition sequence opposite to the target site. When the chain length of spacer 3 is within such a range, there is a tendency for the substitution of the base at the said target site to be further promoted. The chain length of the said spacer 3 is the length from the base adjacent to the 5' side or 3' side of the first DNA binding domain recognition sequence as the 1st base, up to the base adjacent to the 3' side or 5' side of the third DNA binding domain recognition sequence or its complementary sequence. The chain length of such spacer 3 is more preferably 5 to 300 bases, still more preferably 15 to 56 bases.

The nucleotide sequence of such target DNA is not particularly limited, and by designing each DNA binding domain in the first fusion protein and the second fusion protein, and optionally the third fusion protein, such that the first DNA binding domain recognition sequence or its complementary sequence, the second DNA binding domain recognition sequence or its complementary sequence, the total spacer length, and optionally spacer 1, spacer 2, the third DNA binding domain recognition sequence or its complementary sequence, and spacer 3 satisfy the above conditions, and selecting a nucleic acid base converting enzyme according to the target base editing, it can be targeted by the DNA editing method of the present invention.

The target DNA according to the present invention can be DNA existing within cells (intracellular DNA) or DNA existing outside cells, depending on the purpose. DNA existing within cells may be endogenous DNA or exogenous DNA. Examples of endogenous DNA include genomic DNA within the nucleus and chloroplast DNA, and examples of exogenous DNA include DNA introduced into cells. When the target DNA according to the present invention is DNA existing within cells (intracellular DNA), the said DNA needs to be other than mitochondrial DNA, that is, nuclear DNA, chloroplast DNA, or exogenous DNA, more preferably nuclear DNA or exogenous DNA. DNA existing outside cells may be DNA derived from cells, or DNA amplified or synthesized outside cells.

### (Method for Editing Target DNA)

In the method for editing target DNA of the present invention, the said DNA editing system is brought into contact with the said target DNA, and by the said nucleic acid base converting enzyme activity, a base at the target site of the said target DNA is edited.

When the said DNA editing system, namely, the first fusion protein and the second fusion protein, is brought into contact with target DNA, each DNA binding domain of the first fusion protein and the second fusion protein recognizes and binds to the corresponding DNA binding domain recognition sequence on the target DNA, and induces the nicking enzyme linked to the first fusion protein, as well as the nucleic acid base converting enzyme linked to the second DNA binding domain, to the said target DNA. Thereby, at the target site, the nicking enzyme cleaves one strand of the double-stranded DNA and introduces a nick. At this time, for example, when the configuration of the first fusion protein is a configuration linked in the order of "TALE -> ND1n -> ND1 linker -> ND1" from the N-terminal side, a nick can be selectively introduced into the strand having the first DNA binding domain recognition sequence (first DNA binding domain recognition strand). As a result of the nick being introduced, by a new mechanism different from the mechanism dependent on the R-loop by the conventional CRISPR-Cas system, substitution (for example, deamination of the base) of the target base by the nearby nucleic acid base converting enzyme can be efficiently caused. The said new mechanism is presumed to be due to the relaxation of the higher-order structure of the double-stranded DNA or the creation of a partial single-stranded DNA region by the introduction of the nick. Furthermore, at this time, when a third fusion protein is further brought into contact with the target DNA, its DNA binding domain recognizes and binds to the corresponding DNA binding domain recognition sequence on the target DNA, induces the transcription regulatory factor linked to the third fusion protein to the said target DNA, and the substitution of the said target base is promoted. This is presumed to be because the said transcription regulatory factor further causes relaxation of the higher-order structure of the double-stranded DNA.

One embodiment of the positional relationship among the first fusion protein, the second fusion protein, and the target DNA in the method for editing target DNA of the present invention is shown, for example, in the conceptual diagrams of Fig. 16 and Fig. 27, taking the case where TALE is used as each DNA binding domain and deaminase is used as the nucleic acid base converting enzyme as an example.

Taking the embodiment shown in Fig. 16 as an example of one embodiment of the method for editing target DNA of the present invention, for example, embodiments include one where the target DNA, in order from the 5' side, is TALE recognition sequence (second DNA binding domain recognition sequence), spacer 2, target site (C in Fig. 16(A)), spacer 1, complementary sequence of TALE recognition sequence (complementary sequence of first DNA binding domain recognition sequence) (first embodiment: Fig. 16(A)), or, in order from the 5' side, complementary sequence of TALE recognition sequence (complementary sequence of second DNA binding domain recognition sequence), spacer 2, target site (A in Fig. 16(B)), spacer 1, complementary sequence of TALE recognition sequence (complementary sequence of first DNA binding domain recognition sequence) (second embodiment: Fig. 16(B)). Thereby, a nicking enzyme is induced to the vicinity of the target site by the first DNA binding domain, and when the configuration of the first fusion protein is a configuration linked in the order of "TALE -> ND1n -> ND1 linker -> ND1" from the N-terminal side, a nick is introduced into the strand having the first DNA binding domain recognition sequence (first DNA binding domain recognition strand). Simultaneously, since deaminase is also induced to the vicinity of the target site by the second DNA binding domain, substitution of the base at the target site (C or A) can be efficiently caused by deaminase activity on the strand opposite to the first DNA binding domain recognition strand into which the nick was introduced.

Embodiments of the method for editing target DNA of the present invention are not limited to this, and for example, may be such that the target DNA, in order from the 5' side, is first DNA binding domain recognition sequence, spacer 1, target site, spacer 2, complementary sequence of second DNA binding domain recognition sequence (third embodiment), or, in order from the 5' side, first DNA binding domain recognition sequence, spacer 1, target site, spacer 2, second DNA binding domain recognition sequence (fourth embodiment), etc.

Thereby, a one-base substitution occurs at the target site (for example, C->U), and also, for example, within cells, due to mismatch of the double-stranded DNA, the base on the strand opposite to the strand where substitution occurred is repaired to form a pair with the substituted base (for example, G->A), or is substituted with another base during repair (for example, U->A, G), or deletion or insertion of one base or ten-odd bases occurs, whereby various mutations can be introduced.

Therefore, editing of DNA according to the present invention includes deletion of one or more bases, substitution with one or more other bases, or insertion of one or more bases, or a combination of these mutations, at the target site converted by the said nucleic acid base converting enzyme and in the vicinity including it.

The method for editing target DNA of the present invention may be performed within cells or in a cell-free system. The "intracellular" location serving as the site for the method for editing target DNA of the present invention may be within eukaryotic cells or within prokaryotic cells, and is preferably within eukaryotic cells. Examples of the said eukaryotic cells include animal cells (cells of mammals, fish, birds, reptiles, amphibians, insects, etc.), plant cells, algal cells, yeast, and examples of the said prokaryotic cells include Escherichia coli, Salmonella, Bacillus subtilis, lactic acid bacteria, hyperthermophilic bacteria.

"Animal cells" include, for example, cells constituting an individual animal, cells constituting organs/tissues removed from an animal, cultured cells derived from animal tissues, etc. Specifically, examples include germ cells such as oocytes and sperm; embryonic cells at various stages (for example, 1-cell stage embryo, 2-cell stage embryo, 4-cell stage embryo, 8-cell stage embryo, 16-cell stage embryo, morula stage embryo, etc.); stem cells such as induced pluripotent stem (iPS) cells and embryonic stem (ES) cells; somatic cells such as fibroblasts, hematopoietic cells, neurons, muscle cells, bone cells, hepatocytes, pancreatic cells, brain cells, kidney cells, etc. As oocytes used for producing genome-edited animals, oocytes before and after fertilization can be utilized, but oocytes after fertilization, namely fertilized eggs, are preferable. Particularly preferably, the fertilized egg is from a pronuclear stage embryo. Oocytes that have been cryopreserved can be thawed and used.

"Plant cells" include, for example, cells constituting an individual plant, cells constituting organs or tissues separated from a plant, cultured cells derived from plant tissues, etc. Examples of plant organs or tissues include leaves, stems, shoot apexes (growth points), roots, tubers, calluses, etc.

Furthermore, the "cell-free system" serving as the site for the method for editing target DNA of the present invention refers to a system without living cells (the said eukaryotic cells, prokaryotic cells). The cell-free system according to the present invention is not particularly limited as long as it is a system where the said DNA editing system and the said target DNA can come into contact, and examples include within a buffer solution; within a cell lysate or cell extract of the said eukaryotic cells or prokaryotic cells, etc.

The method for bringing the said DNA editing system and the said target DNA into contact is not particularly limited. Within cells, for example, a method of introducing or expressing the said DNA editing system into cells containing the said target DNA, such as the method for producing cells in which target DNA has been edited described below, can be mentioned. In a cell-free system, for example, a solution of the target DNA and a solution of the said DNA editing system may be mixed. The solvent for these solutions is not particularly limited, but for example, buffer solutions such as phosphate buffer, Tris buffer, Good's buffer, borate buffer are preferable.

### <Method for Producing a Cell in Which Target DNA Has Been Edited>

The method for producing a cell in which target DNA has been edited of the present invention comprises:
a step of introducing the DNA editing system of the present invention described above into a cell or expressing it within the cell to bring it into contact with target DNA within the cell other than mitochondrial DNA, and editing a base at a target site of the target DNA by the said nucleic acid base converting enzyme activity, wherein
the distance between a first DNA binding domain recognition sequence recognized by the first DNA binding domain or its complementary sequence and a second DNA binding domain recognition sequence recognized by the second DNA binding domain or its complementary sequence is 8 to 48 bases.

Furthermore, as the method for producing a cell in which target DNA has been edited of the present invention, it is also more preferable that
the first DNA binding domain recognition sequence recognized by the first DNA binding domain or its complementary sequence is present via a first spacer of 4 to 16 bases on the 5' side or the 3' side of the target site, and
the second DNA binding domain recognition sequence recognized by the second DNA binding domain or its complementary sequence is present via a second spacer of 3 to 31 bases on the side opposite to the first DNA binding domain recognition sequence or its complementary sequence of the target site.

Furthermore, when the DNA editing system of the present invention further comprises a third fusion protein, as the method for producing a cell in which target DNA has been edited of the present invention, it is preferable that a third DNA binding domain recognition sequence recognized by the third DNA binding domain is present via a third spacer of 1 to 500 bases on the side opposite to the target site of the first DNA binding domain recognition sequence.

In the method for producing a cell in which target DNA has been edited of the present invention (hereinafter, sometimes simply referred to as "production method"), the said DNA editing system and target DNA are as described in the DNA editing system and the method for editing target DNA of the present invention described above. The target DNA in the production method of the present invention is intracellular DNA other than mitochondrial DNA, more preferably genomic DNA within the nucleus, and the first fusion protein and the second fusion protein, and further optionally the third fusion protein, can be designed according to the purpose of editing the said genomic DNA.

Furthermore, in the production method of the present invention, the contact between the said DNA editing system, namely, the first fusion protein and the second fusion protein and optionally the third fusion protein, and the intracellular target DNA is performed by introducing the said respective fusion proteins into the cell in the form of protein, introducing them into the cell in the form of polynucleotide, and/or expressing them within the cell by introducing them into the cell in the form of an expression vector. Therefore, as the said DNA editing system, the said respective fusion proteins may be introduced into the cell in the form of protein, introduced into the cell in the form of RNA or DNA (polynucleotide) encoding the said protein and expressed within the cell, or introduced into the cell in the form of a vector (expression vector) that expresses the said protein and expressed within the cell.

When introducing the said respective fusion proteins into the cell in the form of an expression vector and expressing them within the cell, for example, vectors expressing the said respective fusion proteins may be introduced into the cell respectively, or a vector expressing these in combination may be introduced into the cell.

Furthermore, when introducing the said respective fusion proteins into the cell in the form of an expression vector and expressing them within the cell, the polynucleotides encoding the said respective fusion proteins may be codon-optimized appropriately according to the cell to be introduced, independently for each. Furthermore, the said expression vector preferably comprises a promoter and/or other control sequences operably linked to the polynucleotide to be expressed. Furthermore, the said expression vector is preferably one that can stably express the encoded protein without being integrated into the host genome. Such an expression vector can be appropriately produced according to conventionally known methods.

As a method for introducing the said respective fusion proteins, polynucleotides encoding the said proteins, or vectors expressing the said proteins into cells, known methods for introducing proteins, DNA, or RNA fragments into cells can be appropriately adopted according to the cell type, and examples of such methods include electroporation method, microinjection method, particle gun method, calcium phosphate method, polyethyleneimine (PEI) method, liposome method (lipofection method), DEAE-dextran method, cationic lipid-mediated transfection, viruses (adenovirus, lentivirus, adeno-associated virus, baculovirus, etc.), Agrobacterium method, lithium acetate method, spheroplast method, heat shock method (calcium chloride method, rubidium chloride method), etc. Such methods are described in many standard laboratory manuals, such as "Davis et al., Basic methods in molecular biology, New York: Elsevier, 1986".

When the said respective fusion proteins are introduced into cells or expressed within cells, the respective fusion proteins and the intracellular target DNA come into contact, and by the target DNA editing described in the method for editing target DNA of the present invention above, the target base substitution occurs at the target site, and as a result, it becomes possible to obtain cells in which the target DNA has been edited.

The present invention also provides a method for producing a non-human individual comprising cells in which the said target DNA has been edited. This method comprises a step of producing a non-human individual from cells obtained by the production method described above. Examples of the said non-human individual include non-human animals and plants. Examples of the said non-human animals include mammals (mouse, rat, guinea pig, hamster, rabbit, monkey, pig, cow, goat, sheep, etc.), fish, birds, reptiles, amphibians, insects. When producing model animals, mammals are preferably rodents such as mice, rats, guinea pigs, hamsters, and particularly preferably mice. Examples of the said plants include cereals, oil crops, fodder crops, fruits, vegetables. Specific examples of crops include rice, corn, banana, peanut, sunflower, tomato, rapeseed, tobacco, wheat, barley, potato, soybean, cotton, carnation.

As a method for producing a non-human individual from cells in which the said target DNA has been edited, known methods can be utilized. When producing a non-human individual from cells in animals, usually germ cells or pluripotent stem cells are utilized. For example, the said DNA editing system can be microinjected into an oocyte, the obtained oocyte can be transplanted into the uterus of a female non-human mammal in a pseudopregnant state, and thereafter offspring can be obtained. Furthermore, in plants, it has long been known that their somatic cells possess totipotency, and for example, by microinjecting the said DNA editing system into plant cells and regenerating a plant body from the obtained plant cells, a plant body in which the desired DNA has been edited can be obtained. Furthermore, from the obtained non-human individual, offspring or clones in which the desired DNA has been edited can also be obtained.

Confirmation of the presence or absence of target DNA editing and determination of genotype can be performed based on conventionally known techniques, and for example, PCR method, sequencing method, Southern blotting method, etc., can be utilized.

### <Kit>

The kit of the present invention is a kit for use in the method for editing target DNA of the present invention described above, the method for producing a cell in which target DNA has been edited of the present invention, or the method for producing a non-human individual of the present invention described above, comprising:
at least one selected from the group consisting of the first fusion protein, a first fusion protein expression vector, and a polynucleotide encoding the first fusion protein, and
at least one selected from the group consisting of the second fusion protein, a second fusion protein expression vector, and a polynucleotide encoding the second fusion protein.

It is also preferable that the kit of the present invention further comprises at least one selected from the group consisting of a third fusion protein, a third fusion protein expression vector, and a polynucleotide encoding the third fusion protein.

The first fusion protein, the second fusion protein, and the third fusion protein are respectively as described in the DNA editing system of the present invention described above. These may each independently be in the form of a protein, in the form of a polynucleotide encoding the said protein, or in the form of a vector (expression vector) that expresses the said protein.

In the case of the form of an expression vector, it may be a form in which the user can design the said respective fusion proteins according to the target site of the target DNA,
the first fusion protein expression vector can be at least one selected from the group consisting of:
   (i) a vector comprising a polynucleotide encoding ND1, ND1n, the ND1 linker, and the first DNA binding domain, and
   (ii) a vector comprising a polynucleotide encoding ND1, ND1n, and the ND1 linker, and an insertion site for a polynucleotide encoding the first DNA binding domain,
the second fusion protein expression vector can be at least one selected from the group consisting of:
   (iii) a vector comprising a polynucleotide encoding the nucleic acid base converting enzyme and the second DNA binding domain, and
   (iv) a vector comprising a polynucleotide encoding the nucleic acid base converting enzyme and an insertion site for a polynucleotide encoding the second DNA binding domain,
   and the third fusion protein expression vector can be at least one selected from the group consisting of:
      (vii) a vector comprising a polynucleotide encoding a transcription regulatory factor and a third DNA binding domain, and
      (viii) a vector comprising a polynucleotide encoding the transcription regulatory factor and an insertion site for a polynucleotide encoding the third DNA binding domain.

In the vectors of (i) to (iv), (vii), and (viii) above, the order of each component (domain, linker, insertion site, etc.) can be appropriately adjusted according to the target first fusion protein, second fusion protein, and third fusion protein to be expressed. Furthermore, for example, when the first DNA binding domain, the second DNA binding domain, and the third DNA binding domain are TALE, the polynucleotide inserted into the insertion site in (ii), (iv), and (viii) may be only its TALE repeat sequence, and in this case, the vectors of (ii), (iv), and (viii) may contain a polynucleotide encoding the N-terminal domain of TALE and optionally a polynucleotide encoding the C-terminal domain. Furthermore, the first fusion protein expression vector, the second fusion protein expression vector, and optionally the third fusion protein expression vector may be a single vector. Note that it is preferable that each vector of (i) to (iv), (vii), and (viii) comprises an expression unit that enables expression of each polynucleotide.

The kit of the present invention may further comprise one or a plurality of additional reagents. Examples of such additional reagents include dilution buffer, reconstitution solution, washing buffer, nucleic acid introduction reagent, protein introduction reagent, control reagent (for example, control deaminase), but are not limited thereto. Furthermore, the said kit may further comprise instructions for use for carrying out the method of the present invention.

Each element included in the kit of the present invention may be housed in separate containers, or may be housed in the same container. Each element may be housed in a container for each single use amount, or an amount for multiple uses may be housed in one container. Each element may be housed in a container in dry form, or may be housed in a container in a form dissolved in a suitable solvent (solvent containing buffer, stabilizer, preservative, antiseptic, etc.).

### <Other Embodiments of DNA Editing>

In the present invention, the first fusion protein of the present invention may be an embodiment further comprising the said nucleic acid base converting enzyme, namely, a fusion protein comprising a first DNA binding domain, the nicking enzyme of the present invention, and a nucleic acid base converting enzyme. Thereby, as a method for editing target DNA, a method comprising a step of bringing the said fusion protein into contact with target DNA and editing a base at a target site of the said target DNA by the said nucleic acid base converting enzyme activity; and as a method for producing a cell in which target DNA has been edited, a method comprising a step of introducing the said fusion protein into a cell or expressing it within the cell to bring it into contact with target DNA within the cell other than mitochondrial DNA, and editing a base at a target site of the said target DNA by the said nucleic acid base converting enzyme activity can also be provided.

In the fusion protein in this case, regarding the order of the first DNA binding domain, nicking enzyme, and nucleic acid base converting enzyme, both the nicking enzyme and the nucleic acid base converting enzyme may be linked to either one of the N-terminal side and C-terminal side of the first DNA binding domain, and in this case, either the nicking enzyme or the nucleic acid base converting enzyme may be on the side of the first DNA binding domain. Furthermore, regarding the said order, the nicking enzyme and the nucleic acid base converting enzyme may be linked sandwiching the first DNA binding domain, and in this case, either the nicking enzyme or the nucleic acid base converting enzyme may be on the N-terminal side or the C-terminal side. Furthermore, the nucleic acid base converting enzyme and the first DNA binding domain may be directly linked, or may be linked via a linker. Examples of the said linker in this case, including its preferred embodiments, include those described as the second linker above. The first DNA binding domain, nicking enzyme, nucleic acid base converting enzyme, and each linker if included (first linker, second linker), as well as the method for linking these, including their preferred embodiments, are respectively as described above.

Furthermore, the method for bringing the fusion protein into contact with target DNA in this case, and the method for introducing it into cells or expressing it within cells, including their preferred embodiments, are respectively the same as the method for bringing the DNA editing system described above into contact with target DNA, and the method for introducing it into cells or expressing it within cells.

Therefore, the present invention also provides a kit for use in the method for editing target DNA and the method for producing a cell in which target DNA has been edited, comprising:
at least one selected from the group consisting of the said fusion protein, a fusion protein expression vector, and a polynucleotide encoding the fusion protein, wherein
the said fusion protein expression vector is at least one selected from the group consisting of (v) a vector comprising a polynucleotide encoding ND1, ND1n, the ND1 linker, a nucleic acid base converting enzyme, and the first DNA binding domain, and (vi) a vector comprising a polynucleotide encoding ND1, ND1n, the ND1 linker, and the nucleic acid base converting enzyme, and an insertion site for a polynucleotide encoding the first DNA binding domain.

It is also preferable that such a kit further comprises at least one selected from the group consisting of a third fusion protein, a third fusion protein expression vector, and a polynucleotide encoding the third fusion protein.

### [Examples]

Hereinafter, the present invention will be described more specifically based on Test Examples, but the present invention is not limited to the following Test Examples.

### [Test Example 1]

### <1. Method>

### (1) Production of TALE vector set and TALE expression plasmid

The sequences and configurations of the TALE vector set were constructed according to this test example. Note that the basic construction method followed the literature (Sakuma et al., (2013) Scientific Reports, 3, p. 1-8). That is, first, modifications were added to the module sequence other than the sequence encoding the four types of variable residues composed of two amino acids (RVD: HD, NG, NI, NN) (non-repeat-variable di-residue: non-RVD), and further, sequences with restriction enzyme BsaI recognition sequences added to both ends thereof (module sequences) were artificially DNA synthesized (total 16 types: 1HD to 4HD, 1NG to 4NG, 1NI to 4NI, 1NN to 4NN). These were inserted into pEX-A2J2 (Eurofins Genomics, Tokyo, Japan) to produce a module plasmid set (total 16 types: pEX1HD to pEX4HD, pEX1NG to pEX4NG, pEX1NI to pEX4NI, and pEX1NN to pEX4NN). Next, the nucleotide sequences FUS2_axx (total 7 types: xx = 1a, 2a, 2b, 3a, 3b, 4a, 4b) and FUS2_b(1-4) (total 4 types) constituting the array plasmid were produced by artificial DNA synthesis. These were inserted into pCR8/GW/TOPO (Thermo Fisher Scientific, Waltham, MA, USA) to produce pCR8_FUS2_axx and pCR8_FUS_b(1-4). Then, this was used as the capture vector for the first assembly step (Step 1) in the Platinum Gate system described in the aforementioned Sakuma et al. literature, and according to the method described in the same literature, array plasmids with linked TALE repeats were produced.

Next, into pcDNA3.1s, from which the drug resistance gene expression unit had been removed from pcDNA3.1(+) (Thermo Fisher Scientific), a destination vector was produced by inserting a sequence encoding the N-terminal domain of TALE, one shortened module sequence corresponding to the final module of the DNA binding repeat, and a sequence encoding the C-terminal domain of TALE following this 3' end, all produced by artificial DNA synthesis. As the C-terminal domain of TALE, three types were used: one with 63 C-terminal domain amino acid residues (63), one with 47 C-terminal domain amino acid residues (47), and one with the WT C-terminal domain (number of amino acid residues: 180); the sequence encoding "63" was a sequence referenced from the sequence of the C-terminal domain contained in Addgene's pTALEN_v2 (ID: 32189-32192) (nucleotide SEQ ID NO: 66 and amino acid SEQ ID NO: 67) and was artificially synthesized, the sequence encoding "47" was a sequence referenced from the sequence of the C-terminal domain contained in Addgene's ptCMV-153/47-VR-NG (Addgene ID: 50704) (nucleotide SEQ ID NO: 70, amino acid SEQ ID NO: 71) and was artificially synthesized, and a DNA sequence encoding "WT" was artificially synthesized with reference to the amino acid sequence of the C-terminal domain contained in Addgene's pTALETF_v2 (ID: 32185-32188). Furthermore, as the N-terminal domain of TALE, corresponding to the C-terminal domains "63" and "WT," 136: a sequence referenced from the sequence of the N-terminal domain contained in Addgene's ptCMV-136/63-VR-HD (ID: 50699) (nucleotide SEQ ID NO: 64, amino acid SEQ ID NO: 65) was synthesized by artificial DNA synthesis, and corresponding to the C-terminal domain "47," 153: a sequence referenced from the sequence of the N-terminal domain contained in Addgene's ptCMV-153/47-VR-HD (ID: 50703) (nucleotide SEQ ID NO: 68, amino acid SEQ ID NO: 69) was synthesized by artificial DNA synthesis. Furthermore, using the array plasmids and destination vectors produced above, according to the method described in the aforementioned Sakuma et al. literature, three types of TALE (TALE63, TALE47, TALEWT) expression plasmids (TALE-136/63, TALE-153/47, TALE-136/WT) were produced by the Golden Gate method.

### (2) Production of single-chain FokI (scFokI), single-chain ND1 (scND1), single-chain ND2 (scND2) expression plasmids

A nucleotide sequence encoding the HTS95 amino acid sequence (Sun, N., & Zhao, H. (2014) Molecular BioSystems, 10(3), p. 446-453, HTS95 linker: amino acid residues 197-291 of the amino acid sequence of SEQ ID NO: 61) was used as a linker sequence, and a sequence linking two copies of the partial gene encoding the nuclease domain of the FokI gene (bases 1-589 of the nucleotide sequence of SEQ ID NO: 60, amino acid residues 1-196 of the amino acid sequence of SEQ ID NO: 61: simply referred to as "FokI" in the test examples) was artificially DNA synthesized (FokI-95-FokI, nucleotide SEQ ID NO: 60, amino acid SEQ ID NO: 61). Also, a nucleotide sequence in which the encoded amino acid sequence was substituted from the amino acid sequence of the HTS95 linker to the amino acid sequence of the GGGGSx12 linker (total 60 amino acid residues) was also artificially DNA synthesized (FokI-60-FokI, nucleotide SEQ ID NO: 62, amino acid SEQ ID NO: 63). Regarding the ND1 gene and ND2 gene, ND1-60-ND1 (nucleotide SEQ ID NO: 72, amino acid SEQ ID NO: 73) in which a linker sequence encoding the GGGGSx12 linker was inserted between partial genes encoding nuclease domain 1 (ND1) (bases 1-585 of the nucleotide sequence of SEQ ID NO: 72, amino acid residues 1-195 of the amino acid sequence of SEQ ID NO: 73 (amino acid sequence of SEQ ID NO: 98)), and ND2-95-ND2 (nucleotide SEQ ID NO: 86, amino acid SEQ ID NO: 87) in which a linker sequence encoding the HTS95 linker was inserted between partial genes encoding nuclease domain 2 (ND2) (bases 1-573 of the nucleotide sequence of SEQ ID NO: 86, amino acid residues 1-191 of the amino acid sequence of SEQ ID NO: 87) were artificially DNA synthesized. Note that during the above artificial DNA synthesis, synthesis was performed by adding restriction enzyme AleI/XmaI recognition sequences upstream of the sequence encoding the GGGGSx12 linker or HTS95 linker, and restriction enzyme PshAI/SacII recognition sequences downstream, respectively, as common sequences. These synthesized sequences were inserted into pEX-A2J2 (Eurofins Genomics, Tokyo, Japan).

Next, ND1-60-ND1/pEX-A2J2 and ND2-95-ND2/pEX-A2J2 were digested with restriction enzymes AleI and PshAI, and respectively, a 190 bp DNA fragment encoding the GGGGSx12 linker (60 aa), a 295 bp DNA fragment encoding the HTS95 linker (95 aa), and fragments of each remaining vector portion were isolated. After linking the 60 amino acid residue DNA fragments by ligation reaction using T4 DNA ligase, they were digested with restriction enzymes XmaI and SacII, and the DNA fragments thus obtained were separated by agarose gel electrophoresis, whereby a 380 bp DNA fragment (120 aa: GGGGSx24 linker) and a 570 bp DNA fragment (180 aa: GGGGSx36 linker) were isolated and purified. The above 95 aa, 120 aa, 180 aa DNA fragments were respectively inserted into the vector portion fragment obtained by digesting ND1-60-ND1/pEX-A2J2 with restriction enzymes AleI and PshAI, and also, the above 60 aa, 120 aa, 180 aa DNA fragments were respectively inserted into the vector portion fragment obtained by digesting ND2-95-ND2/pEX-A2J2 with restriction enzymes AleI and PshAI, thereby obtaining plasmids ND1-95-ND1 (nucleotide SEQ ID NO: 78, amino acid SEQ ID NO: 79)/pEX-A2J2, ND1-120-ND1 (nucleotide SEQ ID NO: 74, amino acid SEQ ID NO: 75)/pEX-A2J2, ND1-180-ND1 (nucleotide SEQ ID NO: 76, amino acid SEQ ID NO: 77)/pEX-A2J2, ND2-60-ND2 (nucleotide SEQ ID NO: 80, amino acid SEQ ID NO: 81)/pEX-A2J2, ND2-120-ND2 (nucleotide SEQ ID NO: 82, amino acid SEQ ID NO: 83)/pEX-A2J2, and ND2-180-ND2 (nucleotide SEQ ID NO: 84, amino acid SEQ ID NO: 85)/pEX-A2J2, respectively.

Next, each sequence of the above scFokI (FokI-y-FokI, y: 95, 60), scND1 (ND1-y-ND1, y: 95, 60, 120, 180), scND2 (ND2-y-ND2, y: 95, 60, 120, 180) was respectively linked downstream of the TALE sequence of the TALE expression plasmid (TALE-136/63) produced in (1), to produce TALE63-scFokI expression plasmids, TALE63-scND1 expression plasmids, TALE63-scND2 expression plasmids by the following method. That is, first, using the above-mentioned plasmids FokI-95-FokI/pEX-A2J2, FokI-60-FokI/pEX-A2J2, ND1-95-ND1/pEX-A2J2, ND1-60-ND1/pEX-A2J2, ND1-120-ND1/pEX-A2J2, ND1-180-ND1/pEX-A2J2, ND2-95-ND2/pEX-A2J2, ND2-60-ND2/pEX-A2J2, ND2-120-ND2/pEX-A2J2, ND2-180-ND2/pEX-A2J2 as templates, each sequence of scFokI, scND1, scND2 was respectively amplified by PCR. These were inserted downstream of the sequence of TALE63 (N-terminal domain: 136 amino acid residues / TALE repeat domain / C-terminal domain: 63 amino acid residues), which is the Platinum TALE structure of the TALE expression plasmid, by the In-Fusion method (TaKaRa Bio Inc, Shiga, Japan) to produce TALE63-scFokI expression plasmids, TALE63-scND1 expression plasmids, TALE63-scND2 expression plasmids, respectively. Note that in the case of inserting the scFokI sequence, two amino acids "LK" are further added to the C-terminal side of the amino acid sequence of the C-terminal domain in the TALE sequence. Also, similarly, regarding each sequence of scND1, TALE47-scND1 expression plasmids linked downstream of the TALE47 sequence of the TALE expression plasmid (TALE-153/47) produced in (1) were also produced. Furthermore, similarly, respective expression plasmids of TALE63-ND1mono, TALE63-ND2mono, TALE47-ND1mono, TALE47-ND2mono, in which the artificially DNA synthesized sequences of ND1 and ND2 were respectively linked downstream of the TALE sequence of the TALE expression plasmid (TALE-136/63 or TALE-153/47) produced in (1), were also produced. The templates and primers used for these productions are shown in Table 1 below.

Into each expression plasmid subjected to the SSA assay, a TALE repeat domain corresponding to the nucleotide sequence of the TALE recognition sequence on Rosa26 (Rosa26-L, R), the TALE recognition sequence on APC (APC-L, R), or the TALE recognition sequence on the HPRT1 gene (HPRT1-L, R) shown in Table 8 below was inserted.

**[Table 1]**

| Products | Template | Forward Primer | SEQ ID NO | Reverse Primer | SEQ ID NO |
|---|---|---|---|---|---|
| scFokI | Synthesized scFokI | FokI_FW | 1 | FokI_RV | 2 |
| TALE63 expression vector | TALE136/63 | TALE63-RinfFokI | 3 | Vector-FinfFokI | 4 |
| scND1 | ND1-60-ND1 | ND1F_inf63-2 | 5 | ND1R_inf63 | 6 |
| scND2 | ND2-95-ND2 | ND2F_inf63 | 7 | ND2R_inf63 | 8 |
| ND1 monomer | ND1-60-ND1 | ND1F_inf63-2 | 5 | ND1monoR_inf63 | 9 |
| ND2 monomer | ND2-95-ND2 | ND2F_inf63 | 7 | ND2monoR_inf63 | 10 |
| TALE63 expression vector | TALE136/63 | TALE_63_R | 11 | TALEvector_F | 12 |
| TALE47 expression vector | TALE153/47 | TALE_47_R | 13 | TALEvector_F | 12 |

### (3) Production of reporter plasmid for single strand annealing (SSA) assay

Regarding the reporter plasmid for the SSA assay, first, amplification products of two fragments, the N-terminal side and C-terminal side of the EGFP sequence, were respectively obtained by PCR using as template the cDNA of EGFP artificially DNA synthesized by referring to the sequence information described in the literature (Mashiko et al., (2013) Scientific Reports, 3, 3355, DOI: 10.1038/srep03355). Next, after performing PCR again with primers to which a linker sequence was added between the N-terminal side and the C-terminal side to obtain amplification products, these were inserted into the restriction enzyme BamHI/EcoRV recognition sequence of pcDNA3.1s by the In-Fusion method, thereby producing a plasmid (pcEGxxFP) expressing a sequence in which a linker sequence was inserted between the N-terminal side and the C-terminal side of the EGFP sequence (referred to as "EGxxFP"). The templates and primers used for these productions are shown in Table 2 below.

**[Table 2]**

| Products | Template | Forward Primer | SEQ ID NO | Reverse Primer | SEQ ID NO |
|---|---|---|---|---|---|
| EGFP-N terminal | Synthetic EGFP | EGFP_F+bam | 14 | EGFP_600R | 15 |
| EGFP-N terminal + linker | EGFP-N-terminal Fragment | EGFP_F+bam | 14 | EGFP_600R+linker | 16 |
| EGFP-C terminal | Synthetic EGFP | EGFP_120F | 17 | EGFP_R+rv | 18 |
| EGFP-C terminal + linker | EGFP-C-terminal Fragment | linker+EGFP_120 F | 19 | EGFP_R+rv | 18 |

As each target gene used for the SSA assay, APC (human adenomatous polyposis coli) was isolated from human genome, and Rosa26 and HPRT1 were isolated from hamster genome, respectively, by PCR, and these were inserted into the restriction enzyme BamHI/EcoRI recognition sequence within the linker inserted between the N-terminal side and C-terminal side of EGFP in pcEGxxFP by the In-Fusion method, thereby producing respective reporter plasmids for the SSA assay. Sequences containing the nucleotide sequences of the target genes APC, Rosa26, and HPRT1 used for SSA assay evaluation (EGxAPCxFP, EGxRosa26xFP, EGxHPRT1xFP) are shown in SEQ ID NO: 94 to 96, respectively.

### (4) Production of plasmid for examining TALE C-terminal length

Examination of the chain length between scND1 and the TALE repeat domain of TALE was performed by changing the C-terminal length starting from the C-terminus of TALE47 of the TALE47-scND1 expression plasmid produced in (2). To make it longer, PCR was performed using TALE-136/WT produced in (1) as template, and the amplification product was linked between the C-terminal domain of TALE47 and scND1 of the TALE47-scND1 expression plasmid produced in (2), thereby producing nine types of TALE47-z-scND1 expression plasmids having C-terminal domains with chain lengths from 63 amino acid residues to 180 amino acid residues (z: 63, 75, 90, 105, 120, 135, 150, 165, 180). Also, to make it shorter, PCR was performed using the TALE47-scND1 expression plasmid produced in (2) as template, thereby producing four types of TALE47-z-scND1 expression plasmids having C-terminal domains with chain lengths from 0 amino acid residues to 36 amino acid residues (z: 0, 12, 24, 36). The templates and primers used for these productions are shown in Table 3 below.

**[Table 3]**

| Products | Template | Forward Primer | SEQ ID NO | Reverse Primer | SEQ ID NO |
|---|---|---|---|---|---|
| TALE47 N-terminal + β-lactamase | TALE47_ND1-95-ND1 | TALE-CR | 20 | Amp_R | 21 |
| ND1-95-ND1 + β-lactamase | TALE47_ND1-95-ND1 | ND1-N_F | 22 | Amp_F | 23 |
| TALE-C terminal-63aa | TALE136/WT | WT-CF | 24 | WT-63R_infND1 | 25 |
| TALE-C terminal-75aa | TALE136/WT | WT-CF | 24 | WT-75R_infND1 | 26 |
| TALE-C terminal-90aa | TALE136/WT | WT-CF | 24 | WT-90R_infND1 | 27 |
| TALE-C terminal-105aa | TALE136/WT | WT-CF | 24 | WT-105R infND1 | 28 |
| TALE-C terminal-120aa | TALE136/WT | WT-CF | 24 | WT-120_infND1 | 29 |
| TALE-C terminal-135aa | TALE136/WT | WT-CF | 24 | WT-135R infND1 | 30 |
| TALE-C terminal-150aa | TALE136/WT | WT-CF | 24 | WT-150R infND1 | 31 |
| TALE-C terminal-165aa | TALE136/WT | WT-CF | 24 | WT-165R infND1 | 32 |
| TALE-C terminal-180aa | TALE136/WT | WT-CF | 24 | WT-180R infND1 | 33 |
| TALE47 C-terminal-0aa + β-lactamase | TALE47_ND1-95-ND1 | TALE-0_R_infND1 | 34 | Amp_R | 21 |
| TALE47 C-terminal-12aa + β-lactamase | TALE47_ND1-95-ND1 | TALE-12_R_infND1 | 35 | Amp_R | 21 |
| TALE47 C-terminal-24aa + β-lactamase | TALE47_ND1-95-ND1 | TALE-24_R_infND1 | 36 | Amp_R | 21 |
| TALE47 C-terminal-36aa + β-lactamase | TALE47_ND1-95-ND1 | TALE-36_R_infND1 | 37 | Amp_R | 21 |

### (5) Production of flexible linkers

Three types of flexible linkers: GSS linker, SAGG linker, and GGGGS linker were produced such that they could be inserted into the target sequence in a form where the number of repeats could be adjusted. First, sequences with a restriction enzyme XmaI recognition sequence arranged at the 5' end and a BamHI or MroI recognition sequence arranged at the 3' end: GSSx4 adapter, SAGGx3 adapter, and GGGGSx3 adapter were synthesized using respective forward oligonucleotides and reverse oligonucleotides. The oligonucleotides used for these productions are shown in Table 4 below.

**[Table 4]**

| Products | Forward Oligo. | SEQ ID NO | Reverse Oligo. | SEQ ID NO | 5' end | 3' end |
|---|---|---|---|---|---|---|
| GSSx4 Adapter | GSSx4+5'ad. F | 38 | GSSx4+5'ad. R | 39 | XmaI | MroI |
| SAGGx3 Adapter | SAGGx3+5'ad._F | 40 | SAGGx3+5'ad.R | 41 | XmaI | BamHI |
| GGGGSx3 Adapter | GGGGSx3+5'ad._F | 42 | GGGGSx3+5'ad._R | 43 | XmaI | MroI |
| GSSx4 Linker | GSSx4_F | 44 | GSSx4_R | 45 | XmaI | MroI |
| GSSx7 Linker | GSSx7_F | 46 | GSSx7_R | 47 | XmaI | MroI |
| SAGGx3 Linker | SAGGx3_F | 48 | SAGGx3_R | 49 | BglII | BamHI |
| SAGGx4 Linker | SAGGx4_F | 50 | SAGGx4_R | 51 | BglII | BamHI |
| GGGGSx3 Linker | GGGGSx3_F | 52 | GGGGSx3_R | 53 | XmaI | MroI |
| GGGGSx4 Linker | GGGGSx4_F | 54 | GGGGSx4_R | 55 | XmaI | MroI |

After phosphorylating the 5' ends of each oligonucleotide and annealing, they were inserted into a plasmid possessing one site each for XmaI, BamHI, and MroI (for example, since the 60 amino acid residue linker sequence of TALE63-ND1-60-ND1 has one site each for XmaI, BamHI, MroI cleavage sequences, TALE63-ND1-60-ND1 was used as a temporary vector in this test example). These were designated as GSS adapter vector, SAGG adapter vector, GGGGS adapter vector, respectively.

Also, as flexible linker repeat sequences to be inserted into each adapter vector, sequences with a restriction enzyme BglII recognition sequence arranged at the 5' end and a BamHI recognition sequence at the 3' end, or a restriction enzyme XmaI recognition sequence arranged at the 5' end and an MroI recognition sequence at the 3' end: GSSx4, GSSx7, SAGGx3, SAGGx4, GGGGSx3, and GGGGSx4 were synthesized using respective forward oligonucleotides and reverse oligonucleotides. The oligonucleotides used for these productions are also shown in Table 4 above. After phosphorylating the 5' ends of each oligonucleotide and annealing, ligation reaction was performed, followed by double digestion with restriction enzymes BglII and BamHI, as well as XmaI and MroI, and fragments with the target number of repeats were recovered by agarose gel electrophoresis. For example, in the case of GSSx4 (number of repeats: 4), repeat sequences such as GSSx8, GSSx12, GSSx16 were obtained, and by performing ligation reaction mixed with GSSx7, DNA fragments having various numbers of repeats such as GSSx11, GSSx14, GSSx15 could be obtained.

DNA fragments having each target number of repeats (final target number of repeats - number of repeats inserted into the adapter vector) were inserted into the BamHI site or MorI site of the GSS adapter vector, SAGG adapter vector, GGGGS adapter vector, thereby obtaining polynucleotides encoding linkers with the final target number of repeats. These polynucleotides were isolated by double digestion with restriction enzymes XmaI and BamHI, as well as XmaI and MorI.

Next, in order to insert the DNA fragment with the final target number of repeats in place of the HTS95 linker (95 amino acid residues) in the sequence ND1-95-ND1, plasmids TALE24-scND1(bam) and TALE24-scND1(mro) were produced by inserting a recognition sequence for restriction enzyme BamHI or MorI immediately upstream (N-terminal side) of the ND1 located on the C-terminal side of the two in the TALE24-scND1 expression plasmid (scND1: ND1-95-ND1) having the C-terminal domain of 24 amino acid residues produced in (4). The templates and primers used for producing these plasmids are shown in Table 5 below.

**[Table 5]**

| Products | Template | Forward Primer | SEQ ID NO | Reverse Primer | SEQ ID NO |
|---|---|---|---|---|---|
| TALE24-scND1 (bam ) | TALE24-scND1 | bamND1_ F | 56 | 95aa_R_inf_bamN D1 | 57 |
| TALE24-scND1 (mro ) | TALE47-scND1 | mroND1___ F | 58 | 95aa_R_inf_mroN D1 | 59 |

Next, each plasmid was double digested with restriction enzymes XmaI and BamHI, as well as XmaI and MorI, followed by dephosphorylation of the 5' ends by alkaline phosphatase. Thereafter, ligation reaction was performed together with the DNA fragments with the final target number of repeats prepared above, finally obtaining TALE24-ND1-GSSx32 linker-ND1 expression plasmid, TALE24-ND1-SAGGx24 linker-ND1 expression plasmid, and TALE24-ND1-GGGGGSx19 linker-ND1 expression plasmid. In each plasmid, the sequence of the TALE repeat domain was made to be a sequence corresponding to the nucleotide sequence of the TALE recognition sequence on APC (APC-L, R), the TALE recognition sequence on Rosa26 (Rosa26-L, R), and the TALE recognition sequence on HPRT1 (HPRT1-L, R) shown in Table 8 below. The nucleotide sequences and amino acid sequences of ND1-GSSx32 linker-ND1, ND1-SAGGx24 linker-ND1, and ND1-GGGGGSx19 linker-ND1 are shown in SEQ ID NO: 88 to 93, respectively.

### (6) Production of TALE-scND1 mutant (scND1n)

In order to introduce a mutation into one ND1 of scND1, first, the N-terminal side ND1 and the C-terminal side ND1 of the two were placed on separate plasmids. That is, using the TALE47-12-ND1-95-ND1 (hereinafter, sometimes referred to as "TALE12-ND1-95-ND1") expression plasmid produced in (4) above as template, PCR was performed with the primer set shown in Table 6 below, each PCR fragment was linked by the In-Fusion method, and a TALE12-ND1/pcDNA3.1s plasmid having up to "TALE12-ND1" and a 95-ND1/pcDNA3.1s plasmid having the remaining "95-ND1" were produced. Next, using TALE12-ND1/pcDNA3.1s as template, site-directed mutagenesis by PCR was performed using the primer sets N-D450N, N-D450A, and N-D467A shown in Table 7 below, respectively, thereby introducing three types of mutations, D450N, D450A, or D467A mutation, into the N-terminal side ND1. Also, using 95-ND1/pcDNA3.1s as template, site-directed mutagenesis by PCR was performed using the primer sets C-D450N, C-D450A, C-D467A shown in Table 7 below, respectively, thereby introducing three types of mutations, D450N, D450A, or D467A mutation, into the C-terminal side ND1.

### (7) Production of TALE-scND1n expression plasmid

The two types of plasmids each having the N-terminal side ND1 and the C-terminal side ND1 before introducing mutations and the six types of plasmids produced in (6) above, a total of eight types of plasmids were double digested with restriction enzymes XmaI and ScaI, and by appropriately combining the N-terminal side and C-terminal side of TALE12-ND1-95-ND1 and performing ligation reaction, three types with mutations introduced into the N-terminal side ND1: TALE12-ND1(D450N)-95-ND1, TALE12-ND1(D450A)-95-ND1, TALE12-ND1(D467A)-95-ND1; three types with mutations introduced into the C-terminal side ND1: TALE12-ND1-95-ND1(D450N), TALE12-ND1-95-ND1(D450A), TALE12-ND1-95-ND1(D467A), a total of six types of TALE12-scND1n (Example) expression plasmids linking each mutant scND1n were produced. Note that "D450" corresponds to the 66th aspartic acid of the amino acid sequence of SEQ ID NO: 98, and "D467" corresponds to the 83rd aspartic acid of the amino acid sequence of SEQ ID NO: 98.

**[Table 6]**

| Products | Template | Forward Primer | SEQ ID NO | Reverse Primer | SEQ ID NO |
|---|---|---|---|---|---|
| TALE47-ND1/pcDNA3.1s | TALE47-ND1-95-ND1 | TALEvector_F | 100 | ND1-N_R_infEcoRI | 101 |
| 95-ND1/pcDNA3.1s | TALE47-ND1-95-ND1 | ND1-C_F_infT7 | 102 | T7-L_R | 103 |

**[Table 7]**

| Primer set | Primer | SEQ ID NO |
|---|---|---|
| N-D450N | ND1-N-D450N F | 104 |
| | ND1-N-D450N R | 105 |
| N-D450A | ND1-N-D450A F | 106 |
| | ND1-N-D450A R | 107 |
| N-D467A | ND1-N-D467A F | 108 |
| | ND1-N-D467A R | 109 |
| C-D450N | ND1-C-D450N F | 110 |
| | ND1-C-D450N R | 111 |
| C-D450A | ND1-C-D450A F | 112 |
| | ND1-C-D450A R | 113 |
| C-D467A | ND1-C-D467A F | 114 |
| | ND1-C-D467A R | 115 |

Into each TALE12-scND1n expression plasmid subjected to the SSA assay, a TALE repeat domain corresponding to the nucleotide sequence of the TALE recognition sequence on Rosa26 (Rosa26-L, R), the nucleotide sequence of the TALE recognition sequence on APC (APC-L, R), or the nucleotide sequence of the TALE recognition sequence on the HPRT1 gene (HPRT1-L, R) was inserted. Also, into the TALE12-scND1n expression plasmid subjected to the T7E1 assay, a TALE repeat domain corresponding to the TALE recognition sequence on APC (off4-L) was inserted. Furthermore, into each TALE12-scND1n expression plasmid subjected to the reporter assay, a TALE repeat domain corresponding to the nucleotide sequence of the TALE recognition sequence on Rosa26 (Rosa26-L) and of the nucleotide sequence of the recognition sequence on the reporter (Rosa26-LM4.2 to LM.2+4) was inserted, and into each TALE12-scND1n expression plasmid subjected to the endogenous DNA editing assay, a TALE repeat domain corresponding to the nucleotide sequence of the TALE recognition sequence on APC (APC-L2-1 to 3) was inserted. Note that, in this specification, when described as "TALEz(ww)-," "z" indicates the chain length of the C-terminal domain of TALE, and "ww" indicates the corresponding TALE recognition sequence. Each TALE recognition sequence is shown in Table 8 below.

**[Table 8]**

| TALE Repeat Domain | SEQ ID NO | TALE Recognition Sequence (5'-3') |
|---|---|---|
| APC-R | 116 | tACAGAAGCGGGCAAAGN |
| APC-L | 117 | tATGTACGCCTCCCTGGN |
| APC-L2-1 | 118 | tCGGCGCCCCCTATGTACGN |
| APC-L2-2 | 119 | tCGGCGCCCCCTATGTACGCN |
| APC-L2-3 | 120 | tCGGCGCCCCCTATGTACGCCN |
| Rosa26-L | 121 | tGCCCAGAAGACTCCCN |
| Rosa26-R | 122 | tGATCTGCAAGTCGAGGN |
| Rosa26-LM4.2 | 123 | tCCTGACCCCTCCCGGGAN |
| Rosa26-LM3.2 | 124 | tCCTGACCCCTCCCGGGN |
| Rosa26-LM2.2 | 125 | tCCTGACCCCTCCCGGN |
| Rosa26-LM1.2 | 126 | tGTCCTGACCCCTCCCGN |
| Rosa26-LM.2 | 127 | tGTCCTGACCCCTCCCN |
| Rosa26-LM.2+1 | 128 | tCCGTGTCCTGACCCCTCCN |
| Rosa26-LM.2+2 | 129 | tCCGTGTCCTGACCCCTCN |
| Rosa26-LM.2+3 | 130 | tCCGTGTCCTGACCCCTN |
| Rosa26-LM.2+4 | 131 | tCCGTGTCCTGACCCCN |
| HPRT1-R | 132 | tCGACATAGTGATTAGGN |
| HPRT1-L | 133 | tGAACCAGGCTATGACCN |
| off4-L | 181 | tGCCCAGCAACCATTAAN |
| Negative control TALE | 134 | tTGCGCGTATAGTCGCGN |

### (8) Production of Cas9 and its mutant expression plasmids

First, the region from the U6 promoter to the BGH polyA addition sequence of pX330 (Addgene, Cambridge, MA; Plasmid 42230) was artificially DNA synthesized, and by insertion using the In-Fusion method between the EcoRV recognition site and BamHI recognition site of pBlueScript II (SK+) (Stratagene, La Jolla, CA, USA), the Cas9 expression plasmid pX330_BS was produced. Next, with respect to the Cas9 gene of pX330_BS, by performing site-directed mutagenesis by PCR, respective mutant expression plasmids, namely, nickase type nCas9(D10A) expression plasmid, nCas9 (H840A) expression plasmid, and dCas9(D10A+H840A) expression plasmid without cleavage activity were respectively produced.

### (9) Production of guide RNA expression plasmid

First, pX330_BS produced in (8) above was treated with restriction enzymes XbaI and NotI to remove the Cas9 gene, and after fill-in reaction, by self-ligation, a plasmid (pX330_BS-ΔCas9) was produced. Next, shown in Table 9 below, oligonucleotides designed to correspond to the target sequence of the guide RNA (Table 9 shows the complementary sequence (sequence other than underlined part) of the target sequence of the guide RNA (on the antisense strand)) were annealed, and by BpiI treatment and ligation, inserted into pX330_BS-ACas9, for each target gene of Rosa26 and HPRT1, plasmids expressing each guide RNA (gRNA-A to D), as well as, for the target gene APC, plasmids expressing each guide RNA (gRNA-A to D and gRNA-off4-L3 to L5) were produced. Table 9 below shows the complementary sequence of the target sequence of the guide RNA (other than underlined part) and the PAM sequence of Cas9 (underlined part).

**[Table 9]**

| Target Gene | Guide RNA | SEQ ID NO | Complementary Sequence of guide RNA Target Sequence plus PAM Sequence |
|---|---|---|---|
| Rosa26 | gRNA-A | 135 | GCCTCGACTTGCAGATCACG AGG |
| Rosa26 | gRNA-B | 136 | TGAGGGCTAACCTGGTGCGT GGG |
| Rosa26 | gRNA-C | 137 | GGAAGGGATCCTTCCAGTCC CGG |
| Rosa26 | gRNA-D | 138 | GGAGATGGGCGGGAGTCTTC TGG |
| APC | gRNA-A | 139 | TCTGTACCACCCTCAGTTCT CGG |
| APC | gRNA-B | 140 | GGGGGCGCCGAGGCCCGAGA AGG |
| APC | gRNA-C | 141 | GTTCTCGGGTCCTGGAGCAC CGG |
| APC | gRNA-D | 142 | CCCAGGGAGGCGTACATAGG GGG |
| HPRT1 | gRNA-A | 143 | TAATCACTATGTCGAGGATT TGG |
| HPRT1 | gRNA-B | 144 | AATAAGCCTTAGTTCGCCAG AGG |
| HPRT1 | gRNA-C | 145 | GAAAAGGTGTTTATTCCTCA TGG |
| HPRT1 | gRNA-D | 146 | AATAAATCTAGGTCATAGCC TGG |
| APC | gRNA-off4-L3 | 147 | GGGGAAATTGAGTTTCGGCC AGG |
| APC | gRNA-off4-L4 | 148 | GGCCGAAACTCAATTTCCCC TGG |
| APC | gRNA-off4-L5 | 149 | TCAATTTCCCCTGGTAGAAA TGG |
| For CBE Reporter | gRNA-CBE-L4M2 | 150 | AGACGGATATCTCCCGGGAG GGG |
| For ABE Reporter | gRNA-ABE-L4M2 | 151 | GCTTAGTCTATCTCCCGGGA GGG |

| | | | |
|---|---|---|---|
| Underlined: PAM sequence | | | |

### (10) Production of other plasmids used in this test example

TALE-deaminase: respective expression plasmids for TALE47-12-AID, ABE8e-32-TALE47, and negative control plasmids thereof; respective expression plasmids for AncBE4max and ABE8e were produced, respectively, in the same manner as the TALE47-12-AID expression plasmid, ABE8e-32-47 expression plasmid, and negative control plasmids thereof, and as the AncBE4max expression plasmid and ABE8e expression plasmid described in International Publication No. WO 2022/050377. Note that "AID" indicates PmCDA1 deaminase, and "ABE8e" indicates TadA-8e deaminase (a variant of TadA), and "CBE" below corresponds to TALE47-12-AID and converts C/G to T/A, and "ABE" corresponds to ABE8e-32-TALE47 and converts A/T to G/C.

Into each TALE-deaminase expression plasmid subjected to the reporter assay and the endogenous DNA editing assay, a TALE repeat domain corresponding to the nucleotide sequence of the TALE recognition sequence on APC (APC-R) was inserted.

### (11) Quantification of double-strand cleavage activity against DNA by SSA assay

### (i) TALE-scFokI, TALE-scND1, TALE-scND2

The SSA assay targeting TALE-scFokI, TALE-scND1, TALE-scND2 was performed as follows. Namely, HEK293T cells grown in DMEM medium containing 10% FBS were seeded at 1x10⁴ cells each into each well of a 96-well plate on the day before transfection. For each of the TALE-scFokI expression plasmid, TALE-scND1 expression plasmid, TALE-scND2 expression plasmid produced in (2) above, both a plasmid having a TALE repeat domain corresponding to Rosa26-L, APC-L, or HPRT1-L (TALE-L), and a plasmid having a TALE repeat domain corresponding to Rosa26-R, APC-R, or HPRT1-R (TALE-R) at 33 ng each, or alternatively, either one at 33 ng; 33 ng of the reporter plasmid for SSA assay produced in (3) above; were introduced into HEK293T cells using Lipofectamine 3000 (Thermo Fisher Scientific). In cases where the types of introduced plasmids were reduced, supplementation was done with pBluescript II (SK+) (Stratagene, La Jolla, CA, USA), making the total amount of introduced plasmid 100 ng. After transfection, cells were cultured for 48 hours, and the fluorescence amount due to EGFP protein per unit area of each well was measured with a plate reader. In order to reduce variation due to cell localization within the well, fluorescence measurement was performed at 16 points in each well, and the average value thereof was taken as the EGFP fluorescence intensity (RFI), and double-strand cleavage activity was evaluated. The average value and standard deviation of values from three independent wells were graphed.

### (ii) TALE-scND1n

The SSA assay targeting TALE-scND1n was performed as follows. Namely, 2 ng of any one of the TALE12-scND1n expression plasmids produced in (7) above; 16.5 ng of the guide RNA expression plasmid produced in (9) above; 33 ng of the nCas9(D10A) expression plasmid produced in (8) above; 33 ng of the reporter plasmid for SSA assay produced in (3) above; except for using these, fluorescence measurement was performed in the same manner as in (i) above, and double-strand cleavage activity was evaluated.

### (12) Production of reporter plasmid for detecting base substitution activity

Using pNLF1-C[CMV Hygro] (manufactured by PROMEGA, WI, USA) as a reporter, by site-directed mutagenesis, the 71st and 107th methionine codons (ATG) were changed to alanine codons (ATA) to make pNLF-M/A. Next, into pNLF-M/A, oligonucleotides designed to correspond to the sequences shown in Table 10 below were annealed, and inserted into pNLF-M/A treated with restriction enzymes NheI and XhoI, producing reporter plasmids into which each sequence for examining the length of spacer 1 (distance between the target base (target site, the same applies hereinafter) and the TALE recognition sequence of TALE-scND1n or its complementary sequence) (sequences shown in Table 10) was inserted. Also, oligonucleotides designed to correspond to the sequences shown in Table 11 below were annealed, and inserted into pNLF-M/A treated with restriction enzymes NheI and XhoI, producing reporter plasmids into which each sequence for examining the length of spacer 2 (distance between the target base and the TALE recognition sequence of TALE-deaminase or its complementary sequence) (sequences shown in Table 11) was inserted. The inserted sequence comprises the TALE recognition sequence of TALE-deaminase (underlined (*1) in Tables 10, 11) or its complementary sequence (underlined (*3) in Table 11), the codon containing the target base (underlined (*2) in Tables 10, 11: 5'-ACG-3'), the complementary sequence of the TALE recognition sequence of TALE-scND1n (underlined (*3) in Table 10, underlined (*4) in Table 11), and spacer sequences between these, and in the examination of spacer 1 length, the length of spacer 1 was set to 4 to 16 bases, and in the examination of spacer 2 length, the length of spacer 2 was set to 7 to 16 bases.

**[Table 10]**

| <For Evaluating Spacer 1 Length> | | |
|---|---|---|
| Products | Sequence (5'-3') | SEQ ID NO |
| Rosa26-L2 | CTAGCTACAGAAGCGGGCAAAGG*¹CTATTTGGGAAGACG*²GATCGGGAGTCTTCTGGGCA*³CGGC | 152 |
| Rosa26-L3 | CTAGCTACAGAAGCGGGCAAAGG*¹CTATTTGGGAAGACG*²GATATCCGGGAGTCTTCTGGGCA*³CGGC | 153 |
| Rosa26-L4 | CTAGCTACAGAAGCGGGCAAAGG*¹CTATTTGGGAAGACG*²GATATCTCCCGGGAGTCTTCTGGGCA*³CGGC | 154 |
| Rosa26-L5 | CTAGCTACAGAAGCGGGCAAAGG*1CTATTTGGGAAGACG*2GATATCTCCTGCCGGGAGTCTTCTGGGCA*3C GGC | 155 |
| Rosa26-R1 | CTAGCTACAGAAGCGGGCAAAGG*1CTATTTGGGAAGACG*2GATATCTCCTGCAGGCGGGAGTCTTCTGGGC A*3CGGC | 156 |

| | | |
|---|---|---|
| *1: TALE recognition sequence of the TALE deaminase (APC-R) *2: Codon containing the target nucleotide *3: The complementary sequence is the TALE recognition sequence of TALE-scND1n (Rosa26-L) | | |

**[Table 11]**

| <For Evaluating Spacer 2 Length> | | | |
|---|---|---|---|
| | Products | Sequence (5'-3') | SEQ ID NO |
| Fo r CB E | CBE-L4M2-7bp | CTAGCTACAGAAGCGGGCAAAGG*¹GGGAAGACG*²GATATCTCCCGGGAGGGGTCAGGACACGGA*⁴GGC | 157 |
| | CBE-L4M2-8bp | CTAGCTACAGAAGCGGGCAAAGG*1TGGGAAGACG*2GATATCTCCCGGGAGGGGTCAGGACACGGA*4GG C | 158 |
| | CBE-L4M2-9bp | CTAGCTACAGAAGCGGGCAAAGG*1TTGGGAAGACG*2GATATCTCCCGGGAGGGGTCAGGACACGGA*4G GC | 159 |
| | CBE-L4M2-10bp | CTAGCTACAGAAGCGGGCAAAGG*1TTTGGGAAGACG*2GATATCTCCCGGGAGGGGTCAGGACACGGA*4 GGC | 160 |
| | CBE-L4M2-11bp | CTAGCTACAGAAGCGGGCAAAGG*1ATTTGGGAAGACG*2GATATCTCCCGGGAGGGGTCAGGACACGGA* 4GGC | 161 |
| | CBE-L4M2-12bp | CTAGCTACAGAAGCGGGCAAAGG*1TATTTGGGAAGACG*2GATATCTCCCGGGAGGGGTCAGGACACGGA *4GGC | 162 |
| | CBE-L4M2-13bp | CTAGCTACAGAAGCGGGCAAAGG*1CTATTTGGGAAGACG*2GATATCTCCCGGGAGGGGTCAGGACACGG A*4GGC | 163 |
| | CBE-L4M2-14bp | CTAGCTACAGAAGCGGGCAAAGG*1TCTATTTGGGAAGACG*2GATATCTCCCGGGAGGGGTCAGGACACG GA*4GGC | 164 |
| | CBE-L4M2-15bp | CTAGCTACAGAAGCGGGCAAAGG*1GTCTATTTGGGAAGACG*2GATATCTCCCGGGAGGGGTCAGGACAC GGA*4GGC | 165 |
| | CBE-L4M2-16bp | CTAGCTACAGAAGCGGGCAAAGG*1AGTCTATTTGGGAAGACG*2GATATCTCCCGGGAGGGGTCAGGACA CGGA*4GGC | 166 |
| Fo r AB E | ABE-L4M2-7bp | CTAGCCCTTTGCCCGCTTCTGTA*³ATGGCTTAG*²TCTATCTCCCGGGAGGGGTCAGGACACGGA*⁴GGC | 167 |
| | ABE-L4M2-8bp | CTAGCCCTTTGCCCGCTTCTGTA*3AATGGCTTAG*2TCTATCTCCCGGGAGGGGTCAGGACACGGA*4GG C | 168 |
| | ABE-L4M2-9bp | CTAGCCCTTTGCCCGCTTCTGTA*3ACATGGCTTAG*2TCTATCTCCCGGGAGGGGTCAGGACACGGA*4G GC | 169 |
| | ABE-L4M2-10bp | CTAGCCCTTTGCCCGCTTCTGTA*3AACATGGCTTAG*2TCTATCTCCCGGGAGGGGTCAGGACACGGA*4 GGC | 170 |
| | ABE-L4M2-11bp | CTAGCCCTTTGCCCGCTTCTGTA*3CAACATGGCTTAG*2TCTATCTCCCGGGAGGGGTCAGGACACGGA* 4GGC | 171 |
| | ABE-L4M2-12bp | CTAGCCCTTTGCCCGCTTCTGTA*3ACATGCGCGCTTAG*2TCTATCTCCCGGGAGGGGTCAGGACACGGA *4GGC | 172 |
| | ABE-L4M2-13bp | CTAGCCCTTTGCCCGCTTCTGTA*3AACATGCGCGCTTAG*2TCTATCTCCCGGGAGGGGTCAGGACACGG A*4GGC | 173 |
| | ABE-L4M2-14bp | CTAGCCCTTTGCCCGCTTCTGTA*3CAACATGCGCGCTTAG*2TCTATCTCCCGGGAGGGGTCAGGACACG GA*4GGC | 174 |
| | ABE-L4M2-15bp | CTAGCCCTTTGCCCGCTTCTGTA*3CCAACATGCGCGCTTAG*2TCTATCTCCCGGGAGGGGTCAGGACAC GGA*4GGC | 175 |
| | ABE-L4M2-16bp | CTAGCCCTTTGCCCGCTTCTGTA*3CCCAACATGCGCGCTTAG*2TCTATCTCCCGGGAGGGGTCAGGACA CGGA*4GGC | 176 |

| | | | |
|---|---|---|---|
| *1: TALE recognition sequence of the TALE deaminase (APC-R) *2: Codon containing the target nucleotide *3: The complementary sequence is the TALE recognition sequence of the TALE deaminase (APC-R) *4: The complementary sequence is the TALE recognition sequence of TALE-scND1n (Rosa26-LM4.2 to LM.2+4) | | | |

A conceptual diagram showing the structure of the completed reporter plasmid is shown in Fig. 17. In this reporter plasmid, the target codon is located at a position corresponding to the start codon (Fig. 17(A)) or stop codon (Fig. 17(B)) of NanoLuc luciferase, and according to such a reporter plasmid, by TALE-deaminase bound to the TALE recognition sequence, when the target codon ACG is converted to ATG (Fig. 17(A)), or when the stop codon (TAG) is converted to the tryptophan codon (TGG) (Fig. 17(B)), NanoLuc luciferase is expressed. Guide RNAs for the positive control AncBE4max (gRNA-CBE-L4M2, gRNA-ABE-L4M2) were produced according to (9) above. The complementary sequence of the target sequence of each guide RNA (other than underlined part) and the PAM sequence of Cas9 (underlined part) are also shown in Table 9 above.

### (13) Transfection into HEK cells

### (i) Reporter assay

The reporter assay using each reporter plasmid produced in (12) above as target DNA was performed as follows. Namely, 20 ng of any one of the reporter plasmids produced in (12) above; 75 ng of either the TALE-deaminase expression plasmid produced in (10) above or its negative control plasmid; 75 ng of any one of the TALE12-scND1n expression plasmids produced in (7) above; 20 ng of the guide RNA expression plasmid produced in (9) above; and 5 ng of the reference plasmid pGL4.54; were combined, and using Lipofectamine LTX (manufactured by Thermo Fisher Scientific), introduced into 5x10⁴ HEK293T cells per well, and cultured for 24 hours. The reference plasmid is a plasmid that expresses firefly luciferase (Fluc).

Also, as a positive control, 20 ng of any one of the reporter plasmids produced in (12) above; 75 ng of either the AncBE4max expression plasmid or the ABE8e expression plasmid produced in (10) above; 20 ng of the guide RNA expression plasmid produced in (9) above; 5 ng of the reference plasmid pGL4.54; except for combining these, in the same manner, introduced into HEK293T cells, and cultured for 24 hours.

### (ii) Endogenous (nuclear) DNA editing assay

When endogenous (nuclear) DNA was used as the target DNA, 60 ng of the TALE-deaminase expression plasmid produced in (10) above; 30 ng of the TALE12-scND1n expression plasmid produced in (7) above; were combined, and using Lipofectamine LTX (manufactured by Thermo Fisher Scientific), introduced into 3x10⁴ HEK293T cells per well, and cultured for 48 hours. When performing the T7E1 assay, 75 ng of the TALE12-scND1n expression plasmid produced in (7) above; 75 ng of either the Cas9 expression plasmid pX330_BS produced in (8) above or its mutant expression plasmid; 25 ng of the guide RNA expression plasmid produced in (9) above; except for combining these, in the same manner, introduced into HEK293T cells, and cultured for 48 hours.

### (14) Measurement of NanoLuc luciferase activity value

After culturing for 24 hours following transfection in (13)(i) above, the medium was removed, after washing with PBS(-), treated with Passive Lysis Buffer (manufactured by PROMEGA) to lyse the cells, making a cell lysate. The said cell lysate was diluted 100-fold with DMEM medium, and using the Nano-Glo Dual-Luciferase Reporter Assay System (manufactured by PROMEGA), with a TriStar S LB942 plate reader (manufactured by Berthold Technologies), the NanoLuc luciferase activity score and firefly luciferase activity score were measured.

As the negative control for each score, the NanoLuc luciferase activity score for cells into which the negative control plasmid of TALE-deaminase was introduced was measured. Note that, for the positive control (cells into which AncBE4max expression plasmid or ABE8e expression plasmid was introduced), the activity score for cells into which the pX330_BS-ACas9 plasmid was introduced (cells not expressing guide RNA) was measured and taken as the negative control (AncBE4max-NC or ABE8e-NC).

Quantification of activity was performed as follows. First, using the firefly luciferase activity score from the reference plasmid, each NanoLuc luciferase activity score was standardized. Next, using the standardized activity score, from the activity score of cells into which the TALE-deaminase expression plasmid was introduced, the activity score of cells into which the negative control plasmid of TALE-deaminase was introduced was subtracted, and taken as the activity value of TALE-deaminase activity. Also, from the activity score of cells into which the AncBE4max expression plasmid or ABE8e expression plasmid was introduced, AncBE4max-NC or ABE8e-NC was subtracted, and taken as the AncBE4max activity value or ABE8e activity value. Furthermore, in order to compare TALE-deaminase activity between each test, the TALE-deaminase activity value was shown as relative activity when the AncBE4max activity value or ABE8e activity value was set to 1. The test was repeated at least three times, and the average value was graphed with standard deviation added.

### (15) Analysis of base editing activity against endogenous (nuclear) DNA

48 hours after transfection in (13) (ii) above, the medium was removed, after washing with PBS(-), 50 µL of DNAzol (Molecular Research Center, Inc.) was added to lyse the cells, making a cell lysate. Using the said cell lysate as template, using the APC-F2 and APC-R primers described in Table 12 below, by PrimeSTAR Max (manufactured by Takara Bio Inc.), the region containing the target site of the said endogenous (nuclear) DNA was amplified. After purifying the obtained PCR product, sequencing was requested from Fasmac Co., Ltd. Using EditR (Kluesner et al., The CRISPR J 1, 239-250 (2018)), the sequence data was analyzed, and the percentage (%) of T at each C within the target range on the target site was calculated.

**[Table 12]**

| Target Gene | Primer | Sequence (5'-3') | SEQ ID NO |
|---|---|---|---|
| APC | APC-F2 | CATGCGCATTGTAGTCTTCCCACCTCCCAC | 177 |
| | APC-R | CGTCTGGCAGGTGAGTGAGGCTGCAG | 178 |
| | off4-F | AGTCCTGTTTCTAAAAAAGGCCCACCCC | 179 |
| | off4-R | GTTCTGTTGGCTCATCTGTCTACCTGG | 180 |

### (16) T7E1 assay

Transfection was performed in (13)(ii) above, and by the method described in (15) above, using the off4-F and off4-R primers described in Table 12 above, PCR was performed. With respect to the obtained PCR product, according to the manufacturer's protocol, a T7E1 assay was performed.

### <2. Results>

### (1) Confirmation of double-strand cleavage activity by SSA assay

### (i) Confirmation of activity of TALE-scFokI

First, as a monomeric nuclease lacking base recognition activity, the TALE-scFokI activity of a previously reported molecule was confirmed. As TALE, already in NPL (Sakuma et al., Sci. Rep., 3:3379 (2013)), intracellular activity has been confirmed for the TALE recognition sequence on the APC gene, namely, TALE having a TALE repeat domain for the Right TALE of TALEN (APC-R: nucleotide sequence of SEQ ID NO: 116 / complementary strand of bases 119-136 of the nucleotide sequence of SEQ ID NO: 94), and Left TALE (APC-L: nucleotide sequence of SEQ ID NO: 117 / bases 85-102 of the nucleotide sequence of SEQ ID NO: 94) was used. As a positive control, TALEN having TALE repeat domains corresponding to APC-R, APC-L (TALEN(APC-R) + TALEN(APC-L)) was placed, and evaluation was performed by the SSA assay method in HEK293T cells ([Test Example 1] <1. Method> (11)(i)). As a result, regarding the TALE-scFokI expression plasmid (scFokI: FokI-95-FokI) produced in [Test Example 1] <1. Method> (2), only the one having the TALE repeat domain corresponding to APC-R (TALE(APC-R)-FokI-95-FokI) and only the one having the TALE repeat domain corresponding to APC-L (TALE(APC-L)-FokI-95-FokI) showed no significant difference in double-strand cleavage activity compared to the case using only the reporter plasmid for SSA assay (mock) (Fig. 1).

Therefore, expecting improvement in activity, the linker sequence between FokI was changed to the GGGGSx12 linker in the same manner as in [Test Example 1] <1. Method> (5), and regarding the TALE-scFokI expression plasmid (scFokI: FokI-60-FokI), the case of only the one having the TALE repeat domain corresponding to APC-R (TALE(APC-R)-FokI-60-FokI) and the case of only the one having the TALE repeat domain corresponding to APC-L (TALE(APC-L)-FokI-60-FokI) were similarly assayed, but similarly to the above, double-strand cleavage activity could not be confirmed (Fig. 1). From this, it was judged that scFokI has very low cleavage activity, and its use for efficient genome editing in human cells is difficult.

### (ii) Confirmation of double-strand cleavage activity of TALE-scND1, TALE-scND2

Next, while following the structure of scFokI, with ND1 and ND2, which were found as alternative factors for the FokI nuclease domain, a similar examination was performed. TALE63-ND1mono expression plasmid having a TALE repeat domain corresponding to APC-R or APC-L produced in [Test Example 1] <1. Method> (2) (TALE63(APC-R/L)-ND1mono expression plasmid) and combinations thereof, TALE63-scND1 expression plasmid having a TALE repeat domain corresponding to APC-L (linker: 60, 95, 120, 180 aa); TALE63-ND2mono expression plasmid having a TALE repeat domain corresponding to APC-R or APC-L (TALE63(APC-R/L)-ND2mono expression plasmid) and combinations thereof, TALE63-scND2 expression plasmid having a TALE repeat domain corresponding to APC-L (linker: 60, 95, 120, 180 aa) were respectively used, and evaluation was performed by SSA assay in HEK293T cells ([Test Example 1] <1. Method> (11)(i)). A conceptual diagram of the configuration of each protein expressed by these expression plasmids (TALE-ND1mono, TALE-scND1, TALE-ND2mono, TALE-scND2) is shown in Fig. 2. To the N-terminal side of each protein, a FLAG tag (3xFLAG: not shown) and a nuclear localization signal NLS were added.

As a result of the assay, the combination of TALE63-ND1mono expression plasmids having TALE repeat domains corresponding to APC-R or APC-L (TALE63(APC-R/L)-ND1mono expression plasmid) (TALE(APC-R)ND1mono + TALE(APC-L)ND1mono) showed remarkably low double-strand cleavage activity compared to the combination of the same TALE63-ND2mono expression plasmids (TALE(APC-R)ND2mono + TALE (APC-L) ND2mono), but when linked to form a single chain (when made into scND1), surprisingly, each of them showed excellent activity (Fig. 3, Fig. 4). On the other hand, with ND2, when linked to form a single chain (when made into scND2), it did not show double-strand cleavage activity (Fig. 3). Furthermore, when the TALE63-scND1 expression plasmid was changed to the TALE47-scND1 expression plasmid, higher activity was shown with TALE47-ND1-95-ND1 (Fig. 4).

The results regarding scND1 in Fig. 4 are for the case where the TALE repeat domain corresponds to APC-R, but in the other five cases, specifically, when the TALE repeat domain corresponds to APC-L; when corresponding to the TALE recognition sequence on HPRT1, namely, the Right TALE (HPRT1-R: nucleotide sequence of SEQ ID NO: 132 / complementary strand of bases 357-374 of the nucleotide sequence of SEQ ID NO: 96) and Left TALE (HPRT1-L: nucleotide sequence of SEQ ID NO: 133 / bases 324-340 of the nucleotide sequence of SEQ ID NO: 96) of TALEN; also when corresponding to the TALE recognition sequence on hamster Rosa26, namely, the Left TALE (Rosa26-L: nucleotide sequence of SEQ ID NO: 121 / complementary strand of bases 163-179 of the nucleotide sequence of SEQ ID NO: 65) and Right TALE (Rosa26-R: nucleotide sequence of SEQ ID NO: 122 / bases 130-147 of the nucleotide sequence of SEQ ID NO: 65) of TALEN (TALE nucleases) (Sato et al., (2015) Stem Cell Reports, 14; 5(1), p. 75-82), similarly, higher double-strand cleavage activity was shown with TALE47-ND1-95-ND1 (Fig. 5A, Fig. 5B). From these results, in scND1, it was found that the HTS95 linker found in the HTS95 amino acid sequence is preferable as the linker between ND1s.

### (2) Examination of TALE-scND1 structure

Next, the effect of the sequence length on the C-terminal side of TALE on activity was examined. First, using TALE47-z(Rosa26-L)-scND1 or TALE47-z (Rosa26-R)scND1 produced in [Test Example 1] <1. Method> (4), in which the C-terminal side sequence in scND1 was extended (z: 63, 75, 90, 105, 120, 135, 150, 165, 180 amino acid residues), and having a TALE repeat domain corresponding to Rosa26-L or Rosa26-R, an SSA assay ([Test Example 1] <1. Method> (11)(i)) was performed, and no change in activity depending on the number of C-terminal side amino acid residues was observed (Fig. 6). On the other hand, using TALE47-z(Rosa26-L/R)-scND1, TALE47-z(APC-L/R)-scND1, or TALE47-z(HPRT1-L/R)-scND1 produced in [Test Example 1] <1. Method> (4), in which the C-terminal side sequence in scND1 was shortened (z: 36, 24, 12, 0 amino acid residues), and having a TALE repeat domain corresponding to Rosa26-L/R, APC-L/R, or HPRT1-L/R, an SSA assay ([Test Example 1] <1. Method> (11)(i)) was performed, and particularly when the C-terminal side sequence was 12 amino acid residues or more, activity was stably high in all cases (Fig. 7A, Fig. 7B) .

In (1)(ii) above, the result was obtained that the optimal linker length between NDls in scND1 is 95 amino acid residues, but whereas such a linker is a sequence specific to the HTS95 (95aa) linker, the other linkers of 60, 120, 180 amino acid residues are composed of repeating sequences of the GGGGS sequence, therefore whether the chain length of 95 amino acid residues as a linker is optimal, or whether the sequence of the HTS95 linker is optimal is unclear. Therefore, using the expression plasmids produced in [Test Example 1] <1. Method> (5), into which sequences encoding three types of linkers (GSSx32 linker, SAGGx24 linker, GGGGSx19 linker) were inserted, activity was compared by SSA assay ([Test Example 1] <1. Method> (11)(i)). A conceptual diagram of the configuration of these expression plasmids is shown in Fig. 8. To the N-terminal side of each protein, a FLAG tag (3xFLAG: not shown) and a nuclear localization signal NLS were added. As a result, although relatively good activity was observed overall when the HTS95 linker was used (Fig. 9(A) to (C)), when APC was the target gene, equivalent activity was observed with all linkers (Fig. 9(B)).

### (3) Conversion of TALE-scND1 into nickase

### (i) SSA assay

Nickase activity by introducing D450N, D450A, or D467A mutation into one of the FokI nuclease domains of TALEN-L and TALEN-R in TALEN has been reported (Wu et al., (2014) Biochemical and Biophysical Research Communications, 446, 261, DOI: 10.1016/j.bbrc.2014.02.099). Since these D450, D467 amino acid residues are also conserved in ND1, into the N-terminal side ND1 or C-terminal side ND1 of TALE-scND1, by introducing mutations corresponding to D450N, D450A, or D467A (mutations D66N, D66A, or D83A in SEQ ID NO: 98), conversion into nickase was examined. First, using the TALE12-scND1n expression plasmids produced in [Test Example 1] <1. Method> (7), which have a TALE repeat domain corresponding to Rosa26-L: three types with mutations introduced into the N-terminal side ND1: TALE12(Rosa26-L)-ND1(D450N)-95-ND1, TALE12(Rosa26-L)-ND1(D450A)-95-ND1, TALE12(Rosa26-L)-ND1(D467A)-95-ND1; three types with mutations introduced into the C-terminal side ND1: TALE12(Rosa26-L)-ND1-95-ND1(D450N), TALE12(Rosa26-L)-ND1-95-ND1(D450A), TALE12(Rosa26-L)-ND1-95-ND1(D467A), a total of six types of expression plasmids, along with the gRNA-A or gRNA-B expression plasmid designed on the target gene Rosa26 (produced in [Test Example 1] <1. Method> (9)) and the nCas9(D10A) expression plasmid (produced in [Test Example 1] <1. Method> (8)), an SSA assay ([Test Example 1] <1. Method> (11) (ii)) using Rosa26-L as the TALE recognition sequence of TALE12-scND1n was performed. Fig. 10(A) shows a conceptual diagram of the positional relationship between the PAM sequence and the cleavage point by nCas9(D10A) on the target gene (Rosa26), and TALE (Rosa26-L) and the guide RNA.

As a result of the assay, the three types with mutations introduced into the N-terminal side ND1, when acted upon with gRNA-A, strong double-strand cleavage activity was confirmed, while when combined with gRNA-B, the double-strand cleavage activity was weak, and when combined with gRNA-A, namely, all three types of TALE12(Rosa26-L)-ND1(mutant)-95-ND1 were shown to preferentially introduce nicks into the TALE recognition strand. On the other hand, all three types with mutations introduced into the C-terminal side ND1, when combined with either gRNA-A or gRNA-B, equivalent double-strand cleavage activity was confirmed, and all three types of TALE12(Rosa26-L)-ND1-95-ND1(mutant) were shown to introduce nicks into either the TALE recognition strand or the non-recognition strand (Fig. 11A).

Also, TALE12-scND1n expression plasmids having a TALE repeat domain corresponding to Rosa26-R: three types with mutations introduced into the N-terminal side ND1: TALE12(Rosa26-R)-ND1(D450N)-95-ND1, TALE12(Rosa26-R)-ND1(D450A)-95-ND1, TALE12(Rosa26-R)-ND1(D467A)-95-ND1, three types with mutations introduced into the C-terminal side ND1: TALE12(Rosa26-R)-ND1-95-ND1(D450N), TALE12(Rosa26-R)-ND1-95-ND1(D450A), TALE12(Rosa26-R)-ND1-95-ND1(D467A), using a total of six types of expression plasmids, an SSA assay ([Test Example 1] <1. Method> (11)(ii)) using Rosa26-R as the TALE recognition sequence of TALE12-scND1n was performed. Fig. 10(A) shows a conceptual diagram of the positional relationship between the PAM sequence and the cleavage point by nCas9(D10A) on the target gene (Rosa26), and TALE (Rosa26-R) and the guide RNA.

As a result of the assay, the three types with mutations introduced into the N-terminal side ND1, all showed relatively strong double-strand cleavage activity when combined with gRNA-D. On the other hand, when combined with gRNA-C, although the double-strand cleavage activity of TALE12(Rosa26-R)-ND1(D450N)-95-ND1 was weak, relatively sufficient activity was confirmed in the other two types, and it was shown that nicks were introduced into either the TALE recognition strand or the non-recognition strand. Also, the three types with mutations introduced into the C-terminal side ND1, all, when combined with gRNA-C, strong double-strand cleavage activity was confirmed, while when combined with gRNA-D, the double-strand cleavage activity was weak, and all three types of TALE12 (Rosa26-R)-ND1-95-ND1(mutant) were shown to preferentially introduce nicks into the TALE non-recognition strand (Fig. 11B).

From these results, it was confirmed that TALE12-ND1(D450N)-95-ND1, in both cases of Rosa26-L and Rosa26-R, introduces nicks particularly preferentially into the TALE recognition strand. Hereinafter, "ND1(D450N)-95-ND1" was used as "scND1n". However, nickase activity has also been confirmed in N-terminal side ND1 mutants other than D450N, and all C-terminal side ND1 mutants, and this does not deny their usability.

Furthermore, regarding other TALE recognition sequences on target genes: APC-L, APC-R, HPRT1-L, HPRT1-R, respective TALE12-scND1n were produced, and using gRNA-A, B, C, D designed on the target gene APC (Fig. 10(B)) or gRNA-A, B, C, D designed on the target gene HPRT1 (Fig. 10(C)), and nCas9(D10A), an SSA assay ([Test Example 1] <1. Method> (11)(ii)) targeting these target genes was performed. Fig. 10(B), (C) show conceptual diagrams of the positional relationship between the PAM sequence and the cleavage point by nCas9(D10A) on the target genes (B: APC, C: HPRT1), and TALE (B: APC-L/R, C: HPRT-1-L/R) and the guide RNA.

As a result of the assay, TALE12(APC-L)-scND1n combined with gRNA-A, TALE12(APC-R)-scND1n combined with gRNA-D, TALE12(HPRT1-L)-scND1n combined with gRNA-A, and TALE12(HPRT1-R)-scND1n combined with gRNA-D, particularly strong double-strand cleavage activity was confirmed when respectively combined (Fig. 12, Fig. 13). On the other hand, TALE12(APC-L)-scND1n combined with gRNA-B, TALE12(APC-R) -scND1n combined with gRNA-C, TALE12(HPRT1-L)-scND1n combined with gRNA-B, and TALE12(HPRT1-R)-scND1n combined with gRNA-C, the double-strand cleavage activity when respectively combined was weak (Fig. 12, Fig. 13), and it was shown that these TALE-scND1n particularly preferentially introduce nicks into the TALE recognition strand.

### (ii) T7E1 assay

Next, that TALE-scND1n preferentially introduces nicks into the TALE recognition strand also in a genome target was confirmed by T7E1 assay. With respect to TALE12(off4-L)-scND1n produced in [Test Example 1] <1. Method> (7), guide RNAs were designed at three locations (Fig. 14(A), gRNA-off4-L3 to 5). By acting each guide RNA with each Cas9 produced in [Test Example 1] <1. Method> (8) and examining whether they function, since cleavage bands were confirmed for all guide RNAs, it was shown that the guide RNAs are functioning (Fig. 14(B)). Next, by acting TALE12(off4-L)-scND1n with any one of nCas9(D10A), nCas9(H840A), and dCas9(D10A+H840A) produced in [Test Example 1] <1. Method> (8), and each guide RNA, which DNA strand TALE12(off4-L)-scND1n cleaves was examined. The positional relationship of the PAM sequence, the cleavage point by nCas9, the guide RNA, and the TALE of TALE12 (off4-L)-scND1n is shown in Fig. 14(A). nCas9(D10A) introduces a nick into the same strand as the target sequence of the guide RNA, and nCas9(H840A) introduces a nick onto the complementary strand of the target sequence of the guide RNA. With gRNA-off4-L3, a cleavage band was confirmed when acted upon with nCas9(H840A). On the other hand, with gRNA-off4-L4, L5, a cleavage band was confirmed when acted upon with nCas9(D10A) (Fig. 14(C)). From the above, it was confirmed that TALE-scND1n preferentially introduces nicks into the TALE recognition strand also in a genome target.

### (4) Confirmation of base substitution activity by TALE-scND1n and TALE-deaminase

In this test example, TALE-AID was used as the cytidine deaminase type TALE-deaminase, and ABE8e-TALE was used as the adenosine deaminase type TALE-deaminase. Fig. 15 shows conceptual diagrams of the configuration of TALE-AID (A) and ABE8e-TALE (B).

Also, Fig. 16(A) shows a conceptual diagram of base editing by cooperation between TALE-scND1n and TALE-AID, and Fig. 16(B) shows a conceptual diagram of base editing by cooperation between TALE-scND1n and ABE8e-TALE, respectively. In these cooperative systems, near the target base ((A): cytosine, (B): adenine) on the target DNA, and such that they respectively bind to TALE recognition sequences sandwiching the same target base, each TALE (TALE-L, TALE-R) is designed. When using TALE-AID (Fig. 16(A)), by TALE-scND1n bound to the TALE recognition sequence of the target DNA, when a nick is introduced into the DNA strand near the target base (TALE recognition strand), the DNA strand containing the target base becomes single-stranded, and by TALE-AID bound to the TALE recognition sequence of the target DNA, the target base C is deaminated to become U, and it is expected to be finally substituted with T. Similarly, in the case of ABE8e-TALE, substitution of the target base A to G is expected (Fig. 16(B)).

Using various reporters produced in [Test Example 1] <1. Method> (12), which differ in the length of spacer 2 (distance between the target base and the TALE recognition sequence of TALE-deaminase or its complementary sequence), and the length of spacer 1 (distance between the target base and the TALE recognition sequence of TALE-scND1n or its complementary sequence) (Fig. 17), respectively, detection of base substitution activity was performed. First, whether base editing occurs by cooperation between TALE-scND1n and TALE-deaminase was examined by the reporter assay of [Test Example 1] <1. Method> (13)(i), (14). As the reporter, Rosa26-L2 to L5, R1 were used (Table 10), as the TALE-scND1n expression plasmid, the TALE12(Rosa26-L)-scND1n expression plasmid produced in [Test Example 1] <1. Method> (7) was used, and as the TALE-deaminase expression plasmid, the TALE-AID (TALE47-12-AID) expression plasmid produced in [Test Example 1] <1. Method> (10) was used (Fig. 15(A)), the spacer 2 length was set to 13 bases, and the spacer 1 length was examined at 4, 7, 10, 13, 16 bases.

As a result of the assay, even in the presence of TALE-AID (47-12-AID), when TALE-scND1n was absent (w/o scND1n), or when it was not bound near the target base (scND1n-NC), base substitution activity was not detected. Also, base substitution activity was not detected with TALE-scND1n alone (w/o 47-12-AID). On the other hand, when both TALE-AID and TALE-scND1n were bound near the target base, and the spacer 1 length was 7 to 10 bases, base substitution activity was detected (Fig. 18). From these results, it was shown that base editing occurs by cooperation between TALE-scND1n and TALE-deaminase.

### (5) Examination of spacer 1 length and spacer 2 length

Since it was shown that base editing occurs by cooperation between TALE-scND1n and TALE-deaminase, examination of the optimization of spacer 1 length and spacer 2 length was performed.

### (TALE-AID)

Using CBE-L4M2-7bp to 16bp as reporter (Table 11), using the TALE12(Rosa26-LM.2)-scND1n expression plasmid produced in [Test Example 1] <1. Method> (7) as the TALE-scND1n expression plasmid, using the TALE-AID (TALE47-12-AID) expression plasmid produced in [Test Example 1] <1. Method> (10) as the TALE-deaminase expression plasmid (Fig. 15(A)), the spacer 1 length was 10 bases, the spacer 2 length was 7 to 16 bases, and examination was performed by the reporter assay of [Test Example 1] <1. Method> (13)(i), (14). As a result of the assay, activity was detected when the spacer 2 length was 8 to 15 bases, and particularly high activity was shown at 10 bases and 11 bases (Fig. 19).

Next, in the case where the spacer 2 length, which showed relatively high activity above, was 9 to 13 bases, examination of the spacer 1 length was performed similarly. The TALE12(Rosa26-LM.3.2 to LM.2+4)-scND1n expression plasmids produced in [Test Example 1] <1. Method> (7) were used as the TALE-scND1n expression plasmid. As a result, particularly high activity was confirmed when the spacer 2 length was 10 to 11 bases and the spacer 1 length was 9 to 11 bases (Fig. 20). When converted to the distance between both TALE recognition sequences, activity was particularly high at 20 to 23 bases, and maximum activity was shown at 22 bases.

### (ABE8e-TALE)

Using ABE-L4M2-7bp to 16bp as reporter (Table 11), using the TALE12(Rosa26-LM1.2)-scND1n expression plasmid produced in [Test Example 1] <1. Method> (7) as the TALEscND1n expression plasmid, using the ABE8e-TALE (ABE8e-32-TALE47) expression plasmid produced in [Test Example 1] <1. Method> (10) as the TALE-deaminase expression plasmid (Fig. 15(B)), the spacer 1 length was 9 bases, the spacer 2 length was 7 to 16 bases, and examination was performed by the reporter assay of [Test Example 1] <1. Method> (13)(i), (14). As a result of the assay, activity was detected at all spacer 2 lengths, and particularly high activity was shown at 11 bases and 12 bases (Fig. 21).

Next, in the case where the spacer 2 length was 9 to 14 bases, examination of the spacer 1 length was performed similarly. The TALE12(Rosa26-LM.4.2 to LM.2+4)-scND1n expression plasmids produced in [Test Example 1] <1. Method> (7) were used as the TALE-scND1n expression plasmid. As a result, particularly high activity was confirmed when the spacer 2 length was 10 to 12 bases and the spacer 1 length was 9 to 11 bases (Fig. 22). When converted to the distance between both TALE recognition sequences, activity was particularly high at 20 to 23 bases, and maximum activity was shown at 21 bases and 22 bases.

### (6) Base editing of endogenous (nuclear) DNA by TALE-scND1n and TALE-deaminase

Since the reporter assay above revealed that the cooperative system of TALE-scND1n and TALE-deaminase has base editing activity, whether base editing is possible also in endogenous (nuclear) DNA was examined by the endogenous DNA editing assay of [Test Example 1] <1. Method> (13)(ii), (15). First, regarding TALE-AID, as the TALE-scND1n expression plasmid, the TALE12-scND1n expression plasmid produced in [Test Example 1] <1. Method> (7) was used, and TALE was made into three types of different lengths having TALE repeat domains corresponding to the TALE recognition sequence on APC: APC-L2-1 to 3 (Fig. 23(A)). Note that the sequence of the target site (APC) described in Fig. 23(A) is shown in SEQ ID NO: 182. Also, as the TALE-deaminase expression plasmid, TALE-AID (TALE47-12-AID) produced in [Test Example 1] <1. Method> (10) was used, and TALE was made to have a TALE repeat domain corresponding to the TALE recognition sequence on APC: APC-R (Fig. 23(A)). As a result of setting the distance between both TALE recognition sequences to three types: 23, 24, 25 bases, in all cases, substitution to T occurred over a wide range of C (C3 to C19: positions shown in Fig. 23(A)), and particularly high activity was confirmed when the distance between both TALE recognition sequences was 23 bases, and the percentage of T at each C was about 40% at C11 to C13 (Fig. 23(B)).

### [Test Example 2]

### <1. Method>

### (1) Production of expression plasmid with ABE8e (V108W) placed on the C-terminal side of TALE

For the purpose of examining activity improvement from ABE8e-TALE (ABE8e-32-TALE47) produced in [Test Example 1] <1. Method> (10), four types of TALE-ABE8e (TALE16-XTEN-ABE8e, TALE47-XTEN-ABE8e, TALE47-ABE8e, TALE47-12-ABE8e) expression plasmids with ABE8e (V108W) placed on the C-terminal side of TALE were respectively produced. Namely, using the ABE8e-TALE (ABE8e-32-TALE47) expression plasmid produced in [Test Example 1] <1. Method> (10) as template, using the primer set described in Table 13 below (combination of ABE_F_infXTEN and ABE_R_infCterm), the gene encoding ABE8e (V108W, nucleotide SEQ ID NO: 193, amino acid SEQ ID NO: 194) was amplified by PCR. Also, using the TALE-153/47 expression plasmid produced in [Test Example 1] <1. Method> (1) as template, using each primer set described in Table 13 below, the sequence encoding each TALE (TALE-153/16 (nucleotide SEQ ID NO: 195, amino acid SEQ ID NO: 196), TALE-153/47) was amplified by PCR. Furthermore, an oligonucleotide (nucleotide SEQ ID NO: 197) encoding a 2 aa+XTEN linker (amino acid SEQ ID NO: 198) with two amino acids added to the XTEN linker and its complementary sequence were artificially DNA synthesized and annealed. These nucleotide fragments were combined, linked by the In-Fusion method, and three types of TALE-ABE8e (TALE16-XTEN-ABE8e, TALE47-XTEN-ABE8e, TALE47-ABE8e) expression plasmids were respectively produced.

Also, to a fragment amplified by PCR using the TALE-AID (TALE47-12-AID) expression plasmid produced in [Test Example 1] <1. Method> (10) as template and using the primer set described in Table 13 below, a fragment (ABE8e(V108W)) amplified by PCR using the ABE8e-TALE expression plasmid above as template and using the primer set described in Table 13 below (combination of ABE_F and ABE_R_infCterm) was linked by the In-Fusion method, producing the TALE-ABE8e (TALE47-12-ABE8e) expression plasmid.

The templates and primers used for producing these plasmids are shown in Table 13 below. For evaluation by reporter assay, into each of these expression plasmids, a TALE repeat domain corresponding to the nucleotide sequence of the TALE recognition sequence (APC-R) shown in Table 8 above was inserted.

**[Table 13]**

| Products | Template | Forward Primer | SEQ ID NO | Reverse Primer | SEQ ID NO |
|---|---|---|---|---|---|
| ABE8e (V108W) | ABE8e-32-TALE47 | ABE_F_infXTEN | 185 | ABE_R_infCterm | 186 |
| ABE8e (V108W) | ABE8e-32-TALE47 | ABE_F | 187 | ABE_R_infCterm | 186 |
| TALE-153/16 | TALE-153/47 | TALE16_R_infXTEN | 188 | Cterm-F | 189 |
| TALE-153/47 | TALE-153/47 | TALE47_R_infXTEN | 190 | Cterm-F | 189 |
| TALE-153/47 | TALE-153/47 | TALE47_R_infABE | 191 | Cterm-F | 189 |
| TALE47-12 | TALE47-12-AID | 12aa_infABE | 192 | Cterm-F | 189 |

### (2) Production of TALE-BspD6I construct for evaluating nicking activity

A structure for evaluating the nicking activity of BspD6I (Nt.BspD6I(C) described in Reference I) was produced. Here, in order to perform activity comparison, similarly to TALE12-scND1n produced in [Test Example 1] <1. Method> (7), BspD6I was placed on the C-terminal side of TALE. The nucleotide sequence encoding the nicking activity domain of the BspD6I gene (nucleotide SEQ ID NO: 199, amino acid SEQ ID NO: 200) was entirely synthesized, and amplified by PCR using the primer set described in Table 14 below. Also, using TALE-153/47 produced in [Test Example 1] <1. Method> (1) as template, amplification was performed by PCR using the primer set described in Table 14 below. These nucleotide fragments were linked by the In-Fusion method, producing the TALE12-BspD6I expression plasmid. A conceptual diagram of the configuration of the protein (TALE12-BspD6I) expressed by this expression plasmid is shown in Fig. 24.

The templates and primers used for producing these plasmids are shown in Table 14 below. Into the TALE12-BspD6I expression plasmid used for evaluating nicking activity, a TALE repeat domain corresponding to the nucleotide sequence of the TALE recognition sequence on Rosa26 (Rosa26-L, R) shown in Table 8 above was inserted. Evaluation of the nickase activity of BspD6I within the nucleus followed the SSA assay of [Test Example 1] <1. Method> (11)(ii).

**[Table 14]**

| Products | Template | Forward Primer | SEQ ID NO | Reverse Primer | SEQ ID NO |
|---|---|---|---|---|---|
| BspD6I | BspD6I | BspD6I-FinfTALE12 | 201 | BspD6I-RnfCterm | 202 |
| TALE47-12 | TALE-153/47 | TALE12-R | 203 | Cterm-F2 | 204 |

### (3) Production of TALE-BspD6I construct for evaluating base substitution activity

For evaluation of base substitution activity, a fusion protein of TALE and BspD6I having the amino acid sequence as described in Reference I was used. The amino acid sequence of such TALE differs partly in the C-terminal domain sequence from the amino acid sequence of TALE-136/63 produced in [Test Example 1] <1. Method> (1), but these can be said to be subspecies derived from the same biological species. A mutation was introduced into the TALE-136/63 expression plasmid produced in [Test Example 1] <1. Method> (1), and also, the chain length of the C-terminal domain was set to 41 amino acids, producing the TALE-136/41N (nucleotide SEQ ID NO: 205, amino acid SEQ ID NO: 206) expression plasmid, and BspD6I produced in [Test Example 2] <1. Method> (2) was added to its C-terminus. Namely, first, using the TALE12-BspD6I expression plasmid produced in [Test Example 2] <1. Method> (2) as template, amplification was performed by PCR using the primer set described in Table 15 below. Also, using the TALE-136/63 expression plasmid produced in [Test Example 1] <1. Method> (1) as template, using each primer set described in Table 15 below, the N-terminal side, inner side, and C-terminal side of TALE were respectively amplified by PCR. These nucleotide fragments were combined and linked by the In-Fusion method, producing the TALE41N-BspD6I expression plasmid.

For analysis of mitochondrial targets and nuclear genome targets, the TALE41N-BspD6I expression plasmid above, the TALE12-scND1n expression plasmid produced in [Test Example 1] <1. Method> (7), the TALE47-12-ABE8e expression plasmid produced in [Test Example 2] <1. Method> (1), and the TALE47-12-AID expression plasmid produced in [Test Example 1] <1. Method> (10) were used. In the analysis of mitochondrial targets, into these plasmids, a TALE repeat domain corresponding to the nucleotide sequence of each TALE recognition sequence described in Table 16 below was inserted. Similarly, in the analysis with nuclear genome targets, a TALE repeat domain corresponding to the nucleotide sequence of each TALE recognition sequence described in Table 17 below was inserted.

Furthermore, regarding the TALE41N-BspD6I expression plasmid and TALE12-scND1n expression plasmid above, in place of 3xFLAG+NLS to be added to the N-terminus, mitochondrial expression type mitoTALE41N-BspD6I expression plasmid and mitoTALE12-scND1n expression plasmid with SOD-MTS+3xFLAG added were produced. Also, regarding the TALE47-12-ABE8e expression plasmid produced in [Test Example 2] <1. Method> (1), in place of 3xFLAG+NLS to be added to the N-terminal side, a mitochondrial expression type mitoTALE47-12-ABE8e expression plasmid with COX8A-MTS+3xHA added was produced. Namely, the SOD-MTS+3xFLAG sequence (nucleotide SEQ ID NO: 220, amino acid SEQ ID NO: 221) was artificially synthesized and used as template, and amplified by PCR using each primer set described in Table 15 below. Also, the COX8A-MTS+3xHA sequence (nucleotide SEQ ID NO: 222, amino acid SEQ ID NO: 223) was artificially synthesized and used as template, and amplified by PCR using the primer set described in Table 15 below. Furthermore, using the TALE41N-BspD6I expression plasmid above as template, amplification was performed by PCR using the primer set below. Also, using the TALE12-scND1n expression plasmid produced in [Test Example 1] <1. Method> (7) and the TALE47-12-ABE8e expression plasmid produced in [Test Example 2] <1. Method> (1) respectively as templates, amplification was performed by PCR using the primer set described in Table 15 below. These nucleotide fragments were combined and linked by the In-Fusion method, producing the mitoTALE41N-BspD6I expression plasmid, mitoTALE12-scND1n expression plasmid, mitoTALE47-12-ABE8e expression plasmid, respectively.

The templates and primers used for producing these plasmids are shown in Table 15 below. Into these mitochondrial expression type plasmids, a TALE repeat domain corresponding to the nucleotide sequence of each TALE recognition sequence on the mitochondrial targets described in Table 16 below was inserted.

**[Table 15]**

| Products | Template | Forward Primer | SEQ ID NO | Reverse Primer | SEQ ID NO |
|---|---|---|---|---|---|
| BspD6I + β-lactamase | TALE12-BspD6I | BspD6I-FinfTALE41 | 207 | Amp_F | 23 |
| β-lactamase + TALE N-terminal | TALE-136/63 | TALE63NTD-R | 209 | Amp_R | 24 |
| inner part of TALE | TALE-136/63 | TALE63NTD-F | 210 | TALE (KQ) R | 211 |
| TALE C-treminal | TALE-136/63 | TALE (KQ) F | 212 | TALE41-R | 208 |
| SOD-MTS + 3xFLAG | SOD-MTS + 3xFLAG | SOD-FinfNterm | 213 | FLAG-RinfTALE63 | 214 |
| SOD-MTS + 3xFLAG | SOD-MTS + 3xFLAG | SOD-FinfNterm | 213 | FLAG-RinfTALE47 | 215 |
| COX8A-MTS + 3xHA | COX8A-MTS + 3xHA | COX-FinfNterm | 216 | HA-RinfTALE47 | 217 |
| TALE41N-BspD6I | TALE41N-BspD6I | pcDNA_R | 219 | TALE63NTD-F | 210 |
| TALE12-scND1n | TALE12-scND1n | pcDNA_R | 219 | TALE47NTD-F | 218 |
| TALE47-12-ABE8e | TALE47-12-ABE8e | pcDNA_R | 219 | TALE47NTD-F | 218 |

**[Table 16]**

| <Mitochondrial Target> | | |
|---|---|---|
| TALE Repeat Domain | TALE Recognition Sequence (5'-3') | SEQ ID NO |
| MT-ND1 ABE8e | tGAGTTTGATGCTCACCCN | 224 |
| MT-ND1 scND1n | tAGCCACCTCTAGCCTAN | 225 |
| MT-ND1 BspD6I | tCTAGCCTAGCCGTTN | 226 |
| MT-ND4site2 ABE8e | aATATGATTAGTTCTGTGGCN | 227 |
| MT-ND4site2 scND1n | tACTAATCTCCCTACAAN | 228 |
| MT-ND4site2 BspD6I | aATCTCCCTACAAATCTCCTN | 229 |

**[Table 17]**

| <Nuclear Genome Target> | | |
|---|---|---|
| TALE Repeat Domain | TALE Recognition Sequence (5'-3') | SEQ ID NO |
| ABE-site9 ABE8e | tGCTTATTGCTGAGGGGCAN | 230 |
| ABE-site9 scND1n (site-9-R2) | tCCATGCTGTCAAGCCCAGN | 231 |
| ABE-site9 BspD6I | tGTCAAGCCCAGCAGTCTN | 232 |
| BCL11A ABE8e | tCTAGTGCAAGCTAACAGTN | 233 |
| BCL11A scND1n (BCL11A-R3) | tAATCAGAGGCCAAACCCTTN | 234 |
| BCL11A BspD6I | tCAGAGGCCAAACCCTTCCTGN | 235 |
| VEGFA-3 ABE8e | tTCTCGCGGCTCCGCTCGN | 236 |
| VEGFA-3 scND1n (VEGFA-3-7) | tCGCTTCGGAGGAGCCGTGN | 237 |
| VEGFA-3 BspD6I | tCGGAGGAGCCGTGGTCCGN | 238 |
| HEK-1 ABE8e | tCCAGCCCTGGCCTGGGTCAN | 239 |
| HEK-1 scND1n (HEK-1-7R) | tCCTCTGCCATCACGTGN | 240 |
| HEK-1 BspD6I | tCCTCTGCCATCACGTGCTCAN | 241 |

### (4) Cloning of mitochondrial target sequence

Using genomic DNA of HEK293T cells as template, amplified DNA fragments obtained by PCR using each primer set described in Table 18 below: MT-ND1 (nucleotide SEQ ID NO: 246) and MT-ND4site2 (nucleotide SEQ ID NO: 247) were respectively cloned into the EcoRV recognition site of pBlueScript II (SK+) (Stratagene, USA), and each plasmid was designated pBS/MT-ND1 and pBS/MT-ND4site2. MT-ND1 and MT-ND4site2 are both specific sequences located within mitochondria (mitochondrial targets).

**[Table 18]**

| Products | Template | Forward Primer | SEQ ID NO | Reverse Primer | SEQ ID NO |
|---|---|---|---|---|---|
| MT-ND1 | genomic DNA | MT-ND1-F | 242 | MT-ND1-R | 243 |
| MT-ND4site2 | genomic DNA | MT-ND4site2-F | 244 | MT-ND4site2-R | 245 |

### (5) Confirmation of base substitution activity against mitochondrial targets

HEK293T cells grown in DMEM medium containing 10% FBS were seeded at 1x10⁴ cells each into each well of a 96-well plate on the day before transfection. Using Lipofectamine 3000 (manufactured by Thermo Fisher Scientific), a combination of 25 ng of the mitochondrial expression type mitoTALE41N-BspD6I expression plasmid or mitoTALE12-scND1n expression plasmid into which a TALE repeat domain sequence for each TALE recognition sequence was inserted, produced in [Test Example 2] <1. Method> (3), and 25 ng of the mitoTALE47-12-ABE8e expression plasmid, was made up to a total amount of 100 ng with pBS and introduced into HEK293T cells.

Also, a combination of 25 ng of the non-mitochondrial expression type TALE41N-BspD6I expression plasmid or TALE12-scND1n expression plasmid into which a TALE repeat domain sequence for the TALE recognition sequence was inserted, and 25 ng of the TALE47-12-ABE8e expression plasmid, and 10 ng of the plasmid into which each mitochondrial target sequence was cloned (pBS/MT-ND1, pBS/MT-ND4site2) produced in [Test Example 2] <1. Method> (4), was made up to a total amount of 100 ng with pcNDA3.1s and introduced into HEK293T cells.

Cells were collected 48 hours after each introduction, and using PureLink Genomic DNA Kits (manufactured by Thermo Fisher Scientific), genomic DNA (also including mitochondrial DNA, plasmid DNA) was respectively collected and purified. Using 10 ng of each genomic DNA as template, using KOD-ONE (manufactured by TOYOBO), the target on the plasmid (pBS), as well as the targets within mitochondria (MT-ND1 (nucleotide SEQ ID NO: 248 of PCR product) and MT-ND4site2 (nucleotide SEQ ID NO: 249 of PCR product)) were respectively amplified by PCR with the EditR amplification primer sets described in Table 19 below. The obtained PCR product was purified using NucleoSpin Gel and PCR Clean-up (manufactured by MACHEREY-NAGEL), and using 10 ng thereof as template, using the sequencing primers described in Table 19 below, regarding MT-ND1 and MT-ND4site2 on the plasmid and within mitochondria, respectively, sequencing was performed by BigDye Terminator V3.1 (manufactured by Thermo Fisher Scientific), the obtained sequence data was analyzed by EditR with a 3730xl DNA Analyzer (manufactured by Thermo Fisher Scientific), and the percentage (%) of G at each A between each TALE recognition sequence was calculated.

**[Table 19]**

| Forward Primer for EditR | SEQ ID NO | Reverse Primer for EditR | SEQ ID NO | Sequencing Primer | SEQ ID NO |
|---|---|---|---|---|---|
| pBS-F | 250 | pBS-R | 251 | MT-ND1-F | 242 |
| MT-ND1-F0 | 252 | MT-ND1-R0 | 253 | pBS-R1 | 256 |
| MT-ND4site2-F0 | 254 | MT-ND4site2-R0 | 255 | MT-ND4site2-R4 | 257 |

### (6) Confirmation of base substitution activity against nuclear genome targets

Similarly to [Test Example 2] <1. Method> (5), 25 ng of the TALE41N-BspD6I expression plasmid or TALE12-scND1n expression plasmid into which a TALE repeat domain sequence for each TALE recognition sequence described in Table 17 was inserted, produced in [Test Example 2] <1. Method> (3), and 50 ng of the TALE47-12-ABE8e expression plasmid or the TALE47-12-AID expression plasmid produced in [Test Example 1] <1. Method> (10), were made up to a total amount of 100 ng with pBS and introduced into HEK293T cells, and using the collected and purified genomic DNA as template, four types of targets on the nuclear genome (ABE-site9, BCL11A, VEGFA-3, HEK-1) were respectively amplified by PCR with the EditR amplification primer sets described in Table 20 below. Also, sequencing was performed using the sequencing primers described in Table 20 below, analyzed by EditR, and the percentage (%) of T at each C, or G at each A between each TALE recognition sequence was calculated.

**[Table 20]**

| Forward Primer for EditR | SEQ ID NO | Reverse Primer for EditR | SEQ ID NO | Sequencing Primer | SEQ ID NO |
|---|---|---|---|---|---|
| ABE-site9-F2 | 258 | ABE-site9-R | 259 | ABE-site9 | 266 |
| BCL11A-F | 260 | BCL11A-R | 261 | BCL11A | 267 |
| VEGFA3-R | 262 | VEGFA3-F | 263 | VEGFA3 | 268 |
| HEK-1-F | 264 | HEK-1-R | 265 | HEK-1 | 269 |

### (7) Production of TALE-deaminase expression plasmid for base substitution of endogenous (nuclear) DNA

Similarly to [Test Example 1] <1. Method> (10) above, a TALE47-12-AID expression plasmid for base substitution of endogenous DNA, and similarly to [Test Example 2] <1. Method> (1) above, a TALE47-12-ABE8e expression plasmid for base substitution of endogenous DNA were respectively produced. Into these expression plasmids, a TALE repeat domain corresponding to the nucleotide sequence of each TALE recognition sequence described in Table 21 below was inserted.

**[Table 21]**

| <TALE-deaminase> | | |
|---|---|---|
| TALE Repeat Domain | SEQ ID NO | TALE Recognition Sequence (5'-3') |
| AAVS1-2-L | 270 | tACCCAGAACCAGAGCCACa |
| ABE-site5-L | 271 | tGTTTTGTCTGAGGGTGAGg |
| ABE-site9-L | 230 | tGCTTATTGCTGAGGGGCAa |
| APC-R3 | 272 | tACAGAAGCGGGCAAAGGGGc |
| BCL11A-L | 233 | tCTAGTGCAAGCTAACAGTt |
| DYRK1A-L | 273 | tCCCATCACCATCACCACCAc |
| HEK1-L | 239 | tCCAGCCCTGGCCTGGGTCAa |
| HEK2-L | 274 | tCCAGCCCGCTGGCCCTGTa |
| MALTA1-L | 275 | tGGTATTCTTCAGACTATa |
| RPCI-L | 276 | tGGGTGGGAGTGGCACTTt |
| VEGFA3-L | 236 | tTCTCGCGGCTCCGCTCGg |

### (8) Production of TALE-scND1n expression plasmid for base substitution of endogenous (nuclear) DNA

Similarly to [Test Example 1] <1. Method> (7) above, a plasmid expressing TALE12-scND1n for base substitution of endogenous DNA was produced. Into these expression plasmids, a TALE repeat domain corresponding to the nucleotide sequence of each TALE recognition sequence described in Table 22 below was inserted.

**[Table 22]**

| <TALE-scND1n> | | |
|---|---|---|
| TALE Repeat Domain | SEQ ID NO | TALE Recognition Sequence (5'-3') |
| HEK1-R1 | 277 | tTCCTTTCCTCTGCCATCa |
| HEK1-R2 | 278 | tTCCTTTCCTCTGCCATCAc |
| HEK1-R3 | 279 | tTCCTTTCCTCTGCCATCACg |
| HEK1-R4 | 280 | tTCCTTTCCTCTGCCATCACGt |
| HEK1-R6 | 281 | tCCTTTCCTCTGCCATCACGTg |
| HEK1-R7 | 240 | tCCTCTGCCATCACGTGc |
| HEK1-R8 | 282 | tCCTCTGCCATCACGTGCt |
| HEK1-R9 | 283 | tCCTCTGCCATCACGTGCTc |
| HEK1-R10 | 284 | tCCTCTGCCATCACGTGCTCa |
| VEGFA3-R1 | 285 | tGTTCTCGCTTCGGAGGAGCc |
| VEGFA3-R2 | 286 | tTCTCGCTTCGGAGGAGCCg |
| VEGFA3-R3 | 287 | tTCTCGCTTCGGAGGAGCCGt |
| VEGFA3-R4 | 288 | tTCTCGCTTCGGAGGAGCCGTg |
| VEGFA3-R7 | 237 | tCGCTTCGGAGGAGCCGTGg |
| VEGFA3-R8 | 289 | tCGCTTCGGAGGAGCCGTGGt |
| VEGFA3-R9 | 290 | tTCGGAGGAGCCGTGGTc |
| VEGFA3-R10 | 291 | tTCGGAGGAGCCGTGGTCc |
| VEGFA3-R11 | 292 | tTCGGAGGAGCCGTGGTCCg |
| BCL11A-R1 | 293 | tAATCAGAGGCCAAACCCt |
| BCL11A-R2 | 294 | tAATCAGAGGCCAAACCCTt |
| BCL11A-R3 | 234 | tAATCAGAGGCCAAACCCTTc |
| BCL11A-R4 | 295 | tCAGAGGCCAAACCCTTCc |
| BCL11A-R5 | 296 | tCAGAGGCCAAACCCTTCCt |
| BCL11A-R6 | 297 | tCAGAGGCCAAACCCTTCCTg |
| ABE-site9-R1 | 298 | tCCATGCTGTCAAGCCCAg |
| ABE-site9-R2 | 231 | tCCATGCTGTCAAGCCCAGc |
| ABE-site9-R3 | 299 | tCCATGCTGTCAAGCCCAGCa |
| ABE-site9-R4 | 300 | tGCTGTCAAGCCCAGCAg |
| ABE-site9-R5 | 301 | tGCTGTCAAGCCCAGCAGt |
| ABE-site9-R6 | 302 | tGCTGTCAAGCCCAGCAGTc |
| AAVS1-2-R1 | 303 | tCCCCGAGCTGGGACCACCTt |
| MALTA1-R1 | 304 | tCCTCATTTTGTCCACTGGTGa |
| RPCI-R1 | 305 | tGGGAGATACCCCAGTCAGAg |
| ABE-site5-R1 | 306 | tGTTCATTTCCCTGAAGCCc |
| ABE-site5-R2 | 307 | tGTTCATTTCCCTGAAGCc |
| DYRK1A-R1 | 308 | tTACCCAAGGCTTGTTGTCCa |
| DYRK1A-R2 | 309 | tTACCCAAGGCTTGTTGTCc |
| HEK2-R1 | 310 | tGGCCCGCCCCGCAGTCTATg |
| HEK2-R2 | 311 | tGGCCCGCCCCGCAGTCTAt |
| APC-L2-3 | 120 | tCGGCGCCCCCTATGTACGCCn |

### (9) Production of TALE-VPR

The nucleotide sequence encoding the VPR protein (nucleotide SEQ ID NO: 312, amino acid SEQ ID NO: 313) was artificially synthesized and used as template, and amplified by PCR using the primer set described in Table 23 below. Also, using the TALE-153/47 expression plasmid produced in [Test Example 1] <1. Method> (1) as template, amplification was performed by PCR using the primer set described in Table 23 below. These nucleotide fragments were linked by the In-Fusion method, producing the TALE47-VPR (TALE47-12-VPR) expression plasmid.

The templates and primers used for producing the above plasmid are shown in Table 23 below. In order to confirm the promoting effect of TALE-VPR on base substitution activity, into the TALE47-VPR expression plasmid, a TALE repeat domain corresponding to the nucleotide sequence of the three types of TALE recognition sequences described in Table 24 below, set further outside the TALE recognition sequence to which TALE12-scND1n binds, of the system of TALE47-12-AID and TALE12-scND1n at the APC gene locus, was inserted.

**[Table 23]**

| Products | Template | Forward Primer | SEQ ID NO | Reverse Primer | SEQ ID NO |
|---|---|---|---|---|---|
| VPR | VPR | VPR _F_inf47 | 314 | VPR _R_infCterm | 315 |
| TALE-153/47 | TALE-153/47 | TALE_47_R | 13 | Cterm-F | 189 |

**[Table 24]**

| <TALE-VPR> | | |
|---|---|---|
| TALE Repeat Domain | TALE Recognition Sequence (5'-3') | SEQ ID NO |
| APC-TA-L1 | tGAGGAAGGTGAAGCACTCAg | 316 |
| APC-TA-L2 | tACTGTTGTTGGCTGTTGGTg | 317 |
| APC-TA-L3 | tGGCTCGATGCTGTTCCCAg | 318 |

### (10) Production of reporter plasmid for detecting base substitution activity of TALE-ABE8e

Oligonucleotides designed to correspond to the sequence shown in Table 25 below were annealed, and inserted into pNLF-M/A treated with restriction enzymes NheI and XhoI, producing reporter plasmids into which each sequence for examining the length of spacer 2 (distance between the target base and the TALE recognition sequence of TALE-deaminase) (sequences shown in Table 25) was inserted. The inserted sequence comprises the TALE recognition sequence of TALE-deaminase (underlined (*1) in Table 25), the codon containing the target base (underlined (*2) in Table 25: 5'-TAG-3'), the complementary sequence of the TALE recognition sequence of TALE-scND1n (underlined (*3) in Table 25), and spacer sequences between these, and the length of spacer 2 was set to 7 to 16 bases. In this reporter plasmid, the target codon is located at a position corresponding to the stop codon inserted upstream of NanoLuc luciferase, and according to such a reporter plasmid, by TALE-deaminase bound to the TALE recognition sequence, when the target codon TAG is converted to TGG (the target base (target site) A is substituted with G), NanoLuc luciferase is expressed.

**[Table 25]**

| <For Evaluating Spacer 2 Length> | | | |
|---|---|---|---|
| | Products | Sequence (5'-3') | SEQ ID NO |
| For TALE ABE8 e | ABE-C-L4M2-7bp | CTAGCTACAGAAGCGGGCAAAGg*¹ATGGCTTAG*²TCTATCTCCCGGGAGGGGTCAGGACACGGA*³GGC | 330 |
| | ABE-C-L4M2-8bp | CTAGCTACAGAAGCGGGCAAAGa*¹CATGGCTTAG*²TCTATCTCCCGGGAGGGGTCAGGACACGGA*³GGC | 331 |
| | ABE-C-L4M2-9bp | CTAGCTACAGAAGCGGGCAAAGa*¹ACATGGCTTAG*²TCTATCTCCCGGGAGGGGTCAGGACACGGA*³GGC | 332 |
| | ABE-C-L4M2-10bp | CTAGCTACAGAAGCGGGCAAAGg*¹AACATGGCTTAG*²TCTATCTCCCGGGAGGGGTCAGGACACGGA*³GGC | 333 |
| | ABE-C-L4M2-11bp | CTAGCTACAGAAGCGGGCAAAGg*¹CAACATGGCTTAG*²TCTATCTCCCGGGAGGGGTCAGGACACGGA*³GGC | 334 |
| | ABE-C-L4M2-12bp | CTAGCTACAGAAGCGGGCAAAGa*¹ACATGCGCGCTTAG*²TCTATCTCCCGGGAGGGGTCAGGACACGGA*³GGC | 335 |
| | ABE-C-L4M2-13bp | CTAGCTACAGAAGCGGGCAAAGg*1AACATGCGCGCTTAG*2TCTATCTCCCGGGAGGGGTCAGGACACGGA*3GG C | 336 |
| | ABE-C-L4M2-14bp | CTAGCTACAGAAGCGGGCAAAGg*1CAACATGCGCGCTTAG*2TCTATCTCCCGGGAGGGGTCAGGACACGGA*3G GC | 337 |
| | ABE-C-L4M2-15bp | CTAGCTACAGAAGCGGGCAAAGg*1CCAACATGCGCGCTTAG*2TCTATCTCCCGGGAGGGGTCAGGACACGGA*3 GGC | 338 |
| | ABE-C-L4M2-16bp | CTAGCTACAGAAGCGGGCAAAGg*1CCCAACATGCGCGCTTAG*2TCTATCTCCCGGGAGGGGTCAGGACACGGA *3GGC | 339 |

| | | | |
|---|---|---|---|
| *1: TALE recognition sequence of the TALE deaminase (APC-R) *2: Codon containing the target nucleotide *3: Complementary sequence corresponding to the TALE recognition sequence of TALE-scND1n (Rosa26-LM4.2 to LM.2+4) | | | |

### (11) Transfection into HEK cells and analysis of base editing activity

### (i) Reporter assay

The reporter assay, except for using each reporter plasmid produced in [Test Example 2] <1. Method> (11) above as the reporter plasmid, and using TALE-ABE8e produced in [Test Example 2] <1. Method> (1) as the TALE-deaminase expression plasmid, was performed similarly to [Test Example 1] <1. Method> (13) (i), (14) above.

### (ii) Base editing assay against endogenous (nuclear) DNA

When endogenous (nuclear) DNA was used as the target DNA, 60 ng of the TALE47-12-AID expression plasmid produced in [Test Example 2] <1. Method> (7) above; 7.5 ng or 1.5 ng (VEGFA3 site) of the TALE12-scND1n expression plasmid produced in [Test Example 2] <1. Method> (8) above; 60 ng of the TALE47-12-ABE8e expression plasmid produced in [Test Example 2] <1. Method> (7) above; 30 ng of the TALE12-scND1n expression plasmid produced in [Test Example 2] <1. Method> (8) above were combined, and similarly to [Test Example 1] <1. Method> (13)(ii), (15) above, transfection into HEK293T cells and preparation of cell lysate were performed. Using the said cell lysate as template, using the primers described in Table 26 below and Table 12, by KOD FX Neo (manufactured by TOYOBO), the region containing the target site of the said endogenous (nuclear) DNA was amplified. After purifying the obtained PCR product, sequencing was performed, sequence data was analyzed using EditR, and the percentage (%) of T at each C, or G at each A within the target range on the target site was calculated.

**[Table 26]**

| Target Gene | Primer | Sequence (5'-3') | SEQ ID NO |
|---|---|---|---|
| AAVS1-2 | AAVS1-F | CTCTGGCTCCATCGTAAGCAAACCTTAG | 340 |
| | AAVS1-R | CCTCCAACCCGGGCCCCTATGTCCAC | 341 |
| ABE-site5 | ABE-site5-F | CAGACGCCCAGAGAAGGGAAGACACC | 342 |
| | ABE-site5-R | CCGCGCCTGGTCACATTGACTTTAATTC | 343 |
| ABE-site9 | ABE-site9-F | GAAGCGAGGCATGAGGATCACTTGAGG | 344 |
| | ABE-site9-R | CCTGACAAAGCTCAGCTCCTCTCCTAG | 345 |
| BCL11A | BCL11A-F | GTGAGATGGCTGAAAAGCGATACAGGGC | 346 |
| | BCL11A-R | GCCACCGATGGAGAGGTCTGCCAGTCC | 347 |
| DYRK1A | DYRK1A-F | CTTGGTTGGTCAGGCACTGAAGCTCC | 348 |
| | DYRK1A-R | GGATGTCATGGAGTGGTGACTGTGGCC | 349 |
| HEK-1 | HEK-1-F | TCCTGCAGGGAGCTTGGCATGAGAAAC | 350 |
| | HEK-1-R | TGTCAACCAGTATCCCGGTGCAGGAGC | 351 |
| HEK-2 | HEK-2-F | CCACGTATTGCACTGCCATTCTACCAAC | 352 |
| | HEK-2-R | GTCCAGCCCCATCTGTCAAACTGTGCG | 353 |
| MALTA1 | MALTA1-F | AATTCTTACATGCAGGAACACTCAGCAGAC | 354 |
| | MALTA1-R | CACGCCAACACAGTTTGCTCACATGCC | 355 |
| RPCI | RPCI-F | GGGATAGCACCTCAAATGCCAGGGAGG | 356 |
| | RPCI-R | ATGGCACGATACACACAGACACTGCAG | 357 |
| VEGFA-3 | VEGFA-3-F | GGCTGGAGCACTGTCTGCGCACACCG | 358 |
| | VEGFA-3-R | GCGGCGGCGAGCCGCGGGCAGGGGCC | 359 |

### <2. Results>

### (1) Evaluation of nickase activity of BspD6I within the nucleus

The combination of the TALE12(Rosa26-L)-scND1n (scND1: ND1(D450N)-95-ND1, the same applies hereinafter) expression plasmid and the TALE12(Rosa26-R)-scND1n expression plasmid produced in [Test Example 1] <1. Method> (7), the combination of the TALE12(Rosa26-L)-BspD6I expression plasmid and the TALE12(Rosa26-R)-BspD6I expression plasmid produced in [Test Example 2] <1. Method> (2), or the double nicking activity by the combination of the same TALE12(Rosa26-L, R)-BspD6I expression plasmid with the nCas9(D10A) expression plasmid produced in [Test Example 1] <1. Method> (8)(9) and the guide RNA designed on the target gene Rosa26 (gRNA-A, B, D) was evaluated by a method similar to [Test Example 1] <1. Method> (11) (ii).

A graph showing the EGFP fluorescence intensity in each combination is shown in Fig. 25. As shown in Fig. 25, TALE12-scND1n, by itself, does not show double nicking activity, but by combining with nCas9 and the corresponding guide RNA, or by making the TALE repeat domain a combination of domains (L, R) that bind to two TALE recognition sequences arranged sandwiching the target base, it showed activity comparable to the CRISPR-nCas9 system. On the other hand, with TALE12-BspD6I, such activity was not confirmed by any combination, and the nicking activity of BspD6I within the nucleus could not be detected.

### (2) Evaluation of base substitution activity by BspD6I/scND1n against mitochondrial targets MT-ND1 and MT-ND4site2

By the method of [Test Example 2] <1. Method> (5), regarding the mitochondrial targets MT-ND1 and MT-ND4site2, for each target within mitochondria (mitochondrial) and each target on the introduced plasmid (plasmid), the percentage (%) of G at each A when each TALE-nickase (TALE41N-BspD6I or TALE12-scND1n) was introduced is shown in Table 27 below. Note that, in this specification, "mock" indicates the respective percentage in cells into which only 100 ng of pBS was introduced in the case of mitochondrial targets, or cells into which 10 ng of pBS/MT-ND1 or pBS/MT-ND4site2 and 90 ng of pcNDA3.1s were introduced in the case of targets on the plasmid. Note that, in MT-ND1, the distance between TALE recognition sequences when using TALE41N-BspD6I is 16 bases as described in Reference I, and A2 to A14 are within this range, and the distance between TALE recognition sequences when using TALE12-scND1n was set to 22 bases, and A2 to A19 are within this range. Also, in MT-ND4site2, the distance between TALE recognition sequences when using TALE41N-BspD6I is 15 bases as described in Reference I, and A4 to A13 are within this range, and the distance between TALE recognition sequences when using TALE12-scND1n was set to 22 bases, and A4 to A21 are within this range.

**[Table 27]**

| MT-ND1 | | A19 | A18 | A17 | A14 | A10 | A7 | A5 | A2 | |
|---|---|---|---|---|---|---|---|---|---|---|
| mitochondrial | mitoTALE41N-BspD6I | 0 | 1 | 2 | 4 | 8 | 17 | 11 | 1 | |
| | mitoTALE12-scND1n | 0 | 2 | 3 | 2 | 3 | 2 | 3 | 2 | |
| | mock | 0 | 2 | 3 | 2 | 2 | 2 | 3 | 2 | |
| plasmid | TALE41N-BspD6I | 3 | 2 | 2 | 3 | 4 | 4 | 4 | 2 | |
| | TALE12-scND1n | 2 | 3 | 16 | 26 | 6 | 4 | 5 | 2 | |
| | mock | 0 | 0 | 6 | 2 | 5 | 3 | 4 | 3 | |

| MT-ND4site2 | | A4 | A5 | A10 | A12 | A13 | A16 | A17 | A20 | A21 |
|---|---|---|---|---|---|---|---|---|---|---|
| mitochondrial | mitoTALE-BspD6I | 7 | 9 | 19 | 13 | 3 | 1 | 4 | 2 | 1 |
| | mitoTALE-scND1n | 4 | 3 | 3 | 2 | 2 | 1 | 2 | 1 | 1 |
| | mock | 5 | 3 | 2 | 1 | 2 | 2 | 2 | 3 | 1 |
| plasmid | TALE-BspD6I | 2 | 2 | 2 | 4 | 1 | 3 | 2 | 1 | 0 |
| | TALE-scND1n | 2 | 2 | 11 | 17 | 10 | 12 | 8 | 3 | 3 |
| | mock | 2 | 2 | 2 | 4 | 3 | 4 | 3 | 2 | 1 |

As shown in Table 27, against targets within mitochondria, BspD6I showed base substitution activity, but significant activity could not be detected with scND1n. Conversely, against targets on the plasmid, scND1n showed base substitution activity, but significant activity could not be detected with BspD6I. Thus, against the same mitochondrial target, since the base substitution activity of BspD6I and scND1n differed greatly depending on the target localization, it was confirmed that there is localization-dependent nicking activity. Note this result is also not contradictory to the result of [Test Example 2] <2. Results> (1).

### (3) Evaluation of base substitution activity by BspD6I/scND1n against four types of nuclear genome targets

By the method of [Test Example 2] <1. Method> (6), the base substitution activity of TALE47-12-ABE8e when each TALE-nickase (TALE12-scND1n or TALE41N-BspD6I) was cooperated against the nuclear genome targets: ABE-site-9 and BCL11A was evaluated. The percentage (%) of G at each A is shown in Table 28 below. Also, the base substitution activity of TALE47-12-AID when each TALE-nickase (TALE12-scND1n or TALE41N-BspD6I) was cooperated against the nuclear genome targets: VEGFA-3 and HEK-1 was evaluated. The percentage (%) of T at each C is shown in Table 29 below. Note that the distance between TALE recognition sequences in ABE-site-9, VEGFA-3, and HEK-1 when using TALE41N-BspD6I is 15 bases, the distance between TALE recognition sequences in BCL11A is 16 bases, and A1 to A15 or C1 to C13 are within this range, and the distance between TALE recognition sequences in ABE-site-9 and BCL11A when using TALE12-scND1n is 22 bases, the distance between TALE recognition sequences in VEGFA-3, and HEK-1 was set to 20 bases, and A1 to A22 or C1 to C17 are within this range.

**[Table 28]**

| <TALE47-12-ABE8e + TALE-nickase> | | | | | | | | | |
|---|---|---|---|---|---|---|---|---|---|
| ABE-site-9 | A1 | A5 | A6 | A8 | A11 | A12 | A15 | A17 | A19 |
| TALE12-scND1n | 3 | 4 | 3 | 3 | 10 | 3 | 5 | 2 | 1 |
| TALE41N-BspD6I | 3 | 5 | 2 | 2 | 0 | 2 | 3 | 1 | 1 |
| mock | 3 | 5 | 3 | 2 | 1 | 4 | 4 | 2 | 1 |

| BCL11A | A7 | A10 | A12 | A19 | A22 | | | | |
|---|---|---|---|---|---|---|---|---|---|
| TALE12-scND1n | 3 | 10 | 7 | 3 | 0 | | | | |
| TALE41N-BspD6I | 0 | 2 | 2 | 3 | 3 | | | | |
| mock | 0 | 3 | 3 | 3 | 3 | | | | |

**[Table 29]**

| <TALE47-12-AID + TALE-nickase> | | | | | | | | | | |
|---|---|---|---|---|---|---|---|---|---|---|
| VEGFA-3 | C1 | C3 | C6 | C9 | C10 | C11 | C12 | C13 | C15 | C17 |
| TALE12-scND1n | 6 | 4 | 7 | 14 | 15 | 15 | 14 | 11 | 5 | 2 |
| TALE41N-BspD6I | 8 | 5 | 6 | 5 | 4 | 4 | 5 | 0 | 0 | 0 |
| mock | 8 | 4 | 6 | 4 | 3 | 3 | 4 | 3 | 0 | 0 |

| HEK-1 | C2 | C3 | C10 | C11 | C12 | C16 | | | | |
|---|---|---|---|---|---|---|---|---|---|---|
| TALE12-scND1n | 3 | 5 | 21 | 15 | 13 | 3 | | | | |
| TALE41N-BspD6I | 3 | 2 | 2 | 2 | 0 | 2 | | | | |
| mock | 3 | 3 | 2 | 1 | 0 | 3 | | | | |

As shown in Table 28 and Table 29, with scND1n, base substitution activity was confirmed against all four types of nuclear genome targets, but with BspD6I, significant base substitution activity could not be confirmed against any of them. In this test, it was confirmed with a probability of 4/4 for nuclear genome targets that BspD6I does not show nicking activity.

### (4) Comparison of base editing activity of various structures of TALE-ABE8e

A conceptual diagram of the configuration of each TALE-ABE8e (TALE47-12-ABE8e, TALE16-XTEN-ABE8e, TALE47-XTEN-ABE8e, TALE47-ABE8e) expressed by the expression plasmid produced in [Test Example 2] <1. Method> (1) is shown in Fig. 26. Also, a conceptual diagram of base editing by cooperation between TALE-scND1n and TALE-ABE8e is shown in Fig. 27.

Using ABE-C-L4M2-8bp to 16bp (for TALE-ABE8e, Table 25) produced in [Test Example 2] <1. Method> (10) or ABE-L4M2-8bp to 16bp (for ABE8e-TALE, Table 11) produced in [Test Example 1] <1. Method> (12) as reporter plasmid, using the TALE12(Rosa26-LM.1.2)-scND1n expression plasmid produced in [Test Example 1] <1. Method> (7) as the TALE-scND1n expression plasmid, using the TALE47-12-ABE8e expression plasmid, TALE16-XTEN-ABE8e expression plasmid, TALE47-XTEN-ABE8e expression plasmid, TALE47-ABE8e expression plasmid produced in [Test Example 2] <1. Method> (1), or the ABE8e-32-TALE47 expression plasmid produced in [Test Example 1] <1. Method> (10) as the TALE-deaminase expression plasmid, with spacer 1 length set to 9 bases, spacer 2 length set to 8 to 16 bases, the reporter assay of [Test Example 2] <1. Method> (11)(i) was performed. The results are shown in Fig. 28.

As shown in Fig. 28, although base substitution activity was shown in all cases, among the various structures of TALE-ABE8e, the base substitution activity of TALE47-12-ABE8e was the highest. Also, TALE47-12-ABE8e with the deaminase fused to the C-terminal side tended to have higher activity than ABE8e-32-TALE47 with the deaminase fused to the N-terminal side. Therefore, in subsequent tests, TALE47-12-ABE8e was used as TALE-ABE8e.

### (5) Examination of spacer 1 length and spacer 2 length

Next, examination of the optimization of spacer 1 length and spacer 2 length was performed. Using ABE-C-L4M2-7bp to 16bp (for TALE-ABE8e, Table 25) produced in [Test Example 2] <1. Method> (10) as reporter plasmid, using the TALE12(Rosa26-LM.4.2 to LM.2+3)-scND1n expression plasmid produced in [Test Example 1] <1. Method> (7) as the TALE-scND1n expression plasmid, using the TALE47-12-ABE8e expression plasmid produced in [Test Example 2] <1. Method> (1) as the TALE-deaminase expression plasmid, with spacer 1 length set to 6 to 13 bases, spacer 2 length set to 7 to 16 bases, the reporter assay of [Test Example 2] <1. Method> (11)(i) was performed. The results are shown in Fig. 29.

As shown in Fig. 29, particularly high base substitution activity was shown when the spacer 1 length was 8 to 11 bases and the spacer 2 length was 9 to 13 bases. When converted to the distance between both TALE recognition sequences, base substitution activity was high at 19 to 23 bases, and particularly high base substitution activity was shown at 20 bases and 21 bases.

### (6) Examination of optimal inter-TALE distance in endogenous (nuclear) DNA

As a result of each reporter assay, since it was found that base substitution activity is particularly high with TALE-AID when the distance between TALE recognition sequences is 20 to 23 bases (Fig. 20), and with TALE-ABE8e when the distance between TALE recognition sequences is 19 to 23 bases (Fig. 29), respectively, next, according to the method of the endogenous DNA editing assay of [Test Example 2] <1. Method> (11)(ii), examination of the optimal distance between TALE recognition sequences in endogenous (nuclear) DNA was performed. Namely, using TALE47-12-AID produced in [Test Example 2] <1. Method> (7) as the TALE-AID expression plasmid, using TALE47-12-ABE8e produced in [Test Example 2] <1. Method> (7) as the TALE-ABE8e expression plasmid, using the TALE12-scND1n expression plasmid produced in [Test Example 2] <1. Method> (8) as the TALE-scND1n expression plasmid. Fig. 30(A) to (D) show the positional relationship between the TALE recognition sequence of TALE-deaminase and the TALE recognition sequence of TALE-scND1n at each endogenous (nuclear) target site (HEK1, VEGFA3, ABE-site9, BCL11A). Also, the sense strand of each nucleotide sequence described in Fig. 30(A) to (D) is shown in nucleotide SEQ ID NO: 360 to 363. The distance between TALE recognition sequences was set to 16 to 24 bases (Fig. 30(A), (B)), or 17 to 22 bases (Fig. 30(C), (D)). The TALE recognition sequence of each TALE-deaminase is the sequence described in Table 21 above, and the TALE recognition sequence of each TALE-scND1n is the sequence described in Table 22 above, respectively. The percentage (%) of T at each C, which is the target base between both TALE recognition sequences, or the percentage (%) of G at each A, which is the target base, is shown in Figs. 31 to 35.

Regarding TALE-AID, at the HEK1 site, maximum base substitution activity was shown when the inter-TALE distance was 19 bases (Fig. 31), and at the VEGFA3 site and BCL11A site, when it was 20 bases (Fig. 32, Fig. 33), respectively. Also, regarding TALE-ABE8e, at ABE-site9, maximum base substitution activity was shown when the inter-TALE distance was 21 bases (Fig. 34), and at the BCL11A site, when it was 20 bases (Fig. 35), respectively.

### (7) Verification of versatility of base editing by cooperation between TALE-scND1n and TALE-deaminase

Based on the results of [Test Example 1] <2. Results> (6) above, at other endogenous (nuclear) target sites, a base editing test by cooperation between TALE-scND1n and TALE-deaminase was performed to examine the versatility of the present invention. Using TALE47-12-AID produced in [Test Example 2] <1. Method> (7) as the TALE-AID expression plasmid, using TALE47-12-ABE8e produced in [Test Example 2] <1. Method> (7) as the TALE-ABE8e expression plasmid, using the TALE12-scND1n expression plasmid produced in [Test Example 2] <1. Method> (8) as the TALE-scND1n expression plasmid. Fig. 36(A) to (G) show the positional relationship between the TALE recognition sequence of TALE-deaminase and the TALE recognition sequence of TALE-scND1n at each endogenous (nuclear) target site (AAVS1-2, APC, MALTA1, RPCI, ABE-site5, HEK2, DYRK1A). Also, the sense strand of each nucleotide sequence described in Fig. 36(A) to (G) is shown in nucleotide SEQ ID NO: 364 to 370. The distance between TALE recognition sequences was set to 20 bases for TALE-AID (Fig. 36(A) to (D)), and 20 bases or 21 bases for TALE-ABE8e (Fig. 36(E) to (G)). The additional TALE recognition sequence for each TALE-deaminase is the sequence described in Table 21 above, and the additional TALE recognition sequence for each TALE-scND1n is the sequence described in Table 22 above, respectively. The results for TALE-AID (percentage (%) of T at each C, which is the target base between both TALE recognition sequences) are shown in Fig. 37, and the results for TALE-ABE8e (percentage (%) of G at each A, which is the target base between both TALE recognition sequences) are shown in Fig. 38, respectively.

As shown in Fig. 37 and Fig. 38, both TALE-AID and TALE-ABE8e showed sufficient base substitution activity at multiple target sites, and it was confirmed that the DNA editing system of the present invention comprising TALE-scND1n and TALE-deaminase is a highly versatile technology for nuclear DNA.

### (8) Promotion by TALE-VPR of base editing activity by cooperation between TALE-scND1n and TALE-deaminase

In the DNA editing system of the present invention comprising TALE-scND1n and TALE-deaminase, the promoting effect of TALE-VPR on base editing activity was examined. The APC site was selected as the endogenous (nuclear) target site, and the TALE of TALE-VPR was designed such that the distance between TALE recognition sequences was 15 to 56 bases and it binds upstream of TALE-scND1n. Fig. 39 shows the positional relationship on the APC site between the TALE recognition sequence of the TALE-deaminase produced in [Test Example 2] <1. Method> (7), the TALE recognition sequence of the TALE-scND1n produced in [Test Example 1] <1. Method> (7), and the TALE recognition sequence of the TALE-VPR produced in [Test Example 2] <1. Method> (9). The TALE of TALE-deaminase has a TALE repeat domain corresponding to the TALE recognition sequence on the APC site: APC-R3 (Table 21), and the TALE of TALE-scND1n has a TALE repeat domain corresponding to the TALE recognition sequence on the APC site: APC-L2-3 (Table 8, Table 22), respectively. The TALE of TALE-VPR has TALE repeat domains corresponding to the TALE recognition sequences on the APC site: APC-TA-L1 to L3 (Table 24), respectively. The results for TALE-AID obtained by the method of the endogenous DNA editing assay of [Test Example 2] <1. Method> (11) (ii) (percentage (%) of T at each C, which is the target base between both TALE recognition sequences) are shown in Fig. 40, and the results for TALE-ABE8e (percentage (%) of G at each A, which is the target base between both TALE recognition sequences) are shown in Fig. 41, respectively.

As shown in Fig. 40 and Fig. 41, in both TALE-deaminases, the base substitution activity was enhanced by TALE-VPR. Particularly, in TALE-ABE8e, whose base substitution activity was relatively low, the promoting effect of activity was remarkable. From these results, in the DNA editing system of the present invention, the promoting effect of TALE-VPR on base editing activity was confirmed.

### [Industrial Applicability]

As explained above, the nicking enzyme of the present invention has nickase activity as one molecule, and for example, by making it a fusion protein with a DNA binding domain, exhibits site-specific nickase activity. Therefore, such a fusion protein can substitute for the nicking function of nickase-type Cas9, and enables DNA editing by various nucleic acid editing enzymes at the target site located nearby by performing single-strand cleavage of target DNA (within cells, target DNA within cells other than mitochondrial DNA) using only protein. For example, by combining TALE-deaminase as a nucleic acid editing enzyme, a technology for base substitution on the genome using only protein can be provided, and by using various deaminases, Cas9-independent base editing of the target site can be realized. Thus, according to the present invention, it becomes possible to provide a method capable of specifically and efficiently performing editing of target DNA within cells other than mitochondrial DNA by a nucleic acid base converting enzyme, using only protein, without depending on a CRISPR-Cas system such as Cas9, as well as a nicking enzyme useful therefor. Therefore, the present invention, as an excellent genome editing tool, is expected to be utilized in a wide range of industrial fields such as medicine, agriculture, and industry.

## Claims

1. An artificial nicking enzyme comprising two domains ND1 and ND1n linked via an ND1 linker, wherein
ND1 is at least one polypeptide selected from the group consisting of the following (a) to (c):
(a) a polypeptide comprising an amino acid sequence set forth in SEQ ID NO: 98
(b) a polypeptide comprising an amino acid sequence wherein one or a plurality of amino acids are substituted, deleted, inserted and/or added in the amino acid sequence set forth in SEQ ID NO: 98, and wherein the amino acids corresponding to 66th and 83rd amino acids of the amino acid sequence set forth in SEQ ID NO: 98 are aspartic acid
(c) a polypeptide comprising an amino acid sequence having 80% or more homology with the amino acid sequence set forth in SEQ ID NO: 98, and wherein the amino acids corresponding to the 66th and 83rd amino acids of the amino acid sequence set forth in SEQ ID NO: 98 are aspartic acid
and,
ND1n is at least one polypeptide selected from the group consisting of the following (an) to (cn):
(an) a polypeptide comprising an amino acid sequence which is a substituted amino acid wherein the 66th aspartic acid and/or the 83rd aspartic acid of the amino acid sequence set forth in SEQ ID NO: 98 is substituted with another arbitrary amino acid,
(bn) a polypeptide comprising an amino acid sequence wherein one or a plurality of amino acids are substituted, deleted, inserted and/or added in the amino acid sequence set forth in SEQ ID NO: 98, and wherein the amino acid corresponding to the 66th aspartic acid and/or the 83rd aspartic acid of the amino acid sequence set forth in SEQ ID NO: 98 is a substituted amino acid substituted with an arbitrary amino acid other than aspartic acid, and
(cn) a polypeptide comprising an amino acid sequence having 80% or more homology with the amino acid sequence set forth in SEQ ID NO: 98, and wherein the amino acid corresponding to the 66th aspartic acid and/or the 83rd aspartic acid of the amino acid sequence set forth in SEQ ID NO: 98 is a substituted amino acid substituted with an arbitrary amino acid other than aspartic acid.

2. The nicking enzyme according to claim 1, wherein the length of the ND1 linker is 30 to 300 amino acid residues.

3. A fusion protein comprising a first DNA binding domain and the nicking enzyme according to claim 1.

4. A DNA editing system comprising:
a first fusion protein which is the fusion protein according to claim 3, and
a second fusion protein comprising a second DNA binding domain and a nucleic acid base converting enzyme.

5. The DNA editing system according to claim 4, further comprising a third fusion protein comprising a third DNA binding domain and a transcription regulatory factor.

6. A method for editing target DNA, comprising:
a step of bringing the DNA editing system according to claim 4 into contact with target DNA and editing a base at a target site of the target DNA by the nucleic acid base converting enzyme activity, wherein
the distance between a first DNA binding domain recognition sequence recognized by the first DNA binding domain or its complementary sequence and a second DNA binding domain recognition sequence recognized by the second DNA binding domain or its complementary sequence is 8 to 48 bases.

7. A method for producing a cell in which target DNA has been edited, comprising:
a step of introducing the DNA editing system according to claim 4 into a cell or expressing it within the cell to bring it into contact with target DNA within the cell other than mitochondrial DNA, and editing a base at a target site of the target DNA by the nucleic acid base converting enzyme activity, wherein
the distance between a first DNA binding domain recognition sequence recognized by the first DNA binding domain or its complementary sequence and a second DNA binding domain recognition sequence recognized by the second DNA binding domain or its complementary sequence is 8 to 48 bases.

8. The method according to claim 6 or 7, wherein
the first DNA binding domain recognition sequence or its complementary sequence is present via a first spacer of 4 to 16 bases on the 5' side or the 3' side of the target site, and
the second DNA binding domain recognition sequence or its complementary sequence is present via a second spacer of 3 to 31 bases on the side opposite to the first DNA binding domain recognition sequence or its complementary sequence of the target site.

9. A kit for use in the method according to claim 6 or 7, comprising:
at least one selected from the group consisting of the first fusion protein, a first fusion protein expression vector, and a polynucleotide encoding the first fusion protein, and at least one selected from the group consisting of the second fusion protein, a second fusion protein expression vector, and a polynucleotide encoding the second fusion protein, wherein
the first fusion protein expression vector is at least one selected from the group consisting of (i) a vector comprising a polynucleotide encoding ND1, ND1n, the ND1 linker, and the first DNA binding domain, and (ii) a vector comprising a polynucleotide encoding ND1, ND1n, and the ND1 linker, and an insertion site for a polynucleotide encoding the first DNA binding domain, and
the second fusion protein expression vector is at least one selected from the group consisting of (iii) a vector comprising a polynucleotide encoding the nucleic acid base converting enzyme and the second DNA binding domain, and (iv) a vector comprising a polynucleotide encoding the nucleic acid base converting enzyme and an insertion site for a polynucleotide encoding the second DNA binding domain.

10. The kit according to claim 9, further comprising at least one selected from the group consisting of a third fusion protein comprising a third DNA binding domain and a transcription regulatory factor, a third fusion protein expression vector, and a polynucleotide encoding the third fusion protein, wherein
the third fusion protein expression vector is at least one selected from the group consisting of (vii) a vector comprising a polynucleotide encoding a transcription regulatory factor and a third DNA binding domain, and (viii) a vector comprising a polynucleotide encoding the transcription regulatory factor and an insertion site for a polynucleotide encoding the third DNA binding domain.

11. The fusion protein according to claim 3, further comprising a nucleic acid base converting enzyme.

12. A method for editing target DNA, comprising a step of bringing the fusion protein according to claim 11 into contact with target DNA and editing a base at a target site of the target DNA by the nucleic acid base converting enzyme activity.

13. A method for producing a cell in which target DNA has been edited, comprising:
a step of introducing the fusion protein according to claim 11 into a cell or expressing it within the cell to bring it into contact with target DNA within the cell other than mitochondrial DNA, and editing a base at a target site of the target DNA by the nucleic acid base converting enzyme activity.

14. A kit for use in the method according to claim 12 or claim 13, comprising:
at least one selected from the group consisting of the fusion protein, a fusion protein expression vector, and a polynucleotide encoding the fusion protein, wherein
the fusion protein expression vector is at least one selected from the group consisting of (v) a vector comprising a polynucleotide encoding ND1, ND1n, the ND1 linker, a nucleic acid base converting enzyme, and the first DNA binding domain, and (vi) a vector comprising a polynucleotide encoding ND1, ND1n, the ND1 linker, and the nucleic acid base converting enzyme, and an insertion site for a polynucleotide encoding the first DNA binding domain.
